# EUROPEAN PATENT APPLICATION

(11) **EP 4 818 307 A1**
(43) Date of publication of application: **07.10.2026**
(21) Application number: 24896607.9
(22) Date of filing: 27.11.2024
(51) Int. Cl.: C12N 1/20, A61K 35/74, A61K 45/06, A61P 35/00

(54) **PARABACTEROIDES STRAIN, CULTURE, MICROBIAL AGENT AND USE THEREOF**

(30) Priority: 27.11.2023 CN 202311602627
(71) Applicant: Moon (Guangzhou) Biotech Co., Ltd., Guangzhou, Guangdong 510535 (CN)
(72) Inventor: XIAN, Yibo, Guangzhou, Guangdong 510535 (CN); LIU, Zhenzhen, Guangzhou, Guangdong 510535 (CN); ZHAO, Yingying, Guangzhou, Guangdong 510535 (CN)
(74) Representative: Herzog IP Patentanwalts GmbH
(86) International application number: PCT/CN2024/135050
(87) International publication number: WO 2025/113521

(57) **Abstract**

Provided are *Parabacteroides* strains, culture, microbial agents, and uses thereof. The *Parabacteroides* strain can specifically be a strain under an accession number GDMCC No: 62664 or 62665, a strain, where 16S rRNA of the strain has an identity of at least 96.74% to 16S rRNA of the strain under the accession number GDMCC No: 62664 or 62665, or a strain, where a genome of the strain has an average nucleotide identity (ANI) of at least 87% to a genome of the strain under the accession number GDMCC No: 62664 or 62665. The *Parabacteroides* strain or a product derived from the *Parabacteroides* strain demonstrates excellent efficacy in preventing or treating tumors or cancers, metabolic diseases, inflammatory or autoimmune diseases, infectious diseases, and central nervous system diseases.

## Description

### TECHNICAL FIELD

The present application relates to the technical field of microbiology, and in particular to *Parabacteroides* strains, culture, microbial agents, and uses thereof.

### BACKGROUND

The gut, the largest micro-ecological environment in the human body, is involved in various critical physiological processes such as nutrient absorption, energy metabolism, tissue and organ development, immune defense, and endocrine regulation. Thus, the gut is closely related to human health. In recent decades, gut microecology has experienced leapfrog development, and the understanding of gut microecology has been continuously deepened. The human gut harbors a vast community of symbiotic microorganisms. The collective genetic information carried by these symbiotic microorganisms is 50 times to 100 times greater than the genetic information of the human. The collective genetic information of these symbiotic microorganisms, known as the gut microbiome, is often referred to as the "second genome" of the human. The gut microbiome constitutes the largest and most direct external environment of the human body, and plays an indispensable role in maintaining human health.

Intestinal microorganisms include bacteria, chlamydiae, rickettsiae, mycoplasmas, spirochetes, etc. Intestinal bacteria account for 95% or more of intestinal microorganisms. Therefore, intestinal microorganisms primarily refer to the intestinal microflora. With the continuous advancement of research, the role of the intestinal microflora in intestinal and extraintestinal diseases has become increasingly clear. The intestinal microflora works much like an organ with metabolic, immune, and endocrine functions. The intestinal microflora can affect the human digestive, circulatory, and nervous systems, and is closely associated with the occurrence of various chronic diseases (such as diabetes, hypertension, cardiovascular diseases, and brain diseases) and tumors. Investigating the interrelationships of the intestinal microflora with human health and diseases not only is an important scientific research endeavor, but also holds great significance and value for clinical diagnosis, treatment, and even translational applications.

In most adults, the intestinal microflora primarily includes Gram-positive *Firmicutes* and Gram-negative *Bacteroidetes* as the two largest taxa, and further includes small proportions of *Proteobacteria*, *Actinobacteria*, *Fusobacteria*, methanogens, etc. Some bacteria of *Bacteroidetes* can benefit a host by preventing potential pathogen infections, while some bacteria of *Bacteroidetes* are opportunistic pathogens. Currently, *Bacteroides*, *Prevotella*, and *Porphyromonas* from *Bacteroidetes* are the most extensively studied, while *Parabacteroides* receives relatively little research attention. Existing studies have shown that *Parabacteroides goldsteinii* and *Parabacteroides distasonis* possess anti-inflammatory effects and *Parabacteroides distasonis* is a potential probiotic for inhibiting colon cancer. Therefore, it is necessary to further investigate intestinal *Parabacteroides* and identify strains with therapeutic activity accordingly.

### SUMMARY

The present application is intended to solve at least one of the technical problems existing in the prior art. In view of this, the present application proposes novel *Parabacteroides* strains, culture, microbial agents, and uses thereof. The novel *Parabacteroides* strains exhibit favorable therapeutic efficacy against tumors.

A first aspect of the present application provides a *Parabacteroides* strain, where the *Parabacteroides* strain is any one of the following A1) to A5):
A1) a strain, where 16S rRNA of the strain has an identity of at least 96.74%, 97%, 98%, 98.5%, 98.65%, 99%, 99.5%, 99.6%, 99.7%, 99.8%, 99.9%, 99.99%, or 100% to a sequence shown in SEQ ID NO: 1;
A2) a strain with an accession number GDMCC No: 62664 or GDMCC No: 62665;
A3) a strain, where 16S rRNA of the strain has an identity of at least 96.74%, 97%, 98%, 98.5%, 98.65%, 99%, 99.5%, 99.6%, 99.7%, 99.8%, 99.9%, 99.99%, or 100% to 16S rRNA of the strain described in the A2);
A4) a strain, where 16S rRNA of the strain has an identity of at least 97%, 98%, 98.65%, 99%, 99.5%, 99.9%, or 100% to a sequence shown in SEQ ID NO: 2, where preferably, the strain belongs to the species *Parabacteroides hominis*;
A5) a strain, where a genome of the strain has an average nucleotide identity (ANI) of at least 87%, 88%, 88.81%, 88.89%, 89%, 90%, 95%, 96%, 97%, 98%, 99%, 99.5%, 99.6%, 99.7%, 99.8%, 99.9%, 99.99%, or 100% to a genome of a strain described in any of the A1) to the A4); and/or, a strain, where a genome of the strain has an alignment fraction (AF) of at least 70%, 73.54%, 74.55%, 75%, 80%, 85%, 90%, 95%, 96%, 97%, 98%, 99%, or 100% to a genome of a strain described in any of the A1) to the A4).

In some embodiments, in the A2), a 16S rRNA sequence of the strain with the accession number GDMCC No: 62664 is shown in SEQ ID NO: 1, and a 16S rRNA sequence of the strain with the accession number GDMCC No: 62665 is shown in SEQ ID NO: 2.

In some embodiments, the *Parabacteroides* strain is derived from a human gut.

In some embodiments, the *Parabacteroides* strain is capable of producing short-chain fatty acids (SCFAs) with a high yield.

In some embodiments, the *Parabacteroides* strain is capable of producing acetic acid and/or propionic acid with a high yield.

The *Parabacteroides* strain according to the embodiments of the present application exhibits at least the following beneficial effects:

In the embodiments of the present application, a novel *Parabacteroides* strain has been discovered. The *Parabacteroides* strain or a product derived from the *Parabacteroides* strain demonstrates excellent efficacy in preventing or treating tumors or cancers, metabolic diseases, inflammatory or autoimmune diseases, infectious diseases, and central nervous system diseases.

A second aspect of the present application provides a culture of the *Parabacteroides* strain.

In some embodiments, the culture includes any one of the following B1) to B5):
B1) a fermentation medium in which the *Parabacteroides* strain has been cultured; B2) a supernatant resulting from the fermentation medium in which the *Parabacteroides* strain has been cultured; B3) a concentrate of the B1) or the B2); B4) an extract of the B1) or the B2); and B5) a dried product of any of the B1) to the B4).

In some embodiments, the fermentation medium is a liquid medium.

In some embodiments, the culture is produced by culturing the *Parabacteroides* strain in the liquid medium under anaerobic culture conditions.

In some embodiments, the fermentation medium is an MM01 liquid medium, and/or the *Parabacteroides* strain is cultured at 35°C to 42°C, and/or the *Parabacteroides* strain is cultured for 24 h to 72 h, and/or a pH of the fermentation medium is 6 to 10.

A third aspect of the present application further provides a microbial agent, including the *Parabacteroides* strain, metabolites of the *Parabacteroides* strain, or the culture.

A fourth aspect of the present application further provides a composition, where the composition or a raw material for the composition includes the *Parabacteroides* strain, metabolites of the *Parabacteroides* strain, the culture, or the microbial agent.

In some embodiments, the composition further includes at least one of an excipient, a diluent, and a carrier.

In some embodiments, the composition further includes an adjuvant.

In some embodiments, the adjuvant includes at least one of a lubricant, a wetting agent, a binder, an emulsifier, a suspension stabilizer, a solubilizer, a preservative, a sweetener, and a flavoring agent.

In some embodiments, the composition is at least one of a pharmaceutical, a nutritional supplement, a food, a food additive, a feed, and a feed additive.

In some embodiments, the composition is a solid preparation or a liquid preparation.

In some embodiments, a dosage form of the composition is any one of a powder, a granule, a tablet, a capsule, a gel, a suspension, a drop, a dropping pill, an injection, a suppository, an aerosol, an oral liquid, an ointment, an emulsion, and a lavage solution.

In some embodiments, the composition further includes a package insert.

A fifth aspect of the present application provides a pharmaceutical composition, including the following C1) and C2):
C1) the *Parabacteroides* strain, metabolites of the *Parabacteroides* strain, the culture, or the microbial agent; and
C2) a drug for combination therapy.

In some embodiments, the drug for combination therapy in the C2) includes at least one of an anti-tumor drug, an anti-metabolic disease drug, an anti-inflammatory or autoimmune disease drug, an anti-infectious disease drug, and an anti-central nervous system disease drug.

In some embodiments, the anti-tumor drug includes at least one of a chemotherapeutic drug, a photosensitizer, a photothermal agent, and an immunotherapeutic drug.

In some embodiments, the chemotherapeutic drug includes at least one of paclitaxel, camptothecin, 5-fluorouracil, cisplatin, doxorubicin, mitomycin, and epirubicin.

In some embodiments, the photosensitizer includes at least one of boron dipyrromethene, chlorin, and rose bengal.

In some embodiments, the photothermal agent includes at least one of a noble metal nanoparticle, an organic polymer, a carbon-based nanomaterial, a magnetic nanomaterial, and a semiconductor nanomaterial.

In some embodiments, the immunotherapeutic drug includes at least one of an immune cell therapy drug and an immune checkpoint inhibitor.

In some embodiments, the immune cell therapy drug includes at least one of a T cell therapy drug, a tumor-infiltrating lymphocyte therapy drug, and a natural killer (NK) cell therapy drug.

In some embodiments, the immune checkpoint inhibitor includes an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA.

In some embodiments, the pharmaceutical composition includes 1 × 10³ CFU/g to 1 × 10¹³ CFU/g or 1 × 10³ CFU/mL to 1 × 10¹³ CFU/mL of the *Parabacteroides* strain.

In some embodiments, each gram or each milliliter of the pharmaceutical composition includes at least 1 × 10³, 2 × 10³, 5 × 10³, 1 × 10⁴, 2 × 10⁴, 5 × 10⁴, 1 × 10⁵, 2 × 10⁵, 5 × 10⁵, 1 × 10⁶, 2 × 10⁶, 5 × 10⁶, 1 × 10⁷, 2 × 10⁷, 5 × 10⁷, 1 × 10⁸, 2 × 10⁸, 5 × 10⁸, 1 × 10⁹, 2 × 10⁹, 5 × 10⁹, 1 × 10¹⁰, 2 × 10¹⁰, 5 × 10¹⁰, 1 × 10¹¹, 2 × 10¹¹, 5 × 10¹¹, 1 × 10¹², 2 × 10¹², 5 × 10¹², or 1 × 10¹³ colony-forming units (CFU) of the *Parabacteroides* strain.

In some embodiments, the *Parabacteroides* strain is present in the pharmaceutical composition in a form of live bacteria, attenuated bacteria, killed bacteria, lyophilized bacteria, or irradiated bacteria.

In some embodiments, the pharmaceutical composition or a raw material for the pharmaceutical composition includes the C1) in a mass percentage of about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, or 80%.

In some embodiments, the pharmaceutical composition or the raw material for the pharmaceutical composition includes the C1) in a mass percentage of 1% to 80%, 2% to 70%, 5% to 60%, 10% to 50%, 20% to 40%, or 40% to 60%.

In some embodiments, the pharmaceutical composition further includes at least one of an excipient, a diluent, and a carrier.

In some embodiments, the pharmaceutical composition further includes an adjuvant.

In some embodiments, the adjuvant includes at least one of a lubricant, a wetting agent, a binder, an emulsifier, a suspension stabilizer, a solubilizer, a preservative, a sweetener, and a flavoring agent.

In some embodiments, the pharmaceutical composition is in an infant-suitable dosage form, a child-suitable dosage form, or an adult-suitable dosage form.

In some embodiments, the pharmaceutical composition is in a dosage form for enteral administration or a dosage form for parenteral administration.

In some embodiments, the pharmaceutical composition is an oral preparation or an injection.

In some embodiments, a dosage form of the pharmaceutical composition is any one of a powder, a granule, a tablet, a capsule, a gel, a suspension, a drop, a dropping pill, an injection, a suppository, an aerosol, an oral liquid, an ointment, an emulsion, and a lavage solution.

In some embodiments, the *Parabacteroides* strain is capable of at least partially proliferating in a gut of a subject.

In some embodiments, the pharmaceutical composition further includes a package insert.

A sixth aspect of the present application provides a pharmaceutical combination product, including the following independent components:
a first product including the *Parabacteroides* strain, metabolites of the *Parabacteroides* strain, the culture, or the microbial agent; and
a second product including a drug for combination therapy.

In some embodiments, the drug for combination therapy includes at least one of an anti-tumor drug, an anti-metabolic disease drug, an anti-inflammatory or autoimmune disease drug, an anti-infectious disease drug, and an anti-central nervous system disease drug.

In some embodiments, the anti-tumor drug includes at least one of a chemotherapeutic drug, a photosensitizer, a photothermal agent, and an immunotherapeutic drug.

In some embodiments, the chemotherapeutic drug includes at least one of paclitaxel, camptothecin, 5-fluorouracil, cisplatin, doxorubicin, mitomycin, and epirubicin.

In some embodiments, the photosensitizer includes at least one of boron dipyrromethene, chlorin, and rose bengal.

In some embodiments, the photothermal agent includes at least one of a noble metal nanoparticle, an organic polymer, a carbon-based nanomaterial, a magnetic nanomaterial, and a semiconductor nanomaterial.

In some embodiments, the immunotherapeutic drug includes at least one of an immune cell therapy drug and an immune checkpoint inhibitor.

In some embodiments, the immune cell therapy drug includes at least one of a T cell therapy drug, a tumor-infiltrating lymphocyte therapy drug, and an NK cell therapy drug.

In some embodiments, the immune checkpoint inhibitor includes an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA.

In some embodiments, the first product includes 1 × 10³ CFU/g to 1 × 10¹³ CFU/g or 1 × 10³ CFU/mL to 1 × 10¹³ CFU/mL of the *Parabacteroides* strain.

In some embodiments, each gram or each milliliter of the first product includes at least 1 × 10³, 2 × 10³, 5 × 10³, 1 × 10⁴, 2 × 10⁴, 5 × 10⁴, 1 × 10⁵, 2 × 10⁵, 5 × 10⁵, 1 × 10⁶, 2 × 10⁶, 5 × 10⁶, 1 × 10⁷, 2 × 10⁷, 5 × 10⁷, 1 × 10⁸, 2 × 10⁸, 5 × 10⁸, 1 × 10⁹, 2 × 10⁹, 5 × 10⁹, 1 × 10¹⁰, 2 × 10¹⁰, 5 × 10¹⁰, 1 × 10¹¹, 2 × 10¹¹, 5 × 10¹¹, 1 × 10¹², 2 × 10¹², 5 × 10¹², or 1 × 10¹³ CFU of the *Parabacteroides* strain.

In some embodiments, the *Parabacteroides* strain is present in the first product in a form of live bacteria, attenuated bacteria, killed bacteria, lyophilized bacteria, or irradiated bacteria.

In some embodiments, the first product and/or the second product further includes at least one of an excipient, a diluent, and a carrier.

In some embodiments, the first product and the second product each independently further include at least one adjuvant.

In some embodiments, the adjuvant includes at least one of a lubricant, a wetting agent, a binder, an emulsifier, a suspension stabilizer, a solubilizer, a preservative, a sweetener, and a flavoring agent.

In some embodiments, the pharmaceutical combination product is in an infant-suitable dosage form, a child-suitable dosage form, or an adult-suitable dosage form.

In some embodiments, the first product and the second product are each independently in a dosage form for enteral administration or a dosage form for parenteral administration.

In some embodiments, the first product and the second product are each independently an oral preparation or an injection.

In some embodiments, dosage forms of the first product and the second product are each independently any one of a powder, a granule, a tablet, a capsule, a gel, a suspension, a drop, a dropping pill, an injection, a suppository, an aerosol, an oral liquid, an ointment, an emulsion, and a lavage solution.

In some embodiments, the *Parabacteroides* strain is capable of at least partially proliferating in a gut of a subject.

In some embodiments, the pharmaceutical combination product further includes a package insert.

A seventh aspect of the present application provides use of the *Parabacteroides* strain, the culture, the microbial agent, the composition, the pharmaceutical composition, or the pharmaceutical combination product in preparation of a drug for preventing and/or treating a disease.

In some embodiments, the disease includes at least one of a tumor or cancer, a metabolic disease, an inflammatory or autoimmune disease, an infectious disease, and a central nervous system disease.

In some embodiments, the tumor or cancer includes at least one of a solid tumor, a soft tissue tumor, a hematopoietic tumor, a glandular tumor, and a metastatic tumor.

In some embodiments, the solid tumor includes at least one of head and neck cancer, upper gastrointestinal cancer, lower gastrointestinal cancer, hepatobiliary cancer, neuroendocrine tumor, thoracic cancer, bone tumor, soft tissue sarcoma, skin cancer, breast cancer, female reproductive system cancer, male reproductive system cancer, urinary system cancer, ocular cancer, central nervous system cancer, and endocrine system cancer.

In some embodiments, the hematopoietic tumor includes a tumor in at least one of blood, lymph, and bone marrow.

In some embodiments, the tumor or cancer includes at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejunoileal neuroendocrine tumor, appendiceal neuroendocrine tumor, colorectal neuroendocrine tumor, pancreatic neuroendocrine tumor, thymic carcinoma, lung cancer, malignant pleural mesothelioma, angiosarcoma, desmoid tumor, Ewing sarcoma, fibrosarcoma, gastrointestinal stromal tumor, Kaposi sarcoma, leiomyosarcoma, liposarcoma, myxofibrosarcoma, malignant peripheral nerve sheath tumor, rhabdomyosarcoma, synovial sarcoma, undifferentiated pleomorphic sarcoma, dermatofibrosarcoma protuberans, Merkel cell carcinoma, cutaneous malignant melanoma, vulvar cancer, vaginal cancer, cervical cancer, endometrial cancer, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid cancer, conjunctival cancer, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia.

In some embodiments, the tumor or cancer includes a tumor or cancer caused by a bacterium or virus.

In some embodiments, the bacterium or virus includes at least one of hepatitis B virus, hepatitis C virus, human papillomavirus, Epstein-Barr (EB) virus, *Helicobacter pylori*, and *Fusobacterium nucleatum*.

In some embodiments, the metabolic disease includes a disease characterized by a metabolic disorder in at least one of an amino acid, an organic acid, a sugar, a fat, a purine, a pigment, blood ammonia, and a metal ion.

In some embodiments, the inflammatory or autoimmune disease includes at least one of psoriasis, psoriatic arthritis, multiple sclerosis, inflammatory bowel disease, ankylosing spondylitis, chronic obstructive pulmonary disease, mesangiocapillary glomerulonephritis, myocarditis, dry eye syndrome, uveitis, Behcet's disease, asthma, atopic dermatitis, acne, Crohn's disease, ulcerative colitis, bronchitis, allergic rhinitis, Graves' disease, Hashimoto's thyroiditis, rheumatoid arthritis, type 1 diabetes, systemic lupus erythematosus, scleroderma, glomerulonephritis, systemic vasculitis, Addison's disease, autoimmune myositis, and Sjogren's syndrome.

In some embodiments, the infectious disease includes a disease caused by an infection with at least one of a bacterium, a virus, and a fungus.

In some embodiments, the central nervous system disease includes at least one of stroke, schizophrenia, drug addiction, Alzheimer's disease, Parkinson's disease, anxiety disorder, and depression.

In some embodiments, preventing and/or treating a tumor is achieved through at least one of the following (a) to (l): (a) inhibiting an increase in tumor volume; (b) inhibiting an increase in tumor weight; (c) inhibiting proliferation of a tumor cell; (d) enhancing a response rate of a tumor treatment; (e) enhancing therapeutic efficacy of an immune checkpoint inhibitor, including enhancing therapeutic efficacy of an anti-PD-1 antibody; (f) preventing and/or inhibiting dissemination or metastasis of a tumor cell; (g) reducing resistance to an anti-tumor drug; (h) inhibiting an activity of histone deacetylase (HDAC) through at least one of SCFAs including acetic acid or acetate, propionic acid or propionate, butyric acid or butyrate, and valeric acid or valerate; (i) inhibiting an activity of HDAC and/or promoting a transcriptional activity of interferon beta (IFN-β); (j) regulating or activating an immune system of a subject; (k) promoting senescence or apoptosis of a tumor cell; and (l) inhibiting angiogenesis within a tumor tissue.

An eighth aspect of the present application provides use of the *Parabacteroides* strain, the culture, the microbial agent, the composition, the pharmaceutical composition, or the pharmaceutical combination product in preventing and/or treating a disease.

In some embodiments, the disease includes at least one of a tumor or cancer, a metabolic disease, an inflammatory or autoimmune disease, an infectious disease, and a central nervous system disease.

In some embodiments, the tumor or cancer includes at least one of a solid tumor, a soft tissue tumor, a hematopoietic tumor, a glandular tumor, and a metastatic tumor.

In some embodiments, the solid tumor includes at least one of head and neck cancer, upper gastrointestinal cancer, lower gastrointestinal cancer, hepatobiliary cancer, neuroendocrine tumor, thoracic cancer, bone tumor, soft tissue sarcoma, skin cancer, breast cancer, female reproductive system cancer, male reproductive system cancer, urinary system cancer, ocular cancer, central nervous system cancer, and endocrine system cancer.

In some embodiments, the hematopoietic tumor includes a tumor in at least one of blood, lymph, and bone marrow.

In some embodiments, the tumor or cancer includes at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejunoileal neuroendocrine tumor, appendiceal neuroendocrine tumor, colorectal neuroendocrine tumor, pancreatic neuroendocrine tumor, thymic carcinoma, lung cancer, malignant pleural mesothelioma, angiosarcoma, desmoid tumor, Ewing sarcoma, fibrosarcoma, gastrointestinal stromal tumor, Kaposi sarcoma, leiomyosarcoma, liposarcoma, myxofibrosarcoma, malignant peripheral nerve sheath tumor, rhabdomyosarcoma, synovial sarcoma, undifferentiated pleomorphic sarcoma, dermatofibrosarcoma protuberans, Merkel cell carcinoma, cutaneous malignant melanoma, vulvar cancer, vaginal cancer, cervical cancer, endometrial cancer, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid cancer, conjunctival cancer, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia.

In some embodiments, the tumor or cancer includes a tumor or cancer caused by a bacterium or virus.

In some embodiments, the bacterium or virus includes at least one of hepatitis B virus, hepatitis C virus, human papillomavirus, EB virus, *Helicobacter pylori*, and *Fusobacterium nucleatum*.

In some embodiments, the metabolic disease includes a disease characterized by a metabolic disorder in at least one of an amino acid, an organic acid, a sugar, a fat, a purine, a pigment, blood ammonia, and a metal ion.

In some embodiments, the inflammatory or autoimmune disease includes at least one of psoriasis, psoriatic arthritis, multiple sclerosis, inflammatory bowel disease, ankylosing spondylitis, chronic obstructive pulmonary disease, mesangiocapillary glomerulonephritis, myocarditis, dry eye syndrome, uveitis, Behcet's disease, asthma, atopic dermatitis, acne, Crohn's disease, ulcerative colitis, bronchitis, allergic rhinitis, Graves' disease, Hashimoto's thyroiditis, rheumatoid arthritis, type 1 diabetes, systemic lupus erythematosus, scleroderma, glomerulonephritis, systemic vasculitis, Addison's disease, autoimmune myositis, and Sjogren's syndrome.

In some embodiments, the infectious disease includes a disease caused by an infection with at least one of a bacterium, a virus, and a fungus.

In some embodiments, the central nervous system disease includes at least one of stroke, schizophrenia, drug addiction, Alzheimer's disease, Parkinson's disease, anxiety disorder, and depression.

In some embodiments, preventing and/or treating a tumor is achieved through at least one of the following (a) to (k): (a) inhibiting an increase in tumor volume; (b) inhibiting an increase in tumor weight; (c) inhibiting proliferation of a tumor cell; (d) enhancing a response rate of a tumor treatment; (e) enhancing therapeutic efficacy of an immune checkpoint inhibitor, including enhancing therapeutic efficacy of an anti-PD-1 antibody; (f) preventing and/or inhibiting dissemination or metastasis of a tumor cell; (g) reducing resistance to an anti-tumor drug; (h) inhibiting an activity of HDAC through at least one of SCFAs including acetic acid or acetate, propionic acid or propionate, butyric acid or butyrate, and valeric acid or valerate; (i) inhibiting an activity of HDAC and/or promoting a transcriptional activity of IFN-β; (j) regulating or activating an immune system of a subject; (k) promoting senescence or apoptosis of a tumor cell; (l) inhibiting angiogenesis within a tumor tissue; (m) enhancing an effect of a tumor-killing immune cell; (n) local tumor immunotherapy; and (o) relieving immunosuppression in a tumor microenvironment.

A ninth aspect of the present application provides a method for treating a disease, including administering an effective dose of the *Parabacteroides* strain, the culture, the microbial agent, the composition, the pharmaceutical composition, or the pharmaceutical combination product to a subject.

In some embodiments, the disease includes at least one of a tumor or cancer, a metabolic disease, an inflammatory or autoimmune disease, an infectious disease, and a central nervous system disease.

In some embodiments, the tumor or cancer includes at least one of a solid tumor, a soft tissue tumor, a hematopoietic tumor, a glandular tumor, and a metastatic tumor.

In some embodiments, the composition further includes at least one probiotic, and the at least one probiotic is at least one of *Bifidobacterium spp*., *Lactobacillus spp.*, *Lactococcus spp.*, *Lacticaseibacillus spp.*, *Pediococcus acidilactici*, *Akkermansia*, *Pediococcus pentosaceus*, and *Saccharomyces boulardii*.

Preferably, the probiotic is of *Bifidobacterium adolescentis*, *Bifidobacterium animalis subsp.animalis*, *Bifidobacterium animalis subsp.lactis*, *Bifidobacterium bifidum*, *Bifidobacterium breve*, *Bifidobacterium longum subsp.longum*, *Bifidobacterium longum subsp.infantis*, *Lactobacillus acidophilus*, *Lactobacillus crispatus*, *Lactobacillus delbrueckii subsp.Bulgaricus*, *Lactobacillus delbrueckii subsp.Lactis*, *Lactobacillus gasseri*, *Lactobacillus helveticus*, *Lactobacillus johnsonii*, *Lactobacillus kefiranofaciens subsp.Kefiranofaciens*, *Lacticaseibacillus casei*, *Lacticaseibacillus paracasei*, *Lacticaseibacillus rhamnosus*, *Limosilactobacillus fermentum*, *Limosilactobacillus reuteri*, *Lactiplantibacillus plantarum*, *Ligilactobacillus salivarius*, *Latilactobacillus curvatus*, *Latilactobacillus sakei*, *Streptococcus salivarius subsp.thermophilus*, *Lactococcus lactis subsp.lactis*, *Lactococcus lactis subsp.lactis biovardiacetylactis*, *Lactococcus cremoris*, and *Akkermansia muciniphila*.

In some embodiments, the bacterium or virus includes at least one of hepatitis B virus, hepatitis C virus, human papillomavirus, EB virus, *Helicobacter pylori*, and *Fusobacterium nucleatum*.

In some embodiments, the metabolic disease includes a disease characterized by a metabolic disorder in at least one of an amino acid, an organic acid, a sugar, a fat, a purine, a pigment, blood ammonia, and a metal ion.

In some embodiments, the inflammatory or autoimmune disease includes at least one of psoriasis, psoriatic arthritis, multiple sclerosis, inflammatory bowel disease, ankylosing spondylitis, chronic obstructive pulmonary disease, mesangiocapillary glomerulonephritis, myocarditis, dry eye syndrome, uveitis, Behcet's disease, asthma, atopic dermatitis, acne, Crohn's disease, ulcerative colitis, bronchitis, allergic rhinitis, Graves' disease, Hashimoto's thyroiditis, rheumatoid arthritis, type 1 diabetes, systemic lupus erythematosus, scleroderma, glomerulonephritis, systemic vasculitis, Addison's disease, autoimmune myositis, and Sjogren's syndrome.

In some embodiments, the infectious disease includes a disease caused by an infection with at least one of a bacterium, a virus, and a fungus.

In some embodiments, the central nervous system disease includes at least one of stroke, schizophrenia, drug addiction, Alzheimer's disease, Parkinson's disease, anxiety disorder, and depression.

In some embodiments, preventing and/or treating a tumor is achieved through at least one of the following (a) to (k): (a) inhibiting an increase in tumor volume; (b) inhibiting an increase in tumor weight; (c) inhibiting proliferation of a tumor cell; (d) enhancing a response rate of a tumor treatment; (e) enhancing therapeutic efficacy of an immune checkpoint inhibitor, including enhancing therapeutic efficacy of an anti-PD-1 antibody; (f) preventing and/or inhibiting dissemination or metastasis of a tumor cell; (g) reducing resistance to an anti-tumor drug; (h) inhibiting an activity of histone deacetylase (HDAC) through at least one of SCFAs including acetic acid or acetate, propionic acid or propionate, butyric acid or butyrate, and valeric acid or valerate; (i) inhibiting an activity of HDAC and/or promoting a transcriptional activity of IFN-β; (j) regulating or activating an immune system of a subject; (k) promoting senescence or apoptosis of a tumor cell; and (l) inhibiting angiogenesis within a tumor tissue.

In some embodiments, the *Parabacteroides* strain, the culture, the microbial agent, the composition, the pharmaceutical composition, or the pharmaceutical combination product is administered at least once daily.

In some embodiments, when the administering is conducted twice or more daily, a same dose is administered each time.

In some embodiments, when the administering is conducted twice or more daily, a different dose is administered each time.

In some embodiments, a route for the administering includes at least one of oral administration, sublingual administration, nasal administration, rectal administration, inhalation administration, transdermal administration, intraperitoneal injection, subcutaneous injection, intradermal injection, intramuscular injection, and intravenous injection.

A tenth aspect of the present application provides a method for preparing the culture, including the following step:

inoculating the *Parabacteroides* strain into a fermentation medium to produce the fermentation medium in which the *Parabacteroides* strain has been cultured.

In some embodiments, the method further includes centrifuging the fermentation medium in which the *Parabacteroides* strain has been cultured to produce the supernatant.

In some embodiments, the method further includes concentrating the fermentation medium in which the *Parabacteroides* strain has been cultured or the supernatant through at least one of evaporation, lyophilization, dialysis, extraction, and membrane separation to produce the concentrate.

In some embodiments, the method further includes subjecting the fermentation medium in which the *Parabacteroides* strain has been cultured or the supernatant to at least one of extraction and solvent extraction to produce the extract.

In some embodiments, the method further includes drying any one of the fermentation medium in which the *Parabacteroides* strain has been cultured, the supernatant, the concentrate, and the extract to produce the dried product.

Additional aspects and advantages of the present application will be partly provided in the following description, and partly become evident in the following description or understood through the practice of the present application.

### BRIEF DESCRIPTION OF THE DRAWINGS

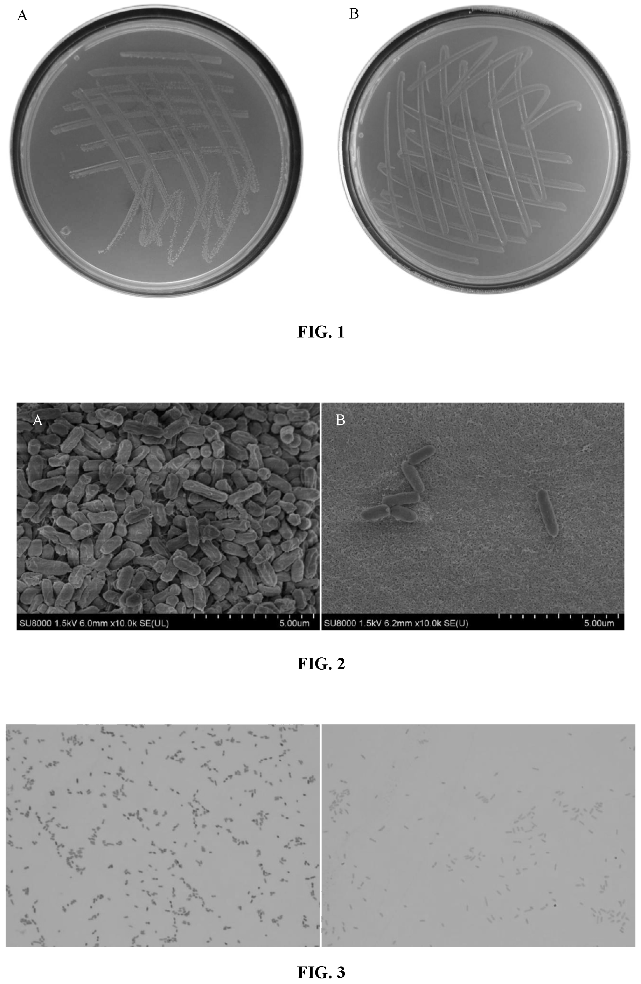

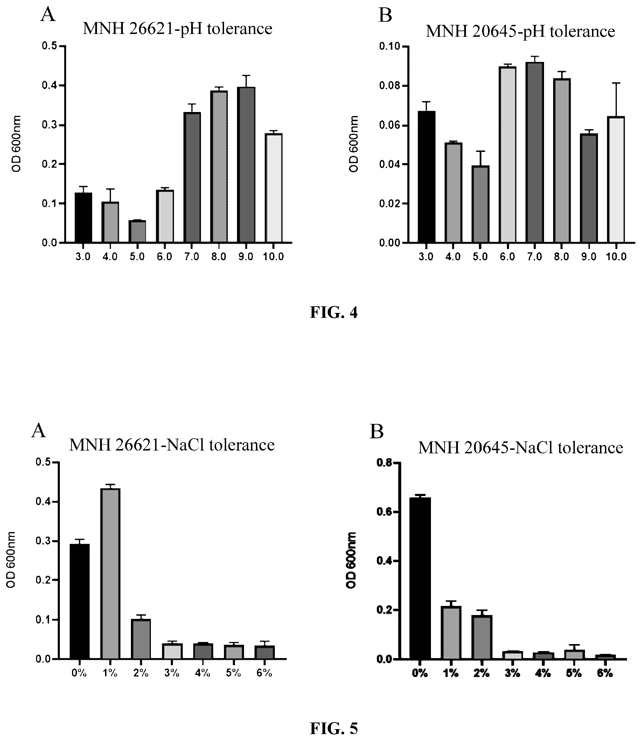

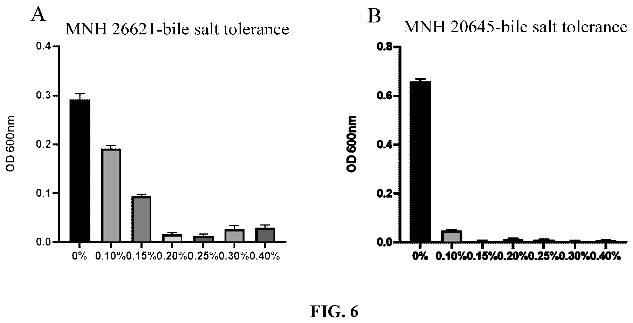

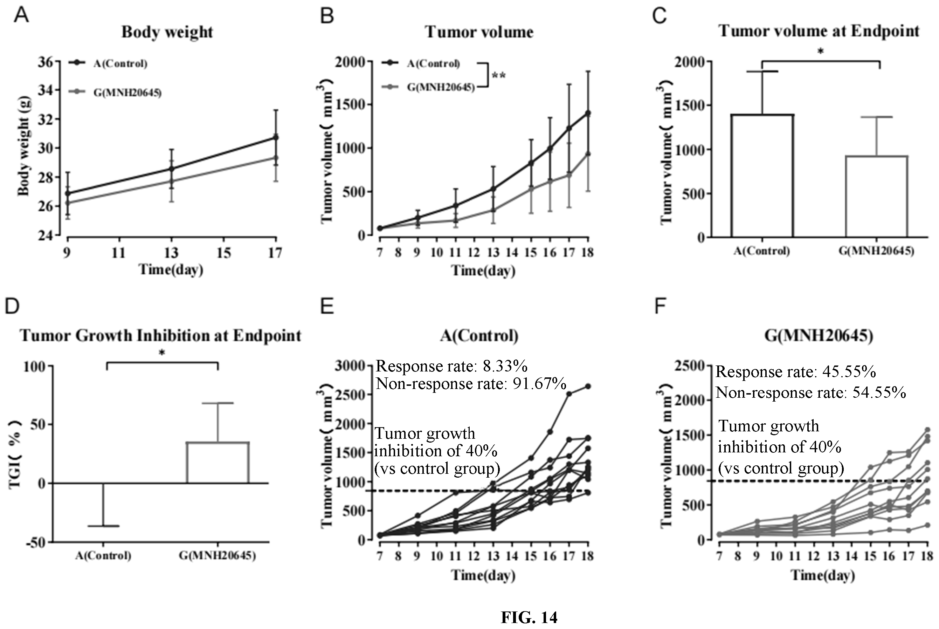

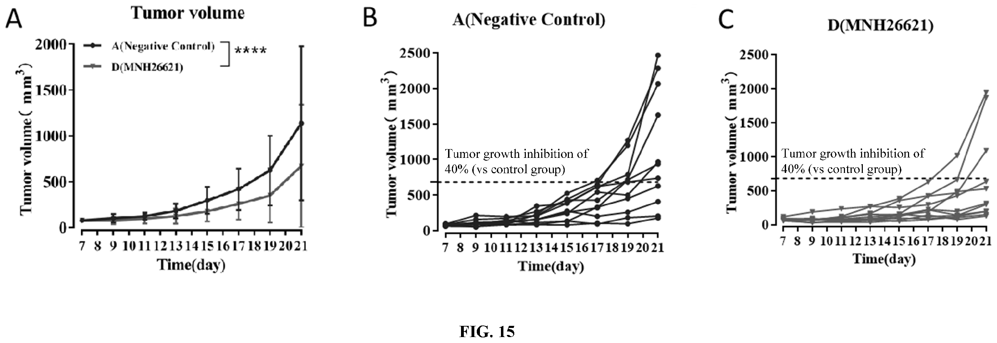

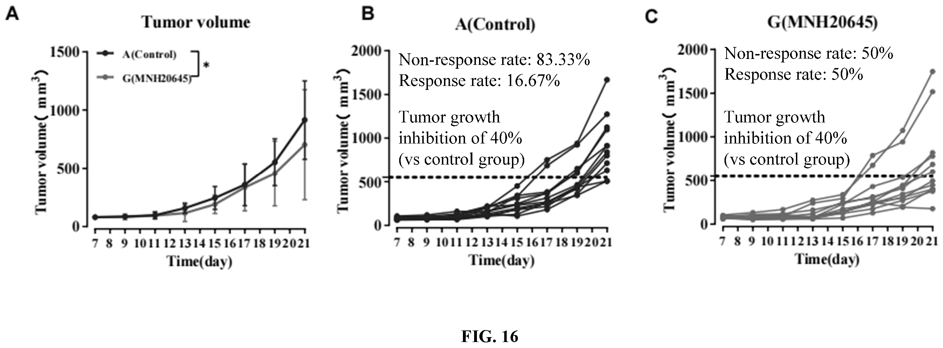

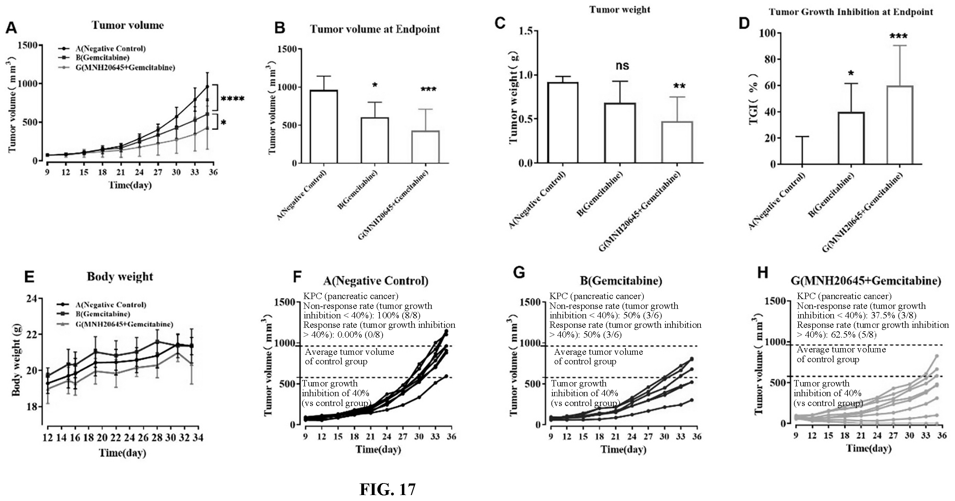

A and B in FIG. 1 show colony morphologies of a strain MNH 26621 and a strain MNH 20645 after being cultured on anaerobic blood agar plates for 48 h, respectively;
A and B in FIG. 2 show microscopic morphologies of the strain MNH 26621 and the strain MNH 20645 at a scale bar of 5 µm, respectively;
A and B in FIG. 3 show microscopic morphologies of the strain MNH 26621 and the strain MNH 20645 under Gram staining, respectively;
A and B in FIG. 4 show tolerance test results of the strain MNH 26621 and the strain MNH 20645 to different pH levels, respectively;
A and B in FIG. 5 show tolerance test results of the strain MNH 26621 and the strain MNH 20645 to NaCl at different concentrations, respectively;
A and B in FIG. 6 show tolerance test results of the strain MNH 26621 and the strain MNH 20645 to a bile salt at different concentrations, respectively;
FIG. 7, FIG. 8, and FIG. 9 show phylogenetic trees constructed based on 16S rRNA of the strain MNH 26621, the strain MNH 20645, and other related strains, respectively;
FIG. 10 shows regulatory effects of the strain MNH 26621 on an immune activity of macrophages;
FIG. 11 shows regulatory effects of the strain MNH 20645 on an immune activity of macrophages;
FIG. 12 shows regulatory effects of the strain MNH 26621 on an immune activity of primary peripheral blood mononuclear cells (PBMCs);
A and B in FIG. 13 show impacts of the strain MNH 26621 and the strain MNH 20645 on IFN-β expression, respectively;
A to F in FIG. 14 show body weight change curves, tumor volume change curves, tumor volumes at endpoint, and tumor growth inhibition rate at endpoint (TGI) of mice in an experimental group and a control group of the strain MNH 20645, response rates of the control group, and response rates of the experimental group according to an embodiment, respectively, where a response rate is presented as a tumor volume value; a dotted line represents a tumor volume of the control group corresponding to TGI of 40%; when a tumor volume of a mouse at an experimental endpoint is greater than the tumor volume of the control group corresponding to TGI of 40%, it indicates non-response; and when a tumor volume of a mouse at the experimental endpoint is less than or equal to the tumor volume of the control group corresponding to TGI of 40%, it indicates response;
A to C in FIG. 15 show tumor volume change curves of mice in an experimental group and a control group of the strain MNH 26621, response rates of the control group, and response rates of the experimental group according to an embodiment, respectively, where a response rate is presented as a tumor volume value; a dotted line represents a tumor volume of the control group corresponding to TGI of 40%; when a tumor volume of a mouse at an experimental endpoint is greater than the tumor volume of the control group corresponding to TGI of 40%, it indicates non-response; and when a tumor volume of a mouse at the experimental endpoint is less than or equal to the tumor volume of the control group corresponding to TGI of 40%, it indicates response;
A to C in FIG. 16 show tumor volume change curves of mice in an experimental group and a control group of the strain MNH 20645, response rates of the control group, and response rates of the experimental group according to an embodiment, respectively, where a response rate is presented as a tumor volume value; a dotted line represents a tumor volume of the control group corresponding to TGI of 40%; when a tumor volume of a mouse at an experimental endpoint is greater than the tumor volume of the control group corresponding to TGI of 40%, it indicates non-response; and when a tumor volume of a mouse at the experimental endpoint is less than or equal to the tumor volume of the control group corresponding to TGI of 40%, it indicates response;
A to H in FIG. 17 show tumor change curves of groups A/B/C, tumor volumes at endpoint of the groups A/B/C, tumor weights at endpoint of the groups A/B/C, TGI levels of the groups A/B/C, body weight change curves of the groups A/B/C, response rates of the group A, response rates of the group B, and response rates of the group G according to an embodiment, respectively, where the strain MNH 20645 is used in combination with gemcitabine in the group G;

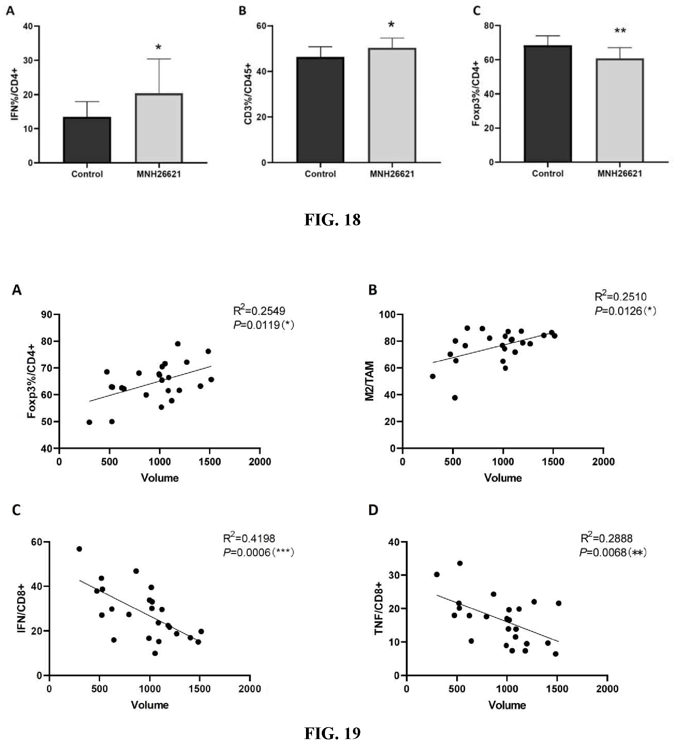

FIG. 18 shows immune activation analysis results for the strain MNH 26621 in lung cancer-bearing mice according to an embodiment;
FIG. 19 shows analysis results for the correlation between relevant cells in tumor tissues and tumor volumes at endpoint according to an embodiment;

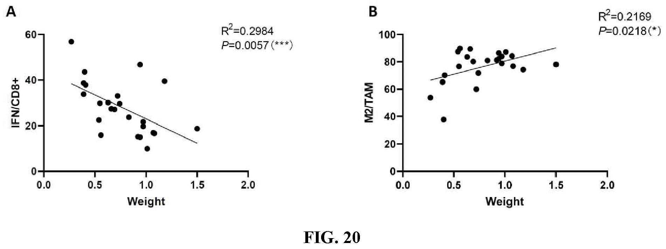

FIG. 20 shows analysis results for the correlation of M2 macrophages and cytotoxic CD8⁺IFNγ⁺ cells in tumor tissues with tumor weights according to an embodiment;

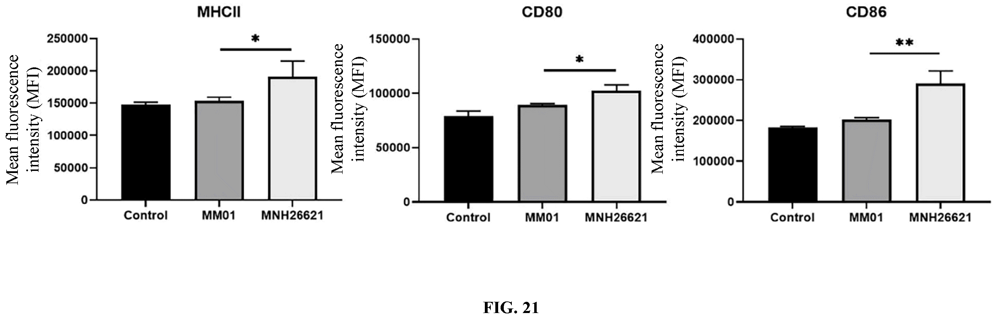

FIG. 21 shows flow cytometry results of molecules MHCII, CD80, and CD86 on the surface of dendritic cells (DCs) in an MNH 26621 group according to an embodiment;

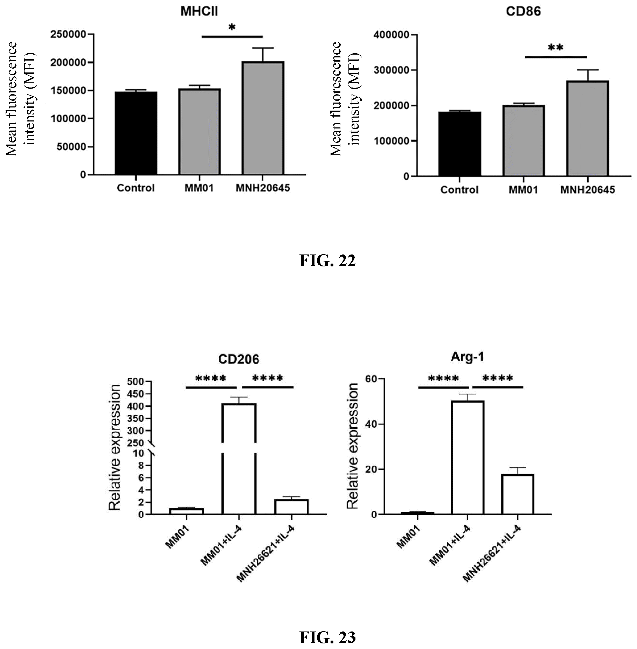

FIG. 22 shows flow cytometry results of molecules MHCII and CD86 on the surface of DCs in an MNH 20645 group according to an embodiment;
FIG. 23 shows quantitative polymerase chain reaction (qPCR) detection results of the expression of M2 markers CD206 and Arg1 by RAW264.7 cells in an MNH 26621 group according to an embodiment; and

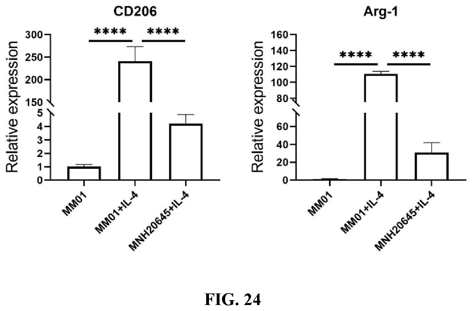

FIG. 24 shows qPCR detection results of the expression of M2 markers CD206 and Arg1 by RAW264.7 cells in an MNH 20645 group according to an embodiment.

In the above figures, if significance analysis is involved, the significance analysis is performed using unpaired t test, two-way analysis of variance (two-way ANOVA), Student's t test, etc. No significant difference (ns) is defined as p ≥ 0.05; * indicates p < 0.05; ** indicates p < 0.01; *** indicates p < 0.001; and **** indicates p < 0.0001.

### DETAILED DESCRIPTION OF THE EMBODIMENTS

The concept of the present application and the technical effects produced by the present application will be described clearly and completely below with reference to embodiments, such that the objectives, features, and effects of the present application can be fully understood. Apparently, the described embodiments are merely some rather than all of the embodiments of the present application. All other embodiments obtained by those skilled in the art based on the embodiments of the present application without creative efforts shall fall within the scope of protection of the present application.

The embodiments of the present application are described in detail below. The described embodiments are exemplary and only intended to explain the present application, but should not be construed as a limitation to the present application.

In the description of the present application, the term "several" means one or more, the term "a plurality of" means two or more, the terms such as "greater than", "less than", and "exceeding" are construed to exclude the listed number, the terms "no less than", "no more than", and "within" are construed to include the listed number, and the term "about" indicates a range of ±20%, 10%, 8%, 5%, 4%, 3%, 2%, 1%, 0.5%, 0.2%, 0.1%, etc., of the listed number. The "first" and "second" in the description are merely intended to distinguish technical features, rather than to indicate or imply relative importance or implicitly indicate a number of the indicated technical features or implicitly indicate a sequential order of the indicated technical features.

In the description of the present application, the description with reference to the terms "one embodiment", "some embodiments", "exemplary embodiments", "examples", "specific examples", or "some examples" means that specific features, structures, materials, or characteristics described in combination with the embodiments or examples are included in at least one embodiment or example of the present application. In this specification, the schematic representations of the above terms do not necessarily refer to a same embodiment or example. In addition, the specific features, structures, materials, or characteristics described may be combined in any suitable manner in any one or more embodiments or examples.

A first aspect of the embodiments of the present application relates to a *Parabacteroides* strain, including two strains MNH 26621 and MNH 20645. The strain MNH 26621 was deposited in the Guangdong Microbial Culture Collection Center (GDMCC) located at the Guangdong Institute of Microbiology, Guangdong Academy of Sciences, No. 100, Xianlie Middle Road, Guangzhou on July 28, 2022, with an accession number GDMCC No: 62664, a taxonomic name of *Parabacteroides* sp., and a deposit name of *Parabacteroides* sp. MNH 26621. The strain MNH 20645 was deposited in the Guangdong Microbial Culture Collection Center (GDMCC) located at the Guangdong Institute of Microbiology, Guangdong Academy of Sciences, No. 100, Xianlie Middle Road, Guangzhou on July 28, 2022, with an accession number GDMCC No: 62665, a taxonomic name of *Parabacteroides* sp., and a deposit name of *Parabacteroides* sp. MNH 20645.

The term "strain" refers to a member of a bacterial species that possesses a genetic characteristic distinguishing the member from closely related members of the same bacterial species. The genetic characteristic may include the complete or partial absence of at least one gene, the complete or partial absence of at least one regulatory region (such as a promoter, a terminator, a riboswitch, and a ribosome binding site), the presence of at least one recombinant gene, the presence of at least one mutated gene, the presence of at least one exogenous gene (a gene from another species), the presence of at least one non-natural plasmid, the presence of at least one antibiotic resistance cassette, or a combination thereof. A genetic characteristic distinguishing between different strains can be identified through polymerase chain reaction (PCR) amplification, and optionally, DNA sequencing can be subsequently conducted for a genomic region of interest or the entire genome. In the case where a strain (compared to another strain of a same species) gains or loses antibiotic resistance, or gains or loses a biosynthetic capacity (such as an auxotrophic strain), the strains can be distinguished through selection based on a specific nutrition/metabolite or counter-selection using an antibiotic.

It is known in the art that strains can be classified and identified through traditional taxonomic approaches and molecular biology techniques. The traditional taxonomic approaches include, for example, cell morphology observation, Gram staining, flagella staining, various metabolic assays, etc. The molecular biology techniques include ribosomal RNA sequencing, whole genome sequencing-based techniques, etc. The strain MNH 26621 and the strain MNH 20645 involved in the embodiments of the present application have been identified as novel *Parabacteroides* strains based on traditional taxonomic characteristics and molecular biology techniques. The traditional taxonomic characteristics include cellular and colonial characteristics: Bacterial cells do not have spores or flagella, are non-motile, rod-shaped or short rod-shaped, and Gram-negative, and have a diameter of approximately (3-5) µm × (8-20) µm. Under anaerobic culture conditions (for example, optionally, anaerobic culture at 37°C for 48 h), visible circular opaque colonies can be formed, with regular and smooth edges and no secretions around the colonies.

In some embodiments, the *Parabacteroides* strain is derived from a gut. For example, the *Parabacteroides* strain can be derived from any one of a large intestine, a small intestine, and a rectum of a mammal, such as a large intestine, a small intestine, or a rectum of an animal from *Monotremata*, *Marsupialia*, *Insectivora*, *Macroscelidea*, *Scandentia*, *Dermoptera*, *Chiroptera*, *Primates*, *Xenarthra*, *Pholidota*, *Lagomorpha*, *Rodentia*, *Carnivora*, *Sirenia*, *Hyracoidea*, *Tubulidentata*, *Perissodactyla*, *Artiodactyla*, *Cetacea*, etc. Common mammals include *Rodentia* (such as mice, rats, hamsters, and guinea pigs), *Lagomorpha* (such as rabbits), *Perissodactyla* (such as horses and donkeys), *Artiodactyla* (such as sheep, goats, camels, cattle, and pigs), *Primates* (such as monkeys, gorillas, chimpanzees, and humans), *Carnivora* (such as dogs and cats), etc. It can be understood that a strain derived from the gut can be isolated from feces, for example.

In some embodiments, the *Parabacteroides* strain or a strain variant thereof has the characteristic of producing SCFAs with a high yield. Generally, SCFAs include saturated fatty acids with a chain length of 1 to 6 carbon atoms, such as formic acid, acetic acid, propionic acid, n-butyric acid, isobutyric acid, n-valeric acid, isovaleric acid, neopentanoic acid, and hexanoic acid. The high yield means that a yield is 100 µg/g or higher as measured in SCFA determination.

In some embodiments, the *Parabacteroides* strain or a strain variant thereof exhibits the characteristic of producing acetic acid and/or propionic acid with a high yield. The high yield of acetic acid means that a yield of acetic acid is higher than or equal to 100 µg/g as measured in SCFA determination. The high yield of acetic acid means that a yield of propionic acid is higher than or equal to 100 µg/g as measured in SCFA determination. For example, after the *Parabacteroides* strain or a strain variant thereof is inoculated into a Trypticase Soy Broth (TSB) liquid medium and cultured anaerobically at 37°C for 48 h and bacterial cells are collected through centrifugation, a detected acetic acid yield is higher than or equal to 100 µg/g, higher than or equal to 200 µg/g, higher than or equal to 300 µg/g, higher than or equal to 400 µg/g, higher than or equal to 500 µg/g, higher than or equal to 600 µg/g, higher than or equal to 700 µg/g, higher than or equal to 800 µg/g, higher than or equal to 900 µg/g, higher than or equal to 1,000 µg/g, or higher than or equal to 1,100 µg/g, and a detected propionic acid yield is higher than or equal to 100 µg/g, higher than or equal to 200 µg/g, higher than or equal to 300 µg/g, higher than or equal to 400 µg/g, higher than or equal to 500 µg/g, higher than or equal to 600 µg/g, higher than or equal to 700 µg/g, higher than or equal to 800 µg/g, higher than or equal to 900 µg/g, higher than or equal to 1,000 µg/g, or higher than or equal to 1,100 µg/g.

According to sequencing results for 16S rRNA of the strain MNH 26621, a sequence of the 16S rRNA is shown in SEQ ID NO: 1. According to sequencing results for 16S rRNA of the strain MNH 20645, a sequence of the 16S rRNA is shown in SEQ ID NO: 2. 16S rRNA is a ribosomal RNA in prokaryotes. A 16S rRNA gene includes variable regions and conserved regions. The conserved regions are shared by all bacteria, while the variable regions vary among different bacteria to different degrees. By comparing sequences of 16S rRNA genes in bacteria and calculating evolutionary distances based on nucleotide divergences, a phylogenetic tree can be plotted.

When an identity between 16S rRNA gene sequences of two strains is lower than 98.65%, these two strains can be classified as belonging to different species (see, Kim, M., Oh, H.-S., Park, S.-C., & Chun, J. (2014). Towards a taxonomic coherence between average nucleotide identity and 16S rRNA gene sequence similarity for species demarcation of prokaryotes. INTERNATIONAL JOURNAL OF SYSTEMATIC AND EVOLUTIONARY MICROBIOLOGY, 64 (Pt 2), 346-351; Liu, C., Du, M.-X., Abuduaini, R., Yu, H.-Y., Li, D.-H., Wang, Y.-J., Liu, S.-J. (2021). Enlightening the taxonomy darkness of human gut microbiomes with a cultured biobank. Microbiome, 9 (1), P23; and Pablo Yarza, Uniting the classification of cultured and uncultured bacteria and archaea using 16S rRNA gene sequences. Microbiology, volume 12, p635-645, 2014).

When an identity between 16S rRNA gene sequences of two strains is lower than 95%, these two strains can be classified as belonging to different genera ("A genus definition for Bacteria and Archaea based on genome relatedness and taxonomic affiliation", R.A. Barco, bioRxiv(doi: https://doi.org/10.1101/392480); Evaluation of 16S rRNA gene sequencing for species and strain-level microbiome analysis, Jethro S. Johnson, Nature Communications 10, Article number: 5029 (2019)).

Therefore, in some embodiments, variants of the strain MNH 26621 based on the 16S rRNA sequence are provided. These variants have an identity of at least 95%, 96%, 96.74%, 97%, 98%, 98.5%, 98.65%, 99%, 99.5%, 99.6%, 99.7%, 99.8%, 99.9%, 99.99%, or 100% with the strain MNH 26621 under the accession number GDMCC No: 62664 in terms of 16S rRNA sequences. Consequently, these variants are of the same species as the strain MNH 26621 or of different species from the strain MNH 26621 in a same genus. Thus, it can be reasonably inferred that these strains, either of a same species or different species in a same genus, possess similar biological activities. In some other embodiments, these variants carry a 16S rRNA sequence having an identity of at least 95%, 96%, 96.74%, 97%, 98%, 98.5%, 98.65%, 99%, 99.5%, 99.6%, 99.7%, 99.8%, 99.9%, 99.99%, or 100% with the sequence shown in SEQ ID NO: 1.

In some embodiments, variants of the strain MNH 20645 based on the 16S rRNA sequence are also provided. These variants have an identity of at least 95%, 96%, 96.74%, 97%, 98%, 98.5%, 98.65%, 99%, 99.5%, 99.6%, 99.7%, 99.8%, 99.9%, 99.99%, or 100% with the strain MNH 20645 under the accession number GDMCC No: 62665 in terms of 16S rRNA sequences. Consequently, these variants are of the same species as the strain MNH 20645 or of different species from the strain MNH 20645 in a same genus. Thus, it can be reasonably inferred that these strains, either of a same species or different species in a same genus, possess similar biological activities. In some other embodiments, these variants carry a 16S rRNA sequence having an identity of at least 95%, 96%, 96.74%, 97%, 98%, 98.5%, 98.65%, 99%, 99.5%, 99.6%, 99.7%, 99.8%, 99.9%, 99.99%, or 100% with the sequence shown in SEQ ID NO: 2.

The "identity" between sequences of two nucleic acid molecules can be determined using a known computer algorithm, such as at least one of a GCG program package, BLASTN, and FASTA. Other commercial or publicly available programs may include, for example, DNAStar "MegAlign" program.

Moreover, with the rapid development of second-generation and third-generation sequencing technologies, the identification of strains based on whole genome sequencing has become feasible, which can enhance the accuracy of identification results of strains. ANI of two bacterial genomes refers to a similarity of homologous genes between the two bacterial genomes. In the field of bacterial taxonomy, it is widely believed that an ANI value of 95% or more is required to define two strains as belonging to a same species (Jain C, Rodriguez-R L M, Phillippy A M, et al., High throughput ANI analysis of 90K prokaryotic genomes reveals clear speciesboundaries[J]. Nature Communications, 2018, 9 (1): 5114.).

With the above method, those skilled in the art can determine whether an isolated strain is a novel strain of the same species as or of a different species from the strain MNH 26621 or MNH 20645 within the genus *Parabacteroides*. For example, when an ANI value of an isolated strain with the strain under the accession number GDMCC No: 62664 is at least 87%, for example, at least 87%, 88%, 88.81%, 88.89%, 89%, 90%, 95%, 95.5%, 96%, 96.5%, 97%, 97.5%, 98%, 98.5%, 99%, 99.5%, 99.6%, 99.7%, 99.8%, 99.9%, 99.99%, or 100%, it can be determined that the isolated strain belongs to a different species from or the same species as the strain under the accession number GDMCC No: 62664 within the genus *Parabacteroides*. Similarly, when an ANI value of an isolated strain with the strain under the accession number GDMCC No: 62665 is at least 87%, for example, at least 87%, 88%, 88.81%, 88.89%, 89%, 90%, 95%, 95.5%, 96%, 96.5%, 97%, 97.5%, 98%, 98.5%, 99%, 99.5%, 99.6%, 99.7%, 99.8%, 99.9%, 99.99%, or 100%, it can be determined that the isolated strain belongs to a different species from or the same species as the strain under the accession number GDMCC No: 62665 within the genus *Parabacteroides*.

There have been various mature calculation tools for ANI, such as the locally operated software Jspecies (/jspecies) and Gegenees (/documentation.html) and the online calculation tools including ANI caculator (http:/enveomics.gatech.edu/), EzGenome (/ezgenome/ani), and ANItools. Therefore, an ANI value between an isolated strain and the strain MNH 26621 or MNH 20645 can be calculated using any of the aforementioned locally operated software or the online calculation tools and other tools or methods known in the art.

Therefore, in some embodiments, variants of the strain MNH 26621 or MNH 20645 based on the aforementioned genome are provided. These variants have an ANI value of at least 95%, 96%, 96.74%, 97%, 98%, 98.5%, 98.65%, 99%, 99.5%, 99.6%, 99.7%, 99.8%, 99.9%, 99.99%, or 100% with the strain under the accession number GDMCC No: 62664 or GDMCC No: 62665 or any of the aforementioned other variants in terms of genomes. Consequently, these variants are of the same species as or of a different species from the strain MNH 26621 or MNH 20645 within the genus *Parabacteroides*. Thus, it can be reasonably inferred that these strains, either of a same species or different species in a same genus, possess similar biological activities. The determination can also be conducted based on AF of corresponding genomes. For example, a variant having AF of at least 60%, 70%, 73.54%, 74.55%, 75%, 80%, 85%, 90%, 95%, 96%, 97%, 98%, 99%, or 100% with the strain under the accession number GDMCC No: 62664 or GDMCC No: 62665 or any of the aforementioned other variants also possesses an identical or similar biological activity.

The term "variant" includes any bacteria derived from the strain MNH 26621 or MNH 20645, such as bacteria generated through natural mutation or recombination (for example, a mutation resulting from cell proliferation or cell division), resistance screening, or mutation or recombination based on any other mechanism including radiation, a virus, a transposon, or a mutagenic chemical. The variant also includes other strains isolated from natural environments that share the same or similar taxonomic characteristics and properties with the strain MNH 26621 or MNH 20645. The variant generally includes strains within the genus *Parabacteroides* that are of a different species from the strain MNH 26621 or MNH 20645, or are of the same species as the strain MNH 26621 or MNH 20645 but exhibit different phenotypic variations.

A second aspect of the embodiments of the present application relates to a culture of the *Parabacteroides* strain. The term "culture" refers to a product obtained by culturing an isolated strain in a fermentation medium. The fermentation medium may be a natural or artificial medium, and can be, for example, a solid medium or a liquid medium. In some embodiments, the fermentation medium includes a peptone, such as at least one of meat peptone, casein peptone, whey peptone, and soybean peptone. The fermentation medium may further include other inorganic salts or organic substances to promote bacterial growth.

In some embodiments, the culture can be a fermentation medium in which the *Parabacteroides* strain has been cultured; a supernatant resulting from the fermentation medium (for example, through centrifugation); a concentrate produced from the fermentation medium or the supernatant (for example, through at least one of evaporation, lyophilization, dialysis, extraction, and membrane separation); an extract resulting from the fermentation medium or the supernatant (for example, through at least one of extraction and solvent extraction using water or an organic solvent); or a dried product produced by drying any one of the fermentation medium, the supernatant, the concentrate, and the extract. Further, in some specific embodiments, the extract may be produced through extraction targeting one or more specific components, such as extraction targeting components with specific molecular weight ranges, specific solubility, specific isoelectric points, etc.; and may also be produced through extraction not targeting one or more specific components. A specific extraction process is as follows: At least one of methanol, ethanol, chloroform, water, an acid, and an alkali is used to conduct extraction in a specific process sequence at a specified temperature. For example, extraction may be conducted in 75 v/v% ethanol at 60°C to 80°C for 1 min to 10 min, or in methanol at -30°C to -10°C for 1 min to 10 min, or in a methanol/chloroform/water solution at -40°C to -20°C for 30 min to 60 min, or in deionized water at 95°C for 1 min to 10 min, or in a perchloric acid, trichloroacetic acid, hydrochloric acid, or sodium hydroxide solution at 0°C to 4°C, so as to extract corresponding metabolites, such as SCFAs or salts thereof, including at least one of acetic acid, butyric acid, valeric acid, acetate, propionate, and valerate.

In some embodiments, the culture is produced by culturing the *Parabacteroides* strain in the liquid medium under anaerobic culture conditions. For example, the liquid medium is an MM01 liquid medium (including about 5 g/L of peptone, about 5 g/L of trypticase, about 10 g/L of yeast extract, about 5 g/L of beef extract, about 5 g/L of glucose, about 2 g/L of K₂HPO₄, about 2 g/L of sodium acetate, about 1 mL/L of Tween 80, about 5 mg/L of hemin, about 0.5 g/L of L-cysteine hydrochloride, about 1 µL/L of vitamin K1, and about 8 mL/L of an inorganic salt solution (including about 0.25 g/L of calcium chloride, about 1 g/L of dipotassium hydrogen phosphate, about 1 g/L of potassium dihydrogen phosphate, about 0.5 g/L of magnesium sulfate, about 10 g/L of sodium bicarbonate, and about 2 g/L of sodium chloride)). In some embodiments, the anaerobic culturing is conducted at 35°C to 42°C, such as 37°C. In some embodiments, the anaerobic culturing is conducted for 24 h to 72 h, including 36 h to 60 h, such as 48 h. In some embodiments, the anaerobic culturing is conducted at a pH of 6 to 10, such as 7.

It can be understood that the culture includes compounds and/or cellular debris of the *Parabacteroides* strain, and/or metabolites secreted by the *Parabacteroides* strain. The metabolites include small molecules (such as amino acids, nucleosides, and nucleotides) and macromolecules (such as polypeptides, carbohydrates, nucleic acids, proteoglycans, and lipids). The metabolites can be primary metabolites directly involved in normal cellular functions, secondary metabolites generally not required for basic cellular functions, or metabolic intermediates generated during the synthesis of primary or secondary metabolites. Thus, the similar biological functions to the *Parabacteroides* strain can be achieved through different types of cultures including compounds and/or metabolites of the *Parabacteroides* strain.

A third aspect of the embodiments of the present application relates to a microbial agent, including one or more of the *Parabacteroides* strain, metabolites of the *Parabacteroides* strain, and the culture, for example, the strain under the accession number GDMCC No: 62664 or metabolites or culture thereof, and/or the strain under the accession number GDMCC No: 62665 or metabolites or culture thereof.

A fourth aspect of the embodiments of the present application relates to a composition. The composition includes the *Parabacteroides* strain, metabolites of the *Parabacteroides* strain, the culture, or the microbial agent. Alternatively, a raw material for preparing the composition includes the *Parabacteroides* strain, metabolites of the *Parabacteroides* strain, the culture, or the microbial agent.

A fifth aspect of the embodiments of the present application relates to a pharmaceutical composition, including: C1) the *Parabacteroides* strain, metabolites of the *Parabacteroides* strain, the culture, or the microbial agent; and C2) a drug for combination therapy. Through the combination therapy, the therapeutic efficacy can be enhanced. In some embodiments, the drug for combination therapy includes at least one of an anti-tumor drug, an anti-metabolic disease drug, an anti-inflammatory or autoimmune disease drug, an anti-infectious disease drug, and an anti-central nervous system disease drug. In some embodiments, the anti-tumor drug can include at least one of a chemotherapeutic drug, a photosensitizer, a photothermal agent, and an immunotherapeutic drug.

The immunotherapeutic drug includes at least one of an immune cell therapy drug and an immune checkpoint inhibitor. The immune cell therapy drug includes at least one of a T cell therapy drug (such as chimeric antigen receptor T-cell therapy (CAR-T) and T-cell receptor T-cell therapy (TCR-T)), a tumor-infiltrating lymphocyte therapy drug, and an NK cell therapy drug. The immune checkpoint inhibitor includes an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA.

The term "immune checkpoint inhibitor" refers to an antagonist targeting a corresponding immune checkpoint protein. The immune checkpoint inhibitor can enhance a protein stimulating an immune response or block a protein inhibiting the immune response, thereby exerting an anti-cancer effect through the immune response. In some embodiments, the immune checkpoint inhibitor may be a protein or peptide, such as a soluble fusion protein. The immune checkpoint inhibitor may be an antibody or an antigen-binding fragment thereof that binds to an immune checkpoint protein to be inhibited, or may be an inhibitory nucleic acid (such as siRNA, shRNA, and antisense RNA) that specifically binds to mRNA encoding the immune checkpoint protein.

In some specific embodiments, the immune checkpoint inhibitor is an antibody, for example, an anti-PD-1 antibody (such as Camrelizumab, Cemiplimab (cemiplimab-rwlc), Dostarlimab, Enlonstobart, Nivolumab, Pembrolizumab, Penpulimab, Prolgolimab, Pucotenlimab, Retifanlimab, Serplulimab, Sintilimab, Tislelizumab, Toripalimab, and Zimberelimab), an anti-PD-L1 antibody (such as Adebrelimab, Atezolizumab, Avelumab, Cosibelimab, Durvalumab, Envafolimab, Socazolimab, Sugemalimab, and Tagitanlimab), and an anti-CTLA-4 antibody (such as Ipilimumab and Tremelimumab).

In some embodiments, the anti-metabolic disease drug includes drugs for treating an amino acid metabolism disorder (such as phenylketonuria, a glycine metabolism disorder, and a tyrosine metabolism disorder), an organic acid metabolism disorder (such as methylmalonic acidemia and propionic acidemia), a carbohydrate metabolism disorder (such as glycogen storage disease, galactosemia, and diabetes), a lipid metabolism disorder (such as hypercholesterolemia and hyperlipidemia), a purine metabolism disorder (such as gout and Lesch-Nyhan syndrome), a pigment metabolism disorder (such as porphyria), a blood ammonia metabolism disorder (such as hyperammonemia and citrin deficiency), and a metal ion metabolism disorder (such as Wilson's disease).

With diabetes as an example, an antidiabetic drug includes at least one of insulin, metformin, pramlintide, sulfonylurea (such as glibenclamide, glipizide, and glimepiride), meglitinide (such as repaglinide and nateglinide), thiazolidinedione (such as rosiglitazone and pioglitazone), a DPP-4 inhibitor (such as sitagliptin, saxagliptin, and linagliptin), a GLP-1 receptor agonist (such as exenatide, liraglutide, and semaglutide), an SGLT2 inhibitor (such as canagliflozin, dapagliflozin, and empagliflozin), entecavir, tenofovir, lamivudine, adefovir, telbivudine, simeprevir, sofosbuvir, interferon (IFN), and ribavirin.

In some embodiments, the pharmaceutical composition further includes a package insert.

In some embodiments, the pharmaceutical composition is in an infant-suitable dosage form, a child-suitable dosage form, or an adult-suitable dosage form, or the pharmaceutical composition is in a dosage form for enteral administration or a dosage form for parenteral administration, or the pharmaceutical composition is an oral preparation or an injection. The *Parabacteroides* strain in the pharmaceutical composition can at least partially proliferate in a gut of a subject. The *Parabacteroides* strain may be present in the pharmaceutical composition in a form of live bacteria, attenuated bacteria, killed bacteria, lyophilized bacteria, or irradiated bacteria.

In some embodiments, the pharmaceutical composition includes 1 × 10³ CFU/g to 1 × 10¹³ CFU/g of the *Parabacteroides* strain, that is, each gram of the pharmaceutical composition includes 1 × 10³ to 1 × 10¹³ CFU of the *Parabacteroides* strain. In some other embodiments, the pharmaceutical composition includes 1 × 10³ CFU/mL to 1 × 10¹³ CFU/mL of the *Parabacteroides* strain, that is, each milliliter of the pharmaceutical composition includes 1 × 10³ to 1 × 10¹³ CFU of the *Parabacteroides* strain. For example, each gram or each milliliter of the pharmaceutical composition includes at least 1 × 10³, 2 × 10³, 5 × 10³, 1 × 10⁴, 2 × 10⁴, 5 × 10⁴, 1 × 10⁵, 2 × 10⁵, 5 × 10⁵, 1 × 10⁶, 2 × 10⁶, 5 × 10⁶, 1 × 10⁷, 2 × 10⁷, 5 × 10⁷, 1 × 10⁸, 2 × 10⁸, 5 × 10⁸, 1 × 10⁹, 2 × 10⁹, 5 × 10⁹, 1 × 10¹⁰, 2 × 10¹⁰, 5 × 10¹⁰, 1 × 10¹¹, 2 × 10¹¹, 5 × 10¹¹, 1 × 10¹², 2 × 10¹², 5 × 10¹², or 1 × 10¹³ CFU of the *Parabacteroides* strain.

In some embodiments, the pharmaceutical composition or a raw material for the pharmaceutical composition includes the C1) in a mass percentage of 1% or more. In some embodiments, the pharmaceutical composition or the raw material for the pharmaceutical composition includes the C1) in a mass percentage of 1% to 80%, 2% to 70%, 5% to 60%, 10% to 50%, 20% to 40%, or 40% to 60%.

In some embodiments, the *Parabacteroides* strain in the pharmaceutical composition is capable of at least partially proliferating in a gut of a subject. For example, at least 1% to 80%, 2% to 70%, 5% to 60%, 10% to 50%, 20% to 40%, or 40% to 60% of the *Parabacteroides* strain is capable of proliferating in the gut of the subject.

In some embodiments, the *Parabacteroides* strain in the pharmaceutical composition is lyophilized. In some embodiments, the *Parabacteroides* strain in the pharmaceutical composition is spray-dried. In some embodiments, the *Parabacteroides* strain in the pharmaceutical composition is lyophilized or spray-dried and remains viable. In some embodiments, the *Parabacteroides* strain in the pharmaceutical composition is lyophilized or spray-dried and is capable of partially or completely colonizing a gut. In some embodiments, a lyophilized *Parabacteroides* strain is reconstituted before administration. In some cases, the reconstitution is conducted with a diluent.

In some embodiments, the pharmaceutical composition is administered orally. Oral administration may involve swallowing such that a compound enters the gastrointestinal tract, and/or may be buccal, lingual, or sublingual administration such that a compound directly enters the bloodstream through the oral cavity. Dosage forms suitable for the oral administration include a solid suppository, a solid microparticle, a semi-solid, and a liquid (including a multiphase or dispersed system), such as a tablet; a soft or hard capsule including multiparticulates or nanoparticles, a liquid (such as an aqueous solution), an emulsion, or a powder; a lozenge (including a liquid filler); a chewable gel; a rapidly dispersing dosage form; an ovule; a spray; and a buccal/mucosal adhesive patch.

In some embodiments, the pharmaceutical composition is an enteric formulation, namely, a gastric fluid-resistant preparation (for example, tolerant to gastric pH) suitable for the delivery of the pharmaceutical composition to a gut through oral administration. When the *Parabacteroides* strain or another component of the pharmaceutical composition is acid-sensitive, for instance, prone to degradation under gastric conditions, the enteric formulation may be particularly useful.

In some embodiments, the enteric formulation includes an enteric coating. In some embodiments, the enteric formulation is in an enteric-coated dosage form. For example, the enteric formulation may be an enteric tablet or an enteric capsule. The enteric coating may be a conventional enteric coating, such as conventional coatings of tablets, capsules, and similar dosage forms for oral delivery. The enteric formulation may include a film coating, for example, a film layer of an enteric polymer, such as an acid-insoluble polymer. In some embodiments, the enteric formulation is intrinsically enteric, for instance, the enteric formulation is intrinsically gastroresistant without requiring an enteric coating. In some embodiments, the enteric formulation is an intrinsically enteric capsule (for example, from Capsugel).

In some embodiments, a dosage form of the pharmaceutical composition is any one of a powder, a granule, a tablet, a capsule, a gel, a suspension, a drop, a dropping pill, an injection, a suppository, an aerosol, an oral liquid, an ointment, an emulsion, and a lavage solution. The *Parabacteroides* strain or the culture of the *Parabacteroides* strain can be prepared into the aforementioned pharmaceutical compositions to achieve the different release requirements of the active ingredient, such as quick release or sustained release.

A sixth aspect of the embodiments of the present application relates to a pharmaceutical combination product, including the following independent components:
a first product including the *Parabacteroides* strain, metabolites of the *Parabacteroides* strain, the culture, or the microbial agent; and
a second product including a drug for combination therapy.

It can be understood that the first product includes an effective amount of the *Parabacteroides* strain or the culture thereof. The first product and the second product may be administered separately at respective doses. In this case, the first product and the second product may be administered at different time points and/or through different administration routes and/or at different administration intervals.

In some embodiments, the drug for combination therapy in the second product includes at least one of an anti-tumor drug, an anti-metabolic disease drug, an anti-inflammatory or autoimmune disease drug, an anti-infectious disease drug, and an anti-central nervous system disease drug. In some embodiments, the anti-tumor drug can include at least one of a chemotherapeutic drug, a photosensitizer, a photothermal agent, and an immunotherapeutic drug.

The chemotherapeutic drug includes at least one of an alkylating agent (such as cyclophosphamide, ifosfamide, thiotepa, melphalan, carmustine, lomustine, semustine, nimustine, fotemustine, estramustine, formylmerphalan, chlormethine, nitrocaphane, nitrogen mustard of phthalic acid, chlorambucil, altretamine, busulfan, and temozolomide), a platinum-based agent (such as cisplatin, carboplatin, oxaliplatin, heptaplatin, lobaplatin, nedaplatin, and dicycloplatin), a podophyllotoxin-based agent (such as podophyllotoxin, etoposide, teniposide, and etoposide phosphate), a camptothecin-based agent (such as camptothecin, hydroxycamptothecin, irinotecan, and topotecan), a taxane (such as paclitaxel and docetaxel), an anthracycline (such as daunorubicin, doxorubicin, epirubicin, pirarubicin, idarubicin, mitoxantrone, and aclarubicin), a fluoropyrimidine (such as gemcitabine, capecitabine, 5-fluorouracil, tegadifur, doxifluridine, tegafur, carmofur, and trifluridine), and an antibiotic (such as mitomycin, bleomycin, and pingyangmycin).

The photosensitizer includes at least one of a porphyrin (such as chlorin, phthalocyanine, and bacteriochlorin), boron dipyrromethene (such as BODIPY and aza-BODIPY), and rose bengal.

The photothermal agent includes at least one of a noble metal nanoparticle (such as a nanodot, a nanorod, a nanowire, and a nanosheet of gold, silver, platinum, palladium, etc.), an organic polymer (such as polypyrrole, polyaniline, poly(3,4-ethylenedioxythiophene), polystyrene sulfonate, indocyanine green, and a porphyrin), a carbon-based nanomaterial (such as graphene, a carbon nanotube, and a carbonized cellulose product), a magnetic nanomaterial (such as Fe₃O₄), and a semiconductor nanomaterial (such as copper sulfide, molybdenum sulfide, bismuth sulfide, antimony sulfide, gold sulfide, copper selenide, molybdenum selenide, bismuth selenide, antimony selenide, and gold selenide).

The immunotherapeutic drug includes at least one of an immune cell therapy drug and an immune checkpoint inhibitor. The immune cell therapy drug includes at least one of a T cell therapy drug (such as CAR-T and TCR-T), a tumor-infiltrating lymphocyte therapy drug, and an NK cell therapy drug. The immune checkpoint inhibitor includes an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA.

In some embodiments, the central nervous system disease includes at least one of stroke, schizophrenia, drug addiction, Alzheimer's disease, Parkinson's disease, anxiety disorder, and depression.

In some embodiments, the *Parabacteroides* strain, the culture, the microbial agent, the composition, the pharmaceutical composition, or the pharmaceutical combination product prevents and/or treats a tumor through at least one of the following (a) to (l): (a) inhibiting an increase in tumor volume; (b) inhibiting an increase in tumor weight; (c) inhibiting proliferation of a tumor cell; (d) enhancing a response rate of a tumor treatment; (e) enhancing therapeutic efficacy of an immune checkpoint inhibitor, including enhancing therapeutic efficacy of an anti-PD-1 antibody; (f) preventing and/or inhibiting dissemination or metastasis of a tumor cell; (g) reducing resistance to an anti-tumor drug; (h) inhibiting an activity of histone deacetylase (HDAC) through at least one of SCFAs including acetic acid or acetate, propionic acid or propionate, butyric acid or butyrate, and valeric acid or valerate; (i) inhibiting an activity of HDAC and/or promoting a transcriptional activity of interferon beta (IFN-β); (j) regulating or activating an immune system of a subject; (k) promoting senescence or apoptosis of a tumor cell; and (l) inhibiting angiogenesis within a tumor tissue.

In some embodiments, the *Parabacteroides* strain, the culture, the microbial agent, the composition, the pharmaceutical composition, or the pharmaceutical combination product treats or prevents a disease (such as a tumor or cancer, a metabolic disease, an inflammatory or autoimmune disease, an infectious disease, or a central nervous system disease) by inhibiting an activity of HDAC and/or enhancing a transcriptional activity of IFN-β.

Current research results show that HDAC inhibitors exhibit significant anti-tumor effects against tumor cells at a site such as bladder, bone, breast, uterus, central nervous system, esophagus, lung, ovary, pancreas, cholangiocarcinoma, and prostate, and can induce remarkable apoptosis, proliferation inhibition, and cell cycle arrest for such tumor cells. As HDACs work like oncogenes to regulate the onset of cancers, the reduction or inhibition of activities of HDACs has been proved to enable various anti-cancer effects. Moreover, trichostatin A (TSA) can inhibit tumor angiogenesis by down-regulating the expression of tumor angiogenesis-associated genes induced by hypoxia and by directly suppressing the migration and proliferation of endothelial cells. HDAC inhibitors can also treat diabetes through a variety of mechanisms, including inhibition of Pdxl (Park et al., 2008, J Clin Invest, 118, 2316-24), enhancement of the expression of a transcription factor Ngn3 to expand the pool of endocrine progenitor cells (Haumaitre et al., 2008, Mol Cell Biol, 28, 6373-83), enhancement of the expression of insulin (Molsey et al., 2003, J Biol Chem, 278, 19660-6), etc. The HDAC inhibition is also a promising therapeutic approach for advanced diabetes-associated complications such as diabetic nephropathy and retinal ischemia (Christensen et al., 2011, Mol Med, 17 (5-6), 370-390). Therefore, the *Parabacteroides* strain, the culture, the microbial agent, the composition, the pharmaceutical composition, or the pharmaceutical combination product treats or prevents a disease (such as a tumor or cancer, a metabolic disease, an inflammatory or autoimmune disease, an infectious disease, or a central nervous system disease) by inhibiting an activity of HDAC.

In some embodiments, the *Parabacteroides* strain, the culture, the microbial agent, the composition, the pharmaceutical composition, or the pharmaceutical combination product treats or prevents a disease (such as a tumor or cancer, a metabolic disease, an inflammatory or autoimmune disease, an infectious disease, or a central nervous system disease) by up-regulating a pro-inflammatory cytokine and a chemokine to enable immune stimulation and/or by down-regulating an anti-inflammatory factor to enable immunomodulation. In some embodiments, the pro-inflammatory cytokine is at least one of IFN-γ, IFN-β, IL-1β, IL-6, IL-1β, MCP-1, MIG, RANTES, and TNF-α.

An eighth aspect of the embodiments of the present application relates to use of the *Parabacteroides* strain, the culture, the microbial agent, the composition, the pharmaceutical composition, or the pharmaceutical combination product in preventing and/or treating a disease. In some embodiments, the disease includes at least one of a tumor or cancer, a metabolic disease, an inflammatory or autoimmune disease, an infectious disease, and a central nervous system disease.

In some embodiments, the *Parabacteroides* strain, the culture, the microbial agent, the composition, the pharmaceutical composition, or the pharmaceutical combination product prevents and/or treats a tumor through at least one of the following (a) to (l): (a) inhibiting an increase in tumor volume; (b) inhibiting an increase in tumor weight; (c) inhibiting proliferation of a tumor cell; (d) enhancing a response rate of a tumor treatment; (e) enhancing therapeutic efficacy of an immune checkpoint inhibitor, including enhancing therapeutic efficacy of an anti-PD-1 antibody; (f) preventing and/or inhibiting dissemination or metastasis of a tumor cell; (g) reducing resistance to an anti-tumor drug; (h) inhibiting an activity of HDAC through at least one of SCFAs including acetic acid or acetate, propionic acid or propionate, butyric acid or butyrate, and valeric acid or valerate; (i) inhibiting an activity of HDAC and/or promoting a transcriptional activity of IFN-β; (j) regulating or activating an immune system of a subject; (k) promoting senescence or apoptosis of a tumor cell; and (l) inhibiting angiogenesis within a tumor tissue.

In some embodiments, the *Parabacteroides* strain, the culture, the microbial agent, the composition, the pharmaceutical composition, or the pharmaceutical combination product treats or prevents a disease (such as a tumor or cancer, a metabolic disease, an inflammatory or autoimmune disease, an infectious disease, or a central nervous system disease) by inhibiting an activity of HDAC and/or enhancing a transcriptional activity of IFN-β.

In some embodiments, the *Parabacteroides* strain, the culture, the microbial agent, the composition, the pharmaceutical composition, or the pharmaceutical combination product treats or prevents a disease (such as a tumor or cancer, a metabolic disease, an inflammatory or autoimmune disease, an infectious disease, or a central nervous system disease) by up-regulating a pro-inflammatory cytokine and a chemokine to enable immune stimulation and/or by down-regulating an anti-inflammatory factor to enable immunomodulation. In some embodiments, the pro-inflammatory cytokine is at least one of IFN-γ, IFN-β, IL-1β, IL-6, IL-1β, MCP-1, MIG, RANTES, and TNF-α.

A ninth aspect of the embodiments of the present application relates to a method for treating a disease, including administering an effective dose of the *Parabacteroides* strain, the culture, the microbial agent, the composition, the pharmaceutical composition, or the pharmaceutical combination product to a subject.

In some embodiments, the disease includes at least one of a tumor or cancer, a metabolic disease, an inflammatory or autoimmune disease, an infectious disease, and a central nervous system disease. The tumor or cancer includes at least one of a solid tumor, a soft tissue tumor, a hematopoietic tumor, a glandular tumor, and a metastatic tumor. In some embodiments, preventing and/or treating a tumor is achieved through at least one of the following (a) to (o): (a) inhibiting an increase in tumor volume; (b) inhibiting an increase in tumor weight; (c) inhibiting proliferation of a tumor cell; (d) enhancing a response rate of a tumor treatment; (e) enhancing therapeutic efficacy of an immune checkpoint inhibitor, including enhancing therapeutic efficacy of an anti-PD-1 antibody; (f) preventing and/or inhibiting dissemination or metastasis of a tumor cell; (g) reducing resistance to an anti-tumor drug; (h) inhibiting an activity of HDAC through at least one of SCFAs or salts thereof; (i) inhibiting an activity of HDAC and/or promoting a transcriptional activity of IFN-β; (j) regulating or activating an immune system of a subject; (k) promoting senescence or apoptosis of a tumor cell; (l) inhibiting angiogenesis within a tumor tissue; (m) enhancing an effect of a tumor-killing immune cell; (n) local tumor immunotherapy; and (o) relieving immunosuppression in a tumor microenvironment.

In some embodiments, the *Parabacteroides* strain, the culture, the microbial agent, the composition, the pharmaceutical composition, or the pharmaceutical combination product treats or prevents a disease (such as a tumor or cancer, a metabolic disease, an inflammatory or autoimmune disease, an infectious disease, or a central nervous system disease) by inhibiting an activity of HDAC and/or enhancing a transcriptional activity of IFN-β.

In some embodiments, the *Parabacteroides* strain, the culture, the microbial agent, the composition, the pharmaceutical composition, or the pharmaceutical combination product treats or prevents a disease (such as a tumor or cancer, a metabolic disease, an inflammatory or autoimmune disease, an infectious disease, or a central nervous system disease) by up-regulating a pro-inflammatory cytokine and a chemokine to enable immune stimulation and/or by down-regulating an anti-inflammatory factor to enable immunomodulation. In some embodiments, the pro-inflammatory cytokine is at least one of IFN-γ, IFN-β, IL-1β, IL-6, IL-1β, MCP-1, MIG, RANTES, and TNF-α.

In some embodiments, the *Parabacteroides* strain, the culture, the microbial agent, the composition, the pharmaceutical composition, or the pharmaceutical combination product is administered at least once daily. In some embodiments, when the administering is conducted twice or more daily, a same dose is administered each time. In some embodiments, when the administering is conducted twice or more daily, a different dose is administered each time. In some embodiments, a route for the administering includes at least one of oral administration, sublingual administration, nasal administration, rectal administration, inhalation administration, transdermal administration, intraperitoneal injection, subcutaneous injection, intradermal injection, intramuscular injection, and intravenous injection.

The term "subject" or "patient" refers to any mammal. A subject or patient described as "in need" refers to a mammal requiring a treatment (or a prevention) of a disease. The term "treating" a disease in a subject or "treating" a subject who has or is suspected of having a disease refers to administering a medication to the subject, for example, administering one or more drugs (for example, including the *Parabacteroides* strain, the culture, the microbial agent, the composition, the pharmaceutical composition, or the pharmaceutical combination product) to reduce or prevent the worsening of at least one symptom of the disease. Therefore, in an embodiment, the "treating" specifically refers to delaying progression, accelerating remission, inducing remission, increasing remission, accelerating recovery, enhancing efficacy of an alternative therapy or reducing resistance to an alternative therapy, or a combination thereof.

The term "prevention" is well known in the art. When used in the context of conditions such as local recurrence, the term "prevention" is widely recognized in the art. The term "prevention" includes the administration of a composition that reduces the occurrence of medical symptoms in a subject or delays the onset of such medical symptoms in contrast to a subject who has not received the composition. Therefore, the prevention of a cancer includes, for example, reducing the number of detectable tumors in a patient population receiving a preventive treatment in contrast to an untreated control population, and/or delaying the occurrence of detectable tumors in a treated population in contrast to an untreated control population, such as a statistically and/or clinically significant amount.

A tenth aspect of the present application also relates to a method for preparing the culture, including the following step: the *Parabacteroides* strain is inoculated into a fermentation medium to produce the fermentation medium in which the *Parabacteroides* strain has been cultured.

In some embodiments, the method further includes the following step: the fermentation medium in which the *Parabacteroides* strain has been cultured is centrifuged to produce the supernatant. In some embodiments, the method further includes the following step: the fermentation medium in which the *Parabacteroides* strain has been cultured or the supernatant is concentrated through at least one of evaporation, lyophilization, dialysis, extraction, and membrane separation to produce the concentrate. In some embodiments, the method further includes the following step: the fermentation medium in which the *Parabacteroides* strain has been cultured or the supernatant is subjected to at least one of extraction and solvent extraction to produce the extract. In some embodiments, the method further includes the following step: any one of the fermentation medium in which the *Parabacteroides* strain has been cultured, the supernatant, the concentrate, and the extract is dried to produce the dried product.

Meanings of some abbreviations involved in the following examples are shown in Table 1 below.

**Table 1 Meanings of some abbreviations**

| Abbreviation | Meaning/full name | Abbreviation | Meaning/full name |
|---|---|---|---|
| IND | Indole test | ESC | Esculin |
| URE | Urease | GLY | Glycerol |
| GLU | Glucose | CEL | Cellobiose |
| MAN | Mannitol | MNE | Mannose |
| LAC | Lactose | MLZ | Melezitose |
| SAC | Sucrose | RAF | Raffinose |
| MAL | Maltose | SOR | Sorbitol |
| SAL | Salicin | RHA | Rhamnose |
| XYL | Xylose | TRE | Trehalose |
| ARA | Arabinose | GEL | Gelatin |

Some media involved in the following examples are as follows:
MM01 liquid medium, including the following components: 5 g/L of peptone, 5 g/L of trypticase, 10 g/L of yeast extract, 5 g/L of beef extract, 5 g/L of glucose, 2 g/L of K₂HPO₄, 2 g/L of sodium acetate, 1 mL/L of Tween 80, 5 mg/L of hemin, 0.5 g/L of L-cysteine hydrochloride, 1 µL/L of vitamin K1, and 8 mL/L of an inorganic salt solution (1 L of the inorganic salt solution includes 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride).
Solid MM01 medium, including the following components: 5 g/L of peptone, 5 g/L of trypticase, 10 g/L of yeast extract, 5 g/L of beef extract, 5 g/L of glucose, 2 g/L of K₂HPO₄, 2 g/L of sodium acetate, 1 mL/L of Tween 80, 5 mg/L of hemin, 0.5 g/L of L-cysteine hydrochloride, 1 µL/L of vitamin K1, 8 mL/L of an inorganic salt solution (1 L of the inorganic salt solution includes 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride), and 15 g/L of agar.
AC liquid medium, including the following components: 20 g/L of peptone, 5 g/L of glucose, 3 g/L of yeast extract, 3 g/L of beef extract, and 0.2 g/L of vitamin C, pH 7.0.
Anaerobic blood agar medium (purchased from Huankai Microbial), including the following components: 10 g/L of pancreatic digest of casein, 3 g/L of pancreatic digest of heart, 1 g/L of corn starch, 5 g/L of peptic digest of meat, 5 g/L of yeast extract, 5 g/L of sodium chloride, 15 g/L of agar, and 50 mL/L to 100 mL/L of sterile defibrinated sheep blood, pH 7.3±0.2.
Columbia blood agar medium (purchased from Huankai Microbial), including the following components: 12 g/L of tryptone, 5 g/L of peptic digest of animal tissue, 1 g/L of corn starch, 3 g/L of beef extract, 3 g/L of yeast extract, 5 g/L of sodium chloride, 13.5 g/L of agar, 0.01 g/L of polymyxin B, 0.01 g/L of nalidixic acid, and 50 mL/L to 100 mL/L of sterile defibrinated sheep blood, pH 7.3±0.2.
Chocolate agar medium (purchased from Huankai Microbial), including the following components: 18 g/L of polypeptone, 1 g/L of starch, 5 g/L of sodium chloride, 15 g/L of agar, and 40 g/L of defibrinated sheep blood, pH 7.4±0.2.
TSB liquid medium, including the following components: 17 g/L of tryptone, 3 g/L of papain digest of soybean meal, 2.5 g/L of dipotassium hydrogen phosphate, 5 g/L of sodium chloride, and 2.5 g/L of glucose, pH 7.3±0.2.
MYPG solid medium, including the following components: 5 g/L of peptone, 3 g/L of malt extract powder, 3 g/L of yeast extract powder, 10 g/L of glucose, and 20 g/L of agar, pH 6.2±0.2.

The aforementioned media can be prepared using conventional preparation methods and sterilization modes.

The above embodiments are illustrated below with reference to specific examples.

### Example 1: Isolation and identification of strains MNH 26621 and MNH 20645

### 1. Isolation of the strains

The strain MNH 26621 was isolated from a fecal sample of a healthy volunteer in Dali City, Yunnan Province, and the strain MNH 20645 was isolated from a fecal sample of a male healthy volunteer in Guangzhou City, Guangdong Province. A specific process was as follows:
(1) 2 g to 5 g of fresh feces was collected from a volunteer in a sample collection and preservation tube, thoroughly shaken for homogenization, then placed in an ice box, and delivered to a laboratory within 24 h for strain isolation.
(2) In a biosafety cabinet, normal saline was dispensed into tubes with 9 mL per tube. A medium for strain isolation was prepared, transferred into an anaerobic workstation (Don Whitley Scientific H35) 24 h in advance, and labeled for sample information, medium type, isolation date, etc.
(3) A fresh fecal sample was placed in the anaerobic workstation, and vortexed by a vortex shaker for 1 min to enable thorough mixing. 1 mL of a resulting sample was taken and added to 9 mL of the normal saline, and thorough mixing was conducted to produce a 10⁻¹ dilution. Then, the 10⁻¹ dilution was serially diluted to produce a 10⁻⁶ dilution for later use.
(4) The 10⁻⁶ dilution was added dropwise on isolation media (anaerobic blood agar plates, Columbia blood agar plates, and chocolate agar plates) at 100 µL/plate, and spread evenly. After a surface of a plate was dried, the plate was inverted and incubated at 37°C for 3 d to 5 d.
(5) A growth status of a strain in an isolation medium was observed, and single colonies were picked using a sterilized toothpick for strain purification. A purified strain was cultured anaerobically at 37°C.
(6) The purified strain was prepared into a 20% glycerol/water-based bacterial suspension and stored at -86°C.

### 2. Identification of the strains

### 2.1 Morphological characteristics

The strain MNH 26621 was inoculated onto an MYPG solid medium plate, cultured anaerobically at 37°C for 48 h, and then observed. An shown in A of FIG. 1, visible circular opaque colonies were formed, with regular and smooth edges and no secretions around the colonies, and these colonies had a diameter of about 2 mm. Additionally, as shown in A of FIG. 2 and A of FIG. 3, microscopic morphology results revealed that this strain did not have spores or flagella, was non-motile, short rod-shaped, and Gram-negative, and had a diameter of approximately (3-5) µm × (8-15) µm.

The strain MNH 20645 was inoculated onto an MYPG solid medium plate, cultured anaerobically at 37°C for 48 h, and then observed. An shown in B of FIG. 1, visible circular opaque colonies were formed, with regular and smooth edges and no secretions around the colonies. Additionally, as shown in B of FIG. 2 and B of FIG. 3, microscopic morphology results revealed that this strain did not have spores or flagella, was non-motile, rod-shaped, and Gram-negative, and had a diameter of approximately 5 µm × (10-20) µm.

### 2.2 Physiological and biochemical characteristics

The strain MNH 26621 had a growth temperature range of 30°C to 42°C and an optimal growth temperature of 37°C. As shown in A of FIG. 4 to FIG. 6, the strain MNH 26621 could grow within a pH range of 7.0 to 10.0, with an optimal growth pH of 7.0 to 9.0. The strain MNH 26621 could tolerate up to 2% NaCl. The strain MNH 26621 could survive and grow at a bile salt concentration ranging from 0% to 0.10%, but failed to grow at a bile salt concentration of 0.15% or higher. Moreover, the strain MNH 26621 could not grow under aerobic conditions, but grew well under anaerobic conditions. Thus, the strain MNH 26621 was an obligate anaerobic strain.

The strain MNH 20645 had a growth temperature range of 30°C to 42°C and an optimal growth temperature of 37°C. As shown in B of FIG. 4 to FIG. 6, the strain MNH 20645 was sensitive to pH, with an optimal growth pH of approximately 7.2 (a medium used at 0% had a pH of approximately 7.2 in FIG. 5 to FIG. 6). The strain MNH 20645 could tolerate up to 2% NaCl, was intolerant to bile salts, and failed to grow at a bile salt concentration of 0.1% or higher. Moreover, the strain MNH 20645 could survive for 4 h under aerobic conditions with a survival rate of approximately 80%, and grew well under anaerobic conditions.

### API 20A test

The API 20A test was conducted using an API 20A test strip (BioMérieux) according to instructions. A strain was cultured anaerobically at 37°C. Test results were shown in Table 2.

**Table 2 API 20A test results for the strains MNH 26621 and MNH 20645**

| Test item | MNH 26621 test result | MNH 20645 test result | Test item | MNH 26621 test result | MNH 20645 test result |
|---|---|---|---|---|---|
| IND | No acid production | No acid production | ESC | No acid production | No acid production |
| URE | No acid production | No acid production | GLY | No acid production | No acid production |
| GLU | Acid production | Acid production | CEL | No acid production | No acid production |
| MAN | No acid production | No acid production | MNE | Acid production | Acid production |
| LAC | Acid production | Acid production | MLZ | No acid production | No acid production |
| SAC | Acid production | Acid production | RAF | Acid production | Acid production |
| MAL | No acid production | No acid production | SOR | No acid production | No acid production |
| SAL | No acid production | No acid production | RHA | Acid production | Acid production |
| XYL | Acid production | Acid production | TRE | No acid production | No acid production |
| ARA | Acid production | Acid production | GEL | No acid production | No acid production |

According to the results in this table, both the strain MNH 26621 and the strain MNH 20645 can utilize glucose, lactose, sucrose, xylose, arabinose, mannose, raffinose, and rhamnose as substrates for metabolism to produce acids, but cannot utilize other types of substrates.

### Antibiotic sensitivity test

The strain MNH 26621 was subjected to the antibiotic sensitivity test by the disk diffusion method, and test results were shown in Table 3.

**Table 3 Antibiotic sensitivity test results for the strains MNH 26621 and MNH 20645**

| Antibiotic | Inhibition zone diameter of MNH 26621 (mm) | Inhibition zone diameter of MNH 20645 (mm) |
|---|---|---|
| Ampicillin (AMP) | 35.77 | 11.47 |
| Sulfamethoxazole/trimethoprim (SXT) | 0 | 0 |
| Erythromycin (E) | 20.64 | 38.01 |
| Ciprofloxacin (CIP) | 21.4 | 0 |
| Lincomycin (MY) | 15.79 | 6.45 |
| Chloramphenicol (C) | 20.15 | 20.38 |
| Penicillin (PEN) | 7.35 | 14.56 |
| Gentamicin (GEN) | 14.75 | 0 |
| Tetracycline (TET) | 12.82 | 12.19 |
| Ceftriaxone (CTR) | 25.6 | 21.02 |

According to the results in Table 3, the strain MNH 26621 is sensitive to antibiotics including AMP, E, CIP, MY, C, PEN, GEN, TET, and CTR, but is resistant to SXT. The strain MNH 20645 is sensitive to antibiotics including E, C, TET, PEN, AMP, MY, and CTR, but is resistant to antibiotics including SXT, CIP, and GEN.

### 2.3 16S rRNA

Fresh cultures of the strains MNH 26621 and MNH 20645 were collected and subjected to genomic DNA extraction by the sodium dodecyl sulfate (SDS) method. DNA was then tested by agarose gel electrophoresis for purity and integrity, and quantified by Qubit.

With the extracted genomic DNA as a template, 16S rRNA was amplified using the following primers:
1492R: AGAGTTTGATCATGGCTCAG (SEQ ID NO: 3) and
27F: TAGGGTTACCTTGTTACGACTT (SEQ ID NO: 4).

A PCR amplification product was purified and then sequenced using ABI3730XL. A 16S rRNA sequence of 1,422 bp was acquired from the strain MNH 26621, which was as follows:

CATGGAGGCTTACACATGCAAGTCGAGGGGCATCGGGGTGTAGCAATACATTGCCG
GCGACCGGCGCACGGGTGAGTAACGCGTATGCAACTTACCTATCAGAGGGAATAGC
CCGGCGAAAGTCGGATTAATGCCCCATAAAACAGGGGTCCCGCATGGGACTATTTGTT
AAAGATTCATCGCTGATAGATAGGCATGCGTTCCATTAGGCAGTTGGCGGGGTAACGG
CCCACCAAACCGACGATGGATAGGGGTTCTGAGAGGAAGGTCCCCCACATTGGAACT
GAGACACGGTCCAAACTCCTACGGGAGGCAGCAGTGAGGAATATTGGTCAATGGAC
GTGAGTCTGAACCAGCCAAGTCGCGTGAAGGAAGAAGGTTCTATGGATTGTAAACTT
CTTTTATACGGGAATAAAGTGCACCACGTGTGGTGTTTTGTATGTAcCGTATGAATAAG
CATCGGCTAACTCCGTGCCAGCAGCCGCGGTAATACGGAGGATGCGAGCGTTATCCG
GATTTATTGGGTTTAAAGGGTGCGTAGGTTGTTTTTAAGTCAGCGGTGAAAGTTTGT
GGCTCAACCATAAAATTGCCGTTGAAACTGGAAGACTTGAGTGTGTTTGAGGTAGGC
GGAATGCGTGGTGTAGCGGTGAAATGCATAGATATCACGCAGAACTCCGATTGCGAA
GGCAGCTTACTAAACCATAACTGACACTGAAGCACGAAAGCGTGGGGATCAAACAG
GATTAGATACCCTGGTAGTCCACGCAGTAAACGATGATTACTAGGAGTTTGCGATAGA
CAGTAAGCTCTACAGCGAAAGCGTTAAGTAATCCACCTGGGGAGTACGCCGGCAACG
GTGAAACTCAAAGGAATTGACGGGGGCCCGCACAAGCGGAGGAACATGTGGTTTAA
TTCGATGATACGCGAGGAACCTTACCCGGGTTTGAACGTAGTCGGACCGGCCTTGAA
AGAGGCCTTCTAGCAaTAGCTGATTACGAGGTGCTGCATGGTTGTCGTCAGCTCGTGC
CGTGAGGTGTCGGCTTAAGTGCCATAACGAGCGCAACCCTTATCACTAGTTACTAACA
GGTAAAGCTGAGGACTCTGGTGAGACTGCCAGCGTAAGCTGTGAGGAAGGTGGGGA
TGACGTCAAATCAGCACGGCCCTTACATCCGGGGCGACACACGTGTTACAATGGCAT
GGACAAAGGGCAGCTACACAGCGATGTGATGCTAATCTCCAAACCATGTCTCAGTTC
GGATCGGAGTCTGCAACTCGACTCCGTGAAGCTGGATTCGCTAGTAATCGCGCATCA
GCCATGGCGCGGTGAATACGTTCCCGGGCCTTGTACACACCGCCCGTCAAGCCATGG
GAGCCGGGGGTACCTGAAGTCCGTAACCGCGAGGATCGGCCTAGGGAAAA (SEQ ID
NO: 1).

The 16S rRNA sequence was aligned with the National Center for Biotechnology Information (NCBI) Nucleotide collection (nr/nt) database, and the closest species was *Parabacteroides merdae* with a similarity of 96.34%. Accordingly, it was preliminarily determined that the strain MNH 26621 belonged to the genus *Parabacteroides* and was a novel species.

A 16S rRNA sequence of 1,406 bp was acquired from the strain MNH 20645, which was as follows:

GGCTTACACAGTGCAAGTCTGAGGGGCAGCGGGGGTAGCAATACCTGCCGGCGAC
CGGCGCACGGGTGAGTAACGCGTATGCAACTTGCCTATCAGAGGGGAATAGCCCGGC
GAAAGTCGGATTAATGCTCCATAAAACAGGGGTCCCGCATGGGAATATTTGTTAAAGA
TTCATCGCTGATAGATAGGCATGCGTTCCATTAGGCAGTTGGCGGGGTAACGGCCCAC
CAAACCGACGATGGATAGGGGTTCTGAGAGGAAGGTCCCCCACATTGGAACTGAGA
CACGGTCCAAACTCCTACGGGAGGCAGCAGTGAGGAATATTGGTCAATGGCCGGGAG
GCTGAACCAGCCAAGTCGCGTGAAGGATGAAGGATCTATGGTTTGTAAACTTCTTTTA
TACGGGAATAAAGTGTGGGACGTGTCCCATTTTGTATGTACCGTATGAATAAGCATCG
GCTAACTCCGTGCCAGCAGCCGCGGTAATACGGAGGATGCGAGCGTTATCCGGATTTA
TTGGGTTTAAAGGGTGCGTAGGTGGTTGTTTAAGTCAGCGGTGAAAGTTTGTGGCTC
AACCATAAAATTGCCGTTGAAACTGGATGACTTGAGTATATTTGAGGTAGGCGGAATG
CGTGGTGTAGCGGTGAAATGCATAGATATCACGCAGAACTCCGATTGCGAAGGCAGC
TTACTAAACTATAACTGACACTGAAGCACGAAAGCGTGGGGATCAAACAGGATTAGA
TACCCTGGTAGTCCACGCAGTAAACGATGATTACTAGGAGTTTGCGATAGACAGTAAG
CTCTACAGCGAAAGCGTTAAGTAATCCACCTGGGGAGTACGCCGGCAACGGTGAAAC
TCAAAGGAATTGACGGGGGCCCGCACAAGCGGAGGAACATGTGGTTTAATTCGATGA
TACGCGAGGAACCTTACCCGGGTTTGAACGCATTCGGACCGGAGTGGAAaCGCTCCT
TTCAGCAATGACCGTTTGCGAGgTGCTGCATGgtTGTCGTCAGCTCGTGCCGTGAGgTG
TCGGCTTAAGTGCCATAaCGAGCGCAACCCTTATCACTAGTTACTAACAGGTCAAGCT
GAGGACTCTGGTGAGACTGCCAGCGTAAGCTGTGAGGAAGGTGGGGATGACGTCAA
ATCAGCACGGCCCTTACATCCGGGGCGACACACGTGTTACAATGGCATGGACAAAGG
GCAGCTACACAGCGATGTGATGCAAATCTCCAAACCATGTCTCAGTTCGGATCGGAG
TCTGCAACTCGACTCCGTGAAGCTGGATTCGCTAGTAATCGCGCATCAGCCATGGCGC
GGTGAATACGTTCCCGGGCCTTGTACACACCGCCCGTCAAGCCATGGAGCCGGGGG
TACCTGAAGTCCGTAACCGCAAGGATCGGCC (SEQ ID NO: 2).

The 16S rRNA sequence was aligned with the NCBI Nucleotide collection (nr/nt) database, and the closest species was *Parabacteroides hominis* with a similarity of 99.85%. Accordingly, it was preliminarily determined that the strain MNH 20645 was *Parabacteroides hominis*.

The 16S rRNA sequence (SEQ ID NO: 1) of the strain MNH 26621 had an identity of 96.74% to the 16S rRNA sequence (SEQ ID NO: 2) of the strain MNH 20645.

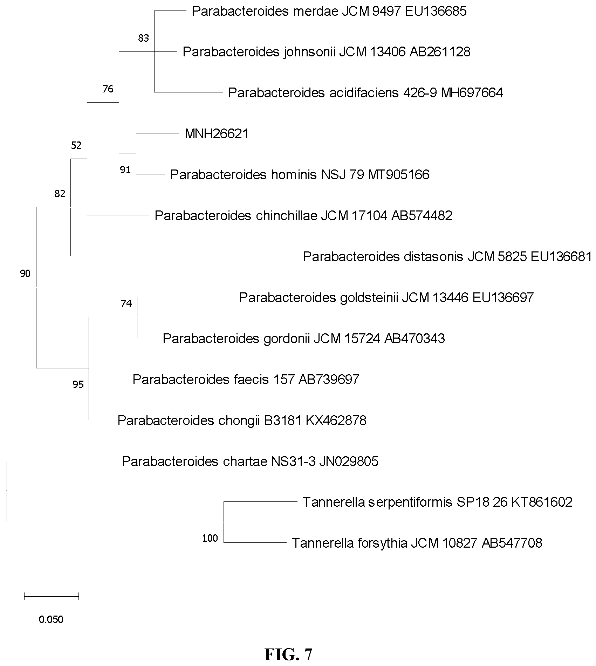

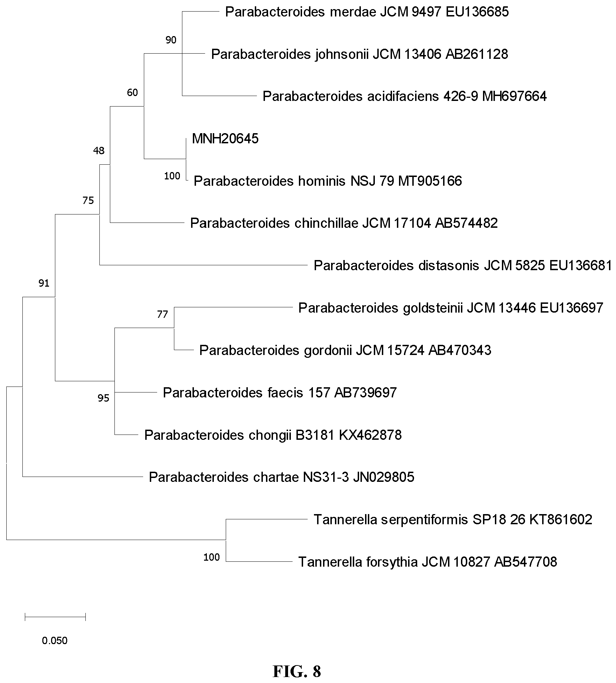

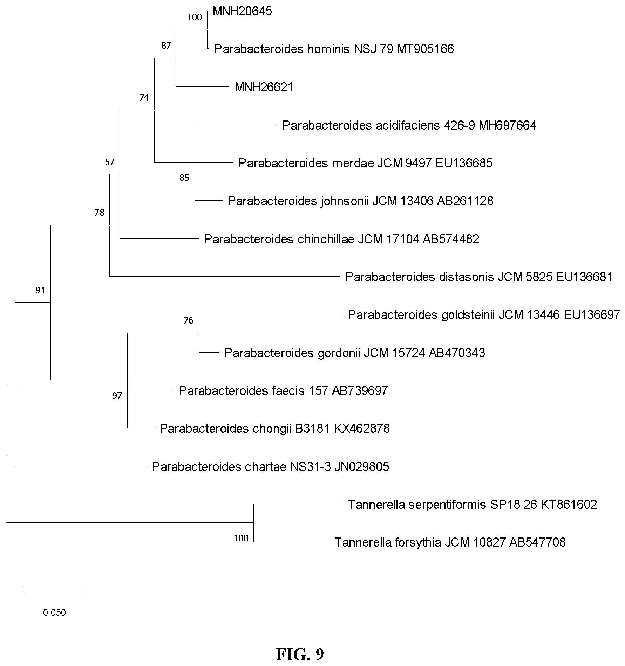

The 16S rRNA sequences of the strains MNH 26621 and MNH 20645 and 16S rRNA sequences of related genera were subjected to multiple sequence alignment by the MUSCLE method. Finally, a phylogenetic tree was constructed using MEGA X by the maximum likelihood method based on 1,000 bootstrap replicates. Based on the phylogenetic trees shown in FIG. 7, FIG. 8, and FIG. 9, the strain MNH 20645 was located in the same clade as *Parabacteroides hominis* NSJ 79 with a short evolutionary distance, whereas the strain MNH 26621 formed a separate clade with a relatively large evolutionary distance from other species.

To further determine ANI values of genomes of the strains MNH 26621 and MNH 20645 with genomes of other members of the genus *Parabacteroides*, the original isolated strains MNH 26621 and MNH 20645 were further subjected to genome preparation, sequencing, assembly, and analysis. Genomic DNA samples from the original isolated strains MNH 26621 and MNH 20645, which had been verified by electrophoresis to be qualified, were fragmented by ultrasonication with a fragment size of approximately 350 bp. Subsequently, an Illumina sequencing library was constructed using a standard DNA library preparation kit (NEB UltraTM). The constructed sequencing library was subjected to paired-end 150 bp sequencing using Illumina NovaSeq. 1.27 Gbp of sequencing data was acquired for the strain MNH 26621, with a Q20 proportion of 97.17%. 1.30 Gbp of sequencing data was acquired for the strain MNH 20645, with a Q20 proportion of 97.04%.

Raw genome sequencing data was filtered using fastp (version: 0.20.0) with the following filtering parameters: "--poly_g_min_len 10--poly_x_min_len 10-q 15-u 40-n 5-l 50". Based on filtered raw data, genome assembly was conducted using SPAdes (version: v3.14.0) with the following assembly parameters: "--isolate--cov-cutoff 10". Genome assembly for the strain MNH 26621 corresponded to a total gene length of 5.06 Mbp, an N50 length of 145.2 kbp, and a GC content of 46.17%. Genome assembly for the strain MNH 20645 corresponded to a total gene length of 5.19 Mbp, an N50 length of 105.3 kbp, and a GC content of 46.27%.

Genomic genes were predicted using the prokaryotic genome annotation software prokka (version: 1.14.5) with the following parameters: "--gcode 11--evalue 1e-09". 1,833 coding sequences (CDSs) were predicted in total for the strain MNH 26621, with an average CDS length of 1,055 bp. 4,192 CDSs were predicted in total for the strain MNH 20645, with an average CDS length of 1,103 bp. Genome information of all type strains within the genus *Parabacteroides* was downloaded from NCBI. A degree of genomic relatedness was determined by evaluating ANI and AF of whole-genome sequences of the strains MNH 26621 and MNH 20645 with whole-genome sequences of reference strains. Alignment results were shown in Table 4 below:

**Table 4 Whole-genome sequence alignment results of the strains MNH 26621 and MNH 20645 with other strains within the genus Parabacteroides**

| | Strain | Ref | ANI | AF |
|---|---|---|---|---|
| MNH 26621 | *Parabacteroides johnsonii* | GCF_000156495.1 | 88.3495 | 69.81818182 |
| | *Parabacteroides merdae* | GCF_000154105.1 | 87.5055 | 65.39393939 |
| | *Parabacteroides massiliensis* | GCF_900155425.1 | 86.3403 | 61.39393939 |
| | *Parabacteroides acidifaciens* | GCF_003363715.1 | 82.9702 | 64.42424242 |
| | *Parabacteroides gordonii* | GCF_000428565.1 | 80.0861 | 34.06060606 |
| | *Parabacteroides faecis* | GCF_014647375.1 | 79.6655 | 31.09090909 |
| | *Parabacteroides bouchesdurhonensis* | GCF_900186615.1 | 79.588 | 17.21212121 |
| | *Parabacteroides goldsteinii* | GCF_000969835.1 | 79.3142 | 34.3030303 |
| | *Parabacteroides timonensis* | GCF_900128505.1 | 79.2889 | 32.72727273 |
| | *Parabacteroides provencensis* | GCF_900232875.1 | 78.8858 | 17.6969697 |
| | *Parabacteroides chinchillae* | GCF_900108035.1 | 78.4272 | 16.96969697 |
| | *Parabacteroides distasonis* | GCF_000012845.1 | 77.9416 | 20.48484848 |
| | *Parabacteroides hominis* | GCF_014287585.1 | 88.8709 | 75.27272727 |
| MNH 20645 | *Parabacteroides merdae* | GCF_000154105.1 | 89.5314 | 64.26872771 |
| | *Parabacteroides johnsonii* | GCF_000156495.1 | 89.2892 | 69.73840666 |
| | *Parabacteroides massiliensis* | GCF_900155425.1 | 88.4055 | 62.00951249 |
| | *Parabacteroides acidifaciens* | GCF_003363715.1 | 83.3652 | 63.07966706 |
| | *Parabacteroides faecis* | GCF_014647375.1 | 79.3267 | 32.81807372 |
| | *Parabacteroides bouchesdurhonensis* | GCF_900186615.1 | 79.2786 | 17.06302021 |
| | *Parabacteroides provencensis* | GCF_900232875.1 | 79.2697 | 16.94411415 |
| | *Parabacteroides goldsteinii* | GCF_000969835.1 | 79.2168 | 35.13674197 |
| | *Parabacteroides gordonii* | GCF_000428565.1 | 79.2144 | 34.42330559 |
| | *Parabacteroides timonensis* | GCF_900128505.1 | 79.1522 | 32.69916766 |
| | *Parabacteroides chinchillae* | GCF_900108035.1 | 78.4865 | 15.51724138 |
| | *Parabacteroides distasonis* | GCF_000012845.1 | 78.2607 | 19.4411415 |
| | *Parabacteroides hominis* | GCF_014287585.1 | 99.9252 | 97.14625446 |

Among the type strains, *Parabacteroides hominis* showed the highest ANI of 88.87% to the strain MNH 26621, with AF of 75.27%. The strain MNH 26621 was subjected to whole-genome sequence alignment with known species. According to alignment results, the strain MNH 26621 could be clearly distinguished from the known species *Parabacteroides johnsonii*, *Parabacteroides merdae*, *Parabacteroides massiliensis*, *Parabacteroides acidifaciens*, *Parabacteroides gordonii*, *Parabacteroides faecis*, *Parabacteroides bouchesdurhonensis*, *Parabacteroides goldsteinii*, *Parabacteroides timonensis*, *Parabacteroides provencensis*, *Parabacteroides chinchillae*, and *Parabacteroides distasonis* based on genotypic characteristics.

Among the type strains, *Parabacteroides hominis* showed the highest ANI of 99.92% with the strain MNH 20645, with AF of 97.14%. Therefore, the strain MNH 20645 could be identified as *Parabacteroides hominis*.

ANI between the genomes of the strains MNH 26621 and MNH 20645 was 88.89%, with AF of 74.55% (using the genome of the strain MNH 20645 as a reference sequence).

ANI between the genomes of the strains MNH 20645 and MNH 26621 was 88.81%, with AF of 73.54% (using the genome of the strain MNH 26621 as a reference sequence).

### 2.4 Genome analysis

Based on genome assembly data acquired above, potential antibiotic resistance genes were analyzed using the Resistance Gene Identifier (RGI) (version: 4.2.2), with the Comprehensive Antibiotic Resistance Database (CARD) (version: 3.0.0, https://card.mcmaster.ca/analyze/rgi) as an antibiotic resistance gene database. Alignment results were shown in Table 5.

**Table 5 Antibiotic resistance gene information of the strains MNH 26621 and MNH 20645**

| Strain gene | Resistance gene | Gene name | Identity (%) |
|---|---|---|---|
| MNH 26621_01561 | ARO:3000191 | tetQ | 96.41 |
| MNH 20645_00602 | ARO:3001302 | chrB | 100 |
| MNH 20645_02466 | ARO:3000191 | tetQ | 99.84 |

Potential virulence factors and related genes in the genomes of the strains were analyzed using NCBI blastp (version: 2.7.1+) in contrast to the Virulence Factor Database (VFDB) (http://www.mgc.ac.cn/cgi-bin/VFs/v5/main.cgi, updated on September 19, 2019). Alignment results were shown in Table 6.

**Table 6 Potential virulence genes of the strains MNH 26621 and MNH 20645**

| Strain gene | VFDB gene | Gene name | Identity (%) |
|---|---|---|---|
| MNH 26621_00822 | VFG001374 | cps4J | 60.983 |
| MNH 26621_00824 | VFG001376 | cps4L | 72.679 |
| MNH 26621_02555 | VFG011430 | acpXL | 63.514 |
| MNH 26621_02753 | VFG001855 | htpB | 61.996 |
| MNH 26621_03220 | VFG048885 | gmd | 60.904 |
| MNH 20645_01048 | VFG011430 | acpXL | 63.514 |
| MNH 20645_01245 | VFG001855 | htpB | 62.572 |
| MNH 20645_01296 | VFG037028 | katA | 69.874 |
| MNH 20645_02496 | VFG048885 | gmd | 61.436 |

Potential primary metabolic gene clusters in the genomes were analyzed using gutSMASH5 (version 1.0.0). Alignment results were shown in Table 7.

**Table 7 Potential primary metabolic gene clusters of the strains MNH 26621 and MNH 20645**

| Strain | Gene cluster region | Type | From | To | The most similar known gene cluster | Abbreviation | Similarity |
|---|---|---|---|---|---|---|---|
| MNH 26621 | Region 4.1 | succinate2propionate | 166829 | 191394 | Succinate to propionate B. theta | PRO | 50% |
| | Region 16.1 | Fumarate2succinate | 56251 | 79730 | Fumarate to succinate B. thetaiotaomicron | SUC | 100% |
| | Region 23.1 | porA | 72990 | 89332 | porA C. sporogenes | POR | 60% |
| | Region 25.1 | Rnf_complex | 56400 | 74347 | Rnf complex C. sporogenes | RNF | 83% |
| | Region 28.1 | Pyruvate2acetate-formate | 25952 | 48927 | Pyruvate to acetate-formate E. coli | PFL_acetate | 100% |
| | Region 58.1 | TPP_AA_metabolism | 1 | 9025 | | | |
| MNH 20645 | Region 1.1 | Fumarate2succinate | 116563 | 140040 | Fumarate to succinate B. thetaiotaomicron | SUC | 100% |
| | Region 2.1 | Rnf_complex | 230896 | 256014 | Rnf complex C. sporogenes | RNF | 83% |
| | Region 5.1 | Arginine2putrescine | 87957 | 115985 | | | |
| | Region 7.1 | TPP_AA_metabolism | 63868 | 89875 | | | |
| | Region 25.1 | porA | 62933 | 84014 | porA C. sporogenes | POR | 60% |
| | Region 65.1 | Pyruvate2acetate-formate | 1 | 10577 | Pyruvate to acetate-formate E. coli | PFL_acetate | 100% |
| | Region 67.1 | succinate2propionate | 1 | 8863 | Succinate to propionate B. theta | PRO | 50% |

### 2.5 Determination of SCFAs

### (1) Preparation of bacterial cells

The strain MNH 26621 was inoculated into a liquid medium and cultured anaerobically at 37°C for 48 h. The bacterial cells were collected through centrifugation and stored at -86°C for later use.

### (2) Preparation of standard solutions

Acetic acid, propionic acid, butyric acid, isobutyric acid, valeric acid, isovaleric acid, and hexanoic acid standards were weighed and prepared with ethyl acetate into eight mixed standard solutions with concentrations of 0.1 µg/mL, 0.5 µg/mL, 1 µg/mL, 5 µg/mL, 10 µg/mL, 20 µg/mL, 50 µg/mL, and 100 µg/mL, respectively.

600 µL of a standard solution was taken, 25 µL of 4-methylvaleric acid was added at a final concentration of 500 µM as an internal standard, and thorough mixing was conducted. A resulting mixture was transferred into a sample vial, and analyzed by gas chromatography-mass spectrometry (GC-MS) with an injection volume of 1 µL and a split ratio of 10:1 (split injection).

### (3) Metabolite extraction

A collected bacterial sample was thawed on ice. 80 mg of a thawed sample was added to a 2 mL glass centrifuge tube, resuspended with 900 µL of 0.5% phosphoric acid, shaken for 2 min, and centrifuged at 14,000 g for 10 min. 800 µL of a resulting supernatant was taken, and an equal amount of ethyl acetate was added for extraction. A resulting mixed solution was vortexed for 2 min, and then centrifuged at 14,000 g for 10 min. 600 µL of a resulting organic phase in an upper layer was collected, 4-methylvaleric acid was added at a final concentration of 500 µM as an internal standard, and thorough mixing was conducted. A resulting test sample was transferred into a sample vial, and analyzed by GC-MS with an injection volume of 1 µL and a split ratio of 10:1 (split injection).

### (4) Sample analysis

A sample was subjected to separation using an Agilent DB-WAX capillary column (30 m × 0.25 mm ID × 0.25 µm) gas chromatography system. Temperature programming: With an initial temperature of 90°C, a temperature was raised at 10°C/min to 120°C, then at 5°C/min to 150°C, and finally at 25°C/min to 250°C, and kept at 250°C for 2 min. A carrier gas was helium at a flow rate of 1.0 mL/min.

Mass spectrometry (MS) analysis was conducted using an Agilent 7890A/5975C GC-MS system. An inlet temperature was 250°C, an ion source temperature was 230°C, a transfer line temperature was 250°C, and a quadrupole temperature was 150°C. An electron impact (EI) ionization source, a full scan mode, and a selected ion monitoring (SIM) scan mode were adopted, with an electron energy of 70 eV.

A chromatographic peak area and a retention time were extracted using MSD ChemStation software. A standard curve was plotted. Contents of SCFAs in the sample were calculated. Results were shown in Table 8.

**Table 8 SCFA yields of the strain MNH 26621**

| | Acetic acid | Isovaleric acid | Propionic acid | Isobutyric acid |
|---|---|---|---|---|
| SCFA yield (µg/g) | 1111.8 | 85.01 | 658.35 | 29.1 |

It can be seen from the results in the table that the strain MNH 26621 provided in this example can produce acetic acid and propionic acid with high yields.

SCFA yields of the strain MNH 20645 were detected by the method described above. Results were shown in Table 9.

**Table 9 SCFA yields of the strain MNH 20645**

| | Acetic acid | Isovaleric acid | Propionic acid | Isobutyric acid |
|---|---|---|---|---|
| SCFA yield (µg/g) | 985.06 | 81.19 | 1118.9 | 38.69 |

It can be seen from the results in the table that the strain MNH 20645 provided in this example can produce acetic acid and propionic acid with high yields.

Based on the morphological, physiological, biochemical, 16S rRNA, and whole-genome analysis results, the strain MNH 26621 exhibits significant differences from other existing strains of the genus *Parabacteroides* and thus can be identified as a novel species of the genus *Parabacteroides*.

The strain MNH 26621 was deposited in the Guangdong Microbial Culture Collection Center (GDMCC) located at the Guangdong Institute of Microbiology, Guangdong Academy of Sciences, No. 100, Xianlie Middle Road, Guangzhou on July 28, 2022, with an accession number GDMCC No: 62664, a taxonomic name of *Parabacteroides* sp., and a deposit name of *Parabacteroides* sp. MNH 26621. It has been confirmed by the Guangdong Microbial Culture Collection Center (GDMCC) that the deposited strain remains viable.

Based on the morphological, physiological, biochemical, 16S rRNA, and whole-genome analysis results, the strain MNH 20645 can be identified as *Parabacteroides hominis*.

The strain MNH 20645 was deposited in the Guangdong Microbial Culture Collection Center (GDMCC) located at the Guangdong Institute of Microbiology, Guangdong Academy of Sciences, No. 100, Xianlie Middle Road, Guangzhou on July 28, 2022, with an accession number GDMCC No: 62665, a taxonomic name of *Parabacteroides* sp., and a deposit name of *Parabacteroides* sp. MNH 20645. It has been confirmed by the Guangdong Microbial Culture Collection Center (GDMCC) that the deposited strain remains viable.

### Example 2: Regulation of the immune activity of macrophages by the strains

THP-1 cells (Wuhan Pricella Biotechnology Co., Ltd.) were treated with phorbol 12-myristate 13-acetate (PMA) at a final concentration of 5 ng/mL for 48 h to enable the differentiation into M0 macrophages. The M0 macrophages were co-incubated with a strain (MNH 26621 or MNH 20645) for 24 h. The strain was added at a multiplicity of infection (MOI) of 10:1 (viable cell count : total cell count). A control group of M1 macrophages was set (M0 macrophages were induced with 20 ng/mL of IFN-γ and 10 pg/mL of lipopolysaccharide (LPS) for 24 h). The macrophages were anaerobically cultured for 1 h. A mixture of antibiotics (1 mg/mL of AMP, 5 mg/mL of streptomycin, and 1 mg/mL of colistin) was added to kill the bacteria, and the macrophages were further cultured for 23 h. A resulting supernatant was then collected.

With the BD^{™} Cytometric Bead Array (CBA) Human Soluble Protein Master Buffer Kit, the supernatant was allowed to react with detection proteins according to instructions, and concentrations of cytokines such as IL-10, IL-6, IL-8, IL-1β, IL-12/IL-13P40, MCP-1, and RANTES in the supernatant were then detected by flow cytometry.

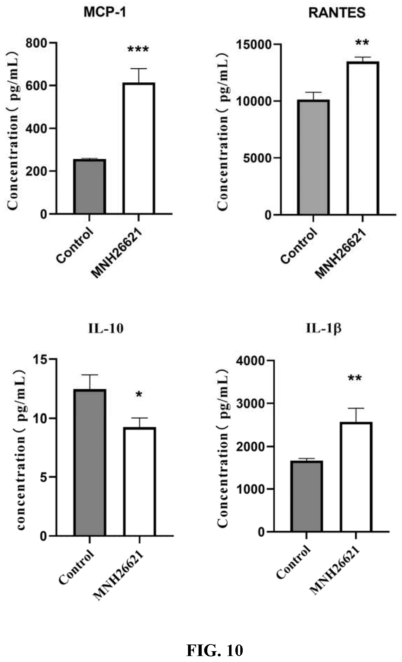

Results for the strain MNH 26621 were shown in FIG. 10. After being co-cultured with the strain MNH 26621, the macrophages exhibited the significant characteristic of differentiation into M1 macrophages. A level of the anti-inflammatory cytokine IL-10 was significantly down-regulated, while levels of the pro-inflammatory cytokine IL-1β and the chemokines RANTES and MCP-1 were significantly up-regulated.

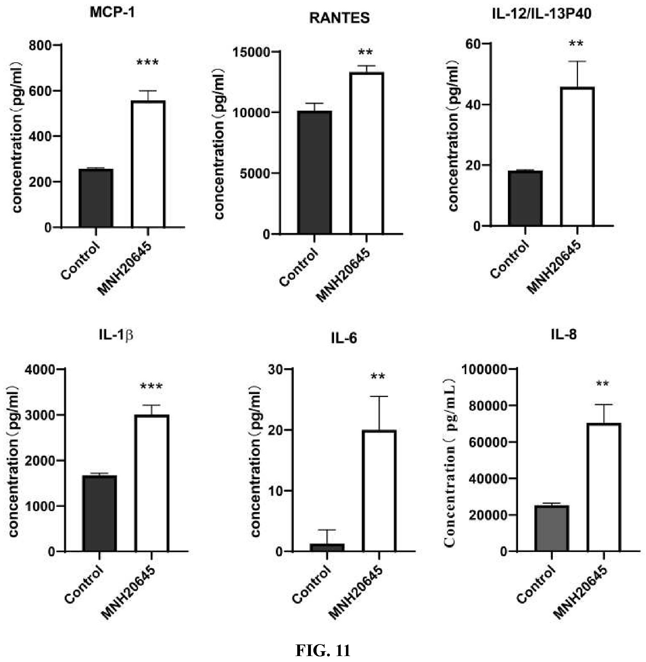

Results for the strain MNH 20645 were shown in FIG. 11. After being co-cultured with the strain MNH 20645, the macrophages also exhibited the significant characteristic of differentiation into M1 macrophages. Levels of the chemokines MCP-1 and RANTES were significantly up-regulated, while levels of the pro-inflammatory cytokines IL-1β, IL-6, IL-12/IL-13P40, and IL-8 were significantly up-regulated.

### Example 3: Regulation of the immune activity of primary PBMCs by the strains

A strain (MNH 26621 or MNH 20645) was co-incubated with primary PBMCs for 24 h, and a resulting supernatant was then collected. A control group in which the strain MNH 26621 was replaced with phosphate buffered saline (PBS) was set. With the BD^{™} CBA Human Soluble Protein Master Buffer Kit, the supernatant was allowed to react with detection proteins according to instructions, and concentrations of cytokines IL-1β, RANTES (CCL5), IL-6, MCP-1 (CCL2), and IL-8 in the supernatant were then detected by flow cytometry.

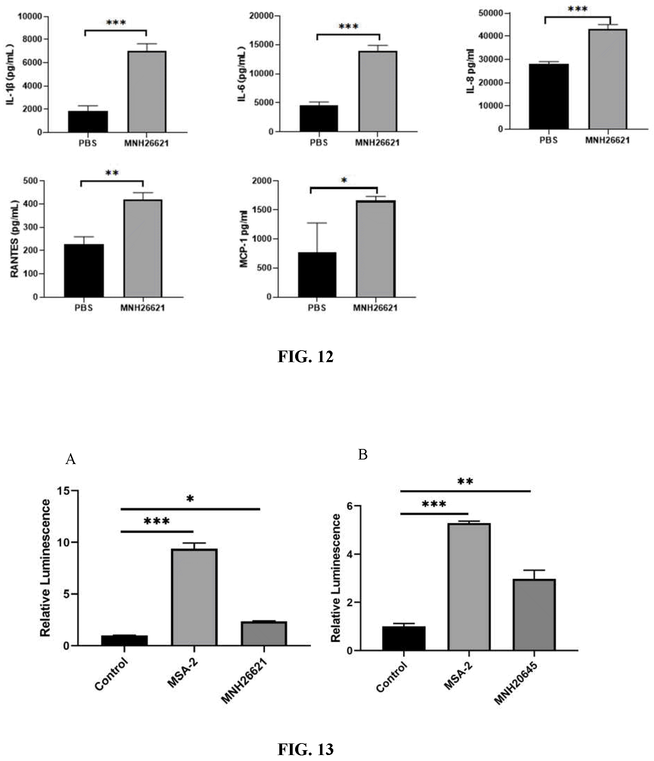

Results were shown in FIG. 12. The strain MNH 26621 could significantly induce marked increases in the inflammatory cytokines IL-1β, IL-6, and IL-8 and the chemokines MCP-1 (CCL2) and RANTES (CCL5).

Results for the strain MNH 20645 revealed that the strain MNH 20645 tended to cause enhanced up-regulation of the expression of the inflammatory cytokines IL-1β and IL-6.

### Example 4: Influence of the strains on the expression of IFN-β

IFN are a class of cytokines secreted by host cells that can regulate immune responses. Viruses, bacterial endotoxins, and synthetic double-stranded RNAs can stimulate the production of IFNs. IFN-β is type I IFN. IFN-β can enhance the activities of NK cells, macrophages, and T lymphocytes to exert anti-viral, anti-tumor, and immunomodulatory effects. To verify whether the strain could promote the expression of IFN-β, THP-1 cells carrying an IFN-β promoter reporter (a cell line established by MoonBiotech) were used to evaluate the influence of the strain on a transcriptional activity of IFN-β in this study.

Preparation of strain culture supernatants: The strain (MNH 26621 or MNH 20645) was inoculated into an MM01 liquid medium and cultured anaerobically at 37°C for 48 h. Centrifugation was conducted. A resulting bacterial pellet was discarded. A resulting culture supernatant was filtered through a 0.22 µm filter, dispensed, and stored at -80°C for later use.

Control group: Complete Dulbecco's Modified Eagle Medium (DMEM) (including 10% of fetal bovine serum (FBS)) including 10% (v/v) of the MM01 medium was used.

MSA-2 group: A compound with CAS No. 129425-81-6 was used as a positive control (40 µM) for IFN-β activation.

Strain groups: 10% (v/v) of the strain culture supernatant was used.

THP-1-IFN-β-promoter reporter cells were inoculated into a 96-well plate at 1 × 10⁵ cells/well. The cells were treated according to the established groups. The cells were further cultured for 24 h. Centrifugation was conducted at 300 g for 5 min. A resulting culture supernatant was discarded, 50 µL of a 1 × Luciferase detection reagent was added, and a reaction was conducted for 1 min. A luminescence value was measured by a microplate reader. Relative luminescence values were normalized to the control group to evaluate the influence of the strains on the transcriptional activity of IFN-β.

Results were shown in FIG. 13. The results revealed that the culture supernatants of the strains MNH 26621 and MNH 20645 both could markedly enhance the transcriptional activity of IFN-β. According to these results, the strains provided in the embodiments of the present application possess potential immunomodulatory, antiviral, and anti-tumor functions.

### Example 5: Animal experiment using the strains for treating liver cancer

To verify whether the strain MNH 26621 or MNH 20645 could be used for tumor prevention and treatment, an experiment of inhibiting liver cancer growth was conducted with a syngeneic mouse tumor model. This experimental protocol had been reviewed and approved by the Institutional Animal Care and Use Committee (IACUC) of MoonBiotech.

Test strains: Glycerol stocks of the strains in Example 1 were each thawed at 37°C, and then inoculated onto an anaerobic blood agar plate in an anaerobic workstation for activation. Each activated strain was inoculated into an MM01 liquid medium and cultured anaerobically to produce a sufficient amount of a culture. The culture was concentrated through centrifugation, and then resuspended in a vehicle (PBS-Cys: PBS including 0.05% of cysteine hydrochloride) to produce a test material with a purity and viable count (3.5 × 10⁸ CFU/mL) required by the animal experiment.

Tumor cells: H22 mouse liver cancer cells, CBP69230.

Experimental animals: A total of 140 BALB/C mice were adopted for the animal experiment, which were 5 to 6 weeks old and purchased from Guangdong Sijiajingda Biotechnology Co., Ltd.

Animal experiment: After a quarantine period under normal raising conditions, an H22 mouse liver cancer cell suspension with a concentration of 2 × 10⁶ cells/mL was intradermally injected at 0.1 mL/mouse to construct a mouse liver cancer model. The day of inoculation was designated as day 1. On day 7, mice with abnormal tumor growth were excluded, and according to tumor volumes, the remaining mice were randomly grouped with 12 mice per group. A negative control group A (Control) was administered a medium, and an experimental group G was administered the strain MNH 20645. Endpoint of this animal experiment: The intragastric administration was conducted 12 times. During the animal experiment, an animal condition, a tumor change, a tumor size, and a body weight were regularly monitored. At an experimental endpoint, a body weight change, a tumor volume change, a response rate, and TGI were analyzed. At the experimental endpoint, all mice were euthanized through cervical dislocation. Experimental results were shown in Tables 10 to 11 below, and could also be referred to FIG. 14.

**Table 10 Statistics of the overall conditions of mice at the experimental endpoint for the strain MNH 20645**

| | Mice with successful tumor engraftment | Mice at the experimental endpoint | Euthanized mice | Average tumor weight (g) | Average terminal body weight (g) | Average tumor volume (mm³) | TGI (%) |
|---|---|---|---|---|---|---|---|
| Group A | 12 | 12 | 0 | 2.25 | 30.72 | 1403.28 | 0% |
| Group G | 12 | 11 | 1 | 1.96 | 29.33 | 934.13 | 35.4% |

**Table 11 Tumor tissue weights after mouse dissection for the strain MNH 20645 experimental group**

| Group | No. | Tumor weight (g) | Average (g) |
|---|---|---|---|
| Group A | A1-1 | 1.53 | 2.25 |
| | A1-2 | 3.71 | |
| | A1-3 | 2.09 | |
| | A1-4 | 1.43 | |
| | A2-1 | 1.71 | |
| | A2-2 | 2.48 | |
| | A2-3 | 2.47 | |
| | A2-4 | 1.20 | |
| | A3-1 | 2.68 | |
| | A3-2 | 3.27 | |
| | A3-3 | 2.42 | |
| | A3-4 | 1.98 | |
| | G1-1 | 1.21 | 1.96 |
| | G1-2 | 0.30 | |
| | G1-3 | 2.32 | |
| | G1-4 | 2.60 | |
| | G2-1 | 2.23 | |
| Group G | G2-2 | 3.33 | |
| | G2-3 | 2.37 | |
| | G2-4 | 2.76 | |
| | G3-1 | 1.39 | |
| | G3-3 | 1.91 | |
| | G3-4 | 1.18 | |

According to the results, a body weight of mice increased steadily during the administration period, and there was no significant difference between the experimental group (group G) and the control group (group A), indicating that the intragastric administration of the strain did not affect the normal growth of mice. Compared with tumors in the control group, tumors in the experimental group were significantly diminished with significantly enhanced TGI, indicating a pronounced tumor growth inhibitory effect. Compared with a low response rate of the control group, a response rate of the experimental group was significantly improved. The experimental group treated with the strain MNH 20645 had a response rate as high as 45.45%, while the control group had a response rate of merely 8.33%. In summary, the strain MNH 20645 exhibited excellent therapeutic efficacy against liver cancer and could significantly lower the growth rate of liver cancer in mice.

Experimental results also revealed that the strain MNH 26621 exhibited therapeutic efficacy against liver cancer to some extent and could lower the growth rate of liver cancer in mice.

### Example 6: Animal experiment using the strains for treating lung cancer

To verify whether the strains could be used for the prevention and treatment of lung cancer, an experiment of inhibiting lung cancer growth was conducted with a syngeneic mouse tumor model. This experimental protocol had been reviewed and approved by the Institutional Animal Care and Use Committee (IACUC) of MoonBiotech.

Test strains: Glycerol stocks of the strains in Example 1 were each thawed at 37°C, and then inoculated onto an anaerobic blood agar plate in an anaerobic workstation for activation. Each activated strain was inoculated into an MM01 liquid medium and cultured anaerobically to produce a sufficient amount of a culture. The culture was concentrated through centrifugation, and then resuspended in a vehicle (PBS-Cys) to produce a test material with a purity and viable count (3.5 × 10⁸ CFU/mL) required by the animal experiment.

Tumor cells: LLC1 mouse lung cancer cells, CL-0140.

Experimental animals: C57BL/6J mice were adopted for the animal experiment, which were 5 weeks old and purchased from GemPharmatech.

### Animal experiment:

After a quarantine period under normal raising conditions, an LLC1 mouse lung cancer cell suspension with a concentration of 2 × 10⁷ cells/mL was intradermally injected at 0.1 mL/mouse to construct a mouse lung cancer model. The day of inoculation was designated as day 1. On day 7, mice with abnormal tumor growth were excluded, and according to tumor volumes, the remaining mice were randomly grouped with 12 mice per group. A negative control group (Control) was administered a medium, and an experimental group was administered a corresponding strain. On the day of grouping, intragastric administration was started. The intragastric administration was conducted at 0.2 mL/mouse/time with an administration frequency of once/day. Endpoint of this animal experiment: On day 21, the intragastric administration was conducted 14 times. During the animal experiment, an animal condition, a tumor change, a tumor size, and a body weight were regularly monitored. At an experimental endpoint, a body weight change, a tumor volume change, a response rate, and TGI were analyzed. At the experimental endpoint, all mice were euthanized through cervical dislocation. Experimental results were shown in tables below and FIG. 15 to FIG. 16.

**Table 12 Statistics of the overall conditions of mice at the experimental endpoint**

| | Group | Mice with successful tumor engraftment | Mice at the experimental endpoint | Euthanized mice | Average tumor weight (g) | Average terminal body weight (g) | Average tumor volume (mm³) | TGI at the experimental endpoint (%) |
|---|---|---|---|---|---|---|---|---|
| MN H 26621 | Control group | 12 | 11 | 1 | 1.33 | 23.34 | 1136.39 | 0% |
| | Experimental group | 12 | 11 | 1 | 0.82 | 23.92 | 668.86 | 44.2% |
| MN H 20645 | Control group | 12 | 12 | 0 | 0.58 | 24.19 | 915.38 | 0% |
| | Experimental group | 12 | 12 | 0 | 0.43 | 23.81 | 703.91 | 25.3% |

**Table 13 Tumor tissue weights after mouse dissection**

| Group | No. | Tumor weight (g) | Average (g) |
|---|---|---|---|
| Control group for MNH 26621 | A1-1 | 0.79 | 1.33 |
| | A1-2 | 0.18 | |
| | A1-3 | 1.75 | |
| | A1-4 | 2.30 | |
| | A2-1 | 2.24 | |
| | A2-2 | 0.35 | |
| | A2-3 | 0.25 | |
| | A2-4 | 2.07 | |
| | A3-2 | 0.76 | |
| | A3-3 | 1.08 | |
| | A3-4 | 2.83 | |
| MNH 26621 experimental group | D1-1 | 0.55 | 0.82 |
| | D1-2 | 2.92 | |
| | D1-3 | 0.27 | |
| | D1-4 | 1.52 | |
| | D2-1 | 1.53 | |
| | D2-2 | 0.35 | |
| | D2-3 | 0.65 | |
| | D2-4 | 0.08 | |
| | D3-1 | 0.24 | |
| | D3-3 | 0.32 | |
| | D3-4 | 0.55 | |
| Control group for MNH 20645 | A1-1 | 0.33 | 0.58 |
| | A1-2 | 0.46 | |
| | A1-3 | 0.32 | |
| | A1-4 | 0.68 | |
| | A2-1 | 1.30 | |
| | A2-2 | 0.55 | |
| | A2-3 | 0.32 | |
| | A2-4 | 0.39 | |
| | A3-1 | 0.31 | |
| | A3-2 | 1.28 | |
| | A3-3 | 0.58 | |
| | A3-4 | 0.45 | |
| MNH 20645 experimental group | G1-1 | 0.18 | 0.43 |
| | G1-2 | 0.23 | |
| | G1-3 | 0.10 | |
| | G1-4 | 0.35 | |
| | G2-1 | 0.27 | |
| | G2-2 | 1.57 | |
| | G2-3 | 0.17 | |
| | G2-4 | 0.51 | |
| | G3-1 | 0.24 | |
| | G3-2 | 0.52 | |
| | G3-3 | 0.40 | |
| | G3-4 | 0.64 | |

According to the results, a body weight of mice increased steadily during the administration period, and there was no significant difference between the experimental groups and the respective control groups before dissection. Compared with tumor volumes and weights in the respective control groups, tumor volumes and weights in the experimental groups were significantly reduced with significantly enhanced TGI, indicating pronounced tumor growth inhibitory effects. Compared with low response rates of the respective control groups, response rates of the experimental groups were significantly improved. For example, the experimental group treated with the strain MNH 26621 had a response rate as high as 72.73%, while the corresponding control group had a response rate of only 36.36%. The experimental group treated with the strain MNH 20645 had a response rate as high as 50%, while the corresponding control group had a response rate of only 16.67%. In summary, the two strains both exhibited pronounced therapeutic efficacy against lung cancer and could considerably lower the growth rate of lung cancer in mice.

### Example 7: Therapeutic effects of combinations of the strains with an anti-PD-1 antibody for lung cancer

To verify the therapeutic effects of the combinations of the strains with the anti-PD-1 antibody for lung cancer, an experiment of inhibiting lung cancer growth was conducted with a syngeneic mouse tumor model. This experimental protocol had been reviewed and approved by the Institutional Animal Care and Use Committee (IACUC) of MoonBiotech.

Test strains: Glycerol stocks of the strains in Example 1 were each thawed at 37°C, and then inoculated onto an anaerobic blood agar plate in an anaerobic workstation for activation. Each activated strain was inoculated into an MM01 liquid medium and cultured anaerobically to produce a sufficient amount of a culture. The culture was concentrated through centrifugation, and then resuspended in a vehicle (PBS-Cys) to produce a test material with a purity and viable count (1.0 × 10⁸ CFU/mL) required by the animal experiment.

Tumor cells: LLC1 mouse lung cancer cells, CL-0140.

Experimental animals: C57BL/6J mice were adopted for the animal experiment, which were 5 weeks old and purchased from GemPharmatech.

Anti-PD-1 antibody: An anti-mPD-1 antibody (RMP1-14) from BioXcell was prepared with normal saline into a 2 mg/mL solution.

### Animal experiment:

After a quarantine period under normal raising conditions, an LLC1 mouse lung cancer cell suspension with a concentration of 2 × 10⁷ cells/mL was intradermally injected at 0.1 mL/mouse to construct a mouse lung cancer model. The day of inoculation was designated as day 1. On day 5, mice with abnormal tumor growth were excluded, and according to tumor volumes, the remaining mice were randomly grouped with 8 mice per group. The grouping was as follows:

**Table 14 Combination experiment grouping**

| Group | Administration route | |
|---|---|---|
| | Intragastric administration (i.g.) | Intraperitoneal injection (i.p.) |
| Group A - negative control | PBS-Cys (glycerol) | Normal saline |
| Group B - positive control (anti-PD-1 antibody) | PBS-Cys (glycerol) | Anti-PD-1 antibody |
| Group F - MNH 20645 combination group (strain + anti-PD-1 antibody) | MNH 20645 suspension | Anti-PD-1 antibody |
| Group G - MNH 26621 combination group (strain + anti-PD-1 antibody) | MNH 26621 suspension | Anti-PD-1 antibody |

The PBS-Cys (glycerol) was prepared by thoroughly mixing PBS-Cys (PBS including 0.05% of cysteine hydrochloride) with 100% glycerol at a ratio of 3:1.

The negative control group A (negative control) received the negative control substance PBS-Cys (glycerol) (i.g.) and the negative control substance normal saline (i.p.). The positive control group B (anti-PD-1 antibody) received the negative control substance PBS-Cys (glycerol) and the positive control substance anti-PD-1 antibody (i.p.). The groups F and G received the test strain (MNH 26621 (i.g.) or MNH 20645 (i.g.)) and the anti-PD-1 antibody. On the day of grouping, administration was initiated. The PBS-Cys (glycerol) or each test strain was intragastrically administered at 0.2 mL/mouse. The normal saline was injected at 5 mL/kg. The anti-PD-1 antibody was injected at 10 mg/kg. The PBS-Cys (glycerol) or each test strain was administered once daily until an experimental endpoint (the administration was conducted 20 times in total). The normal saline or the anti-PD-1 antibody was administered once every three days, and the administration was conducted 6 times in total.

At an experimental endpoint, all surviving mice were dissected for sample collection. A tumor weight was measured, and a tumor volume was calculated. Statistical analysis and comparisons were performed, including a tumor volume change curve, a tumor volume at endpoint, a tumor weight at endpoint, and TGI.

Results revealed that the combinations of the strains with the anti-PD-1 antibody exhibited specific therapeutic efficacy against lung cancer. Tumor volumes at endpoint were as follows: the negative control group: 1,279.80 mm³, the positive control group (anti-PD-1 antibody) group: 710.29 mm³, the MNH 20645/anti-PD-1 antibody combination group: 627.71 mm³, and the MNH 26621/anti-PD-1 antibody combination group: 675.53 mm³. Compared with the positive control group, both the MNH 20645/anti-PD-1 antibody combination group and the MNH 26621/anti-PD-1 antibody combination group had decreased tumor volumes. TGI was calculated. TGI of the positive control group (anti-PD-1 antibody) was 47.3%, TGI of the MNH 20645/anti-PD-1 antibody combination group was 53.9%, and TGI of the MNH 26621/anti-PD-1 antibody combination group was 50.0%. Compared with the positive control group, both the MNH 20645/anti-PD-1 antibody combination group and the MNH 26621/anti-PD-1 antibody combination group showed an increase in TGI to some extent. Therefore, both the MNH 20645/anti-PD-1 antibody combination group and the MNH 26621/anti-PD-1 antibody combination group exhibited specified inhibitory effects on tumor growth.

### Example 8: Therapeutic effects of a combination of the strain MNH 20645 with gemcitabine for pancreatic cancer

To verify the therapeutic effects of the combination of the strain MNH 20645 with gemcitabine for pancreatic cancer, an experiment of inhibiting lung cancer growth was conducted with a syngeneic mouse tumor model. This experimental protocol had been reviewed and approved by the Institutional Animal Care and Use Committee (IACUC) of MoonBiotech.

Test strain: A glycerol stock of the strain MNH 20645 was thawed at 37°C, and then inoculated onto an anaerobic blood agar plate in an anaerobic workstation for activation. An activated strain was inoculated into an MM01 liquid medium and cultured anaerobically to produce a sufficient number of viable bacteria. A resulting bacterial solution was concentrated through centrifugation, and then resuspended in a vehicle to produce a test material with a purity and viable count (1.30 × 10¹⁰ CFU/mL) required by an animal experiment.

Tumor cells: KPC mouse pancreatic cancer cells.

Experimental animals: Female C57BL/6J mice were adopted for the animal experiment, which were 7 to 8 weeks old and purchased from GemPharmatech.

Animal experiment: After a quarantine period under normal raising conditions, a KPC mouse pancreatic cancer cell suspension with a concentration of 3 × 10⁶ cells/mL was subcutaneously inoculated at 0.1 mL/mouse to establish an ectopic syngeneic tumor model. When an average tumor volume reached 60 mm³ to 100 mm³, mice were randomly divided into 10 groups based on tumor volumes. The grouping details were shown in a table below:

**Table 15 Combination experiment grouping**

| Group | Administration route | |
|---|---|---|
| | Intragastric administration | Intraperitoneal injection |
| Group A (negative control) | PBS-Cys (glycerol) | Normal saline |
| Group B (positive control, gemcitabine) | PBS-Cys (glycerol) | Gemcitabine |
| Group G (MNH 20645 & gemcitabine) | MNH 20645 | Gemcitabine |

The PBS-Cys (glycerol) was prepared by thoroughly mixing PBS-Cys (PBS including 0.05% of cysteine hydrochloride) with 100% glycerol at a ratio of 3:1.

On the day of grouping, intragastric administration was initiated. The groups A and B were administered PBS-Cys (glycerol) and the group G was administered MNH 20645, with an intragastric volume of 0.2 mL/mouse. When an average tumor volume of the negative control group A grew to 120 mm³ to 150 mm³, intraperitoneal injection was initiated in the groups A, B, and G. The group A was administered normal saline at a dose of 5 mg/kg, and the groups B and G were each administered gemcitabine at a dose of 50 mg/kg. The intraperitoneal injection was conducted once every three days, with a total of 6 times. Upon receipt of the animals and at the end of the quarantine period, an animal body weight was measured. After tumor inoculation, an animal body weight was measured twice weekly. On the days when the intraperitoneal injection was required, an animal body weight was measured. The day of tumor cell inoculation was designated as day 1. From day 7 to the grouping, a tumor diameter was measured once daily. After the grouping, a tumor diameter was measured once every three days. When an average tumor volume reached or exceeded 600 mm³ in any group, a tumor diameter was measured once every two days, and a tumor growth condition was recorded. The experiment was terminated on day 35. During this experiment, general clinical observations, body weight monitoring, and tumor volume (mm³) measuring were conducted. At an experimental endpoint, all surviving mice were dissected for sample collection. A tumor weight was measured, and a tumor volume was calculated. Statistical analysis and comparisons were performed, including a tumor volume change curve, a tumor volume at endpoint, a tumor weight at endpoint, and TGI.

TGI: (average volume of control group - volume of experimental group)/average volume of control group × 100%. All data was expressed as mean ± standard deviation (SD). Plotting and statistical analysis were performed using GraphPad Prism 8.0.2 software. For comparisons among three or more groups, one-way analysis of variance (one-way ANOVA) was used in combination with Dunnett's multiple comparisons test. For two-factor studies, two-way ANOVA was used in combination with Šídák's multiple comparisons test. A statistical significance was indicated as follows: *p < 0.05, **p < 0.01, ***p < 0.001, and ****p < 0.0001.

Results were shown in tables below and FIG. 17.

**Table 16 Statistics of the overall conditions of mice at the experimental endpoint**

| | Mice with successful tumor engraftment | Mice at the experimental endpoint | Euthanized mice | Average tumor weight (g) | Average terminal body weight (g) | Average tumor volume (mm³) | TGI (%) |
|---|---|---|---|---|---|---|---|
| Negative control group | 8 | 8 | 0 | 0.92 | 21.36 | 961.75 | 0% |
| Positive control group | 8 | 6 | 2 | 0.68 | 21.13 | 605.41 | 40.0 % |
| Experimental group | 8 | 8 | 0 | 0.48 | 20.70 | 429.52 | 60.0 % |

**Table 17 Tumor tissue weights after mouse dissection**

| Group | No. | Tumor weight (g) | Average (g) |
|---|---|---|---|
| | A1-1 | 0.87 | 0.92 |
| Negative control group | A1-2 | 0.88 | |
| | A1-3 | 1.03 | |
| | A1-4 | 0.85 | |
| | A2-1 | 0.94 | |
| | A2-2 | 0.95 | |
| | A2-3 | 0.97 | |
| | A2-4 | 0.86 | |
| Positive control group | B1-1 | 0.35 | 0.68 |
| | B1-2 | 0.49 | |
| | B1-3 | 0.89 | |
| | B1-4 | 0.81 | |
| | B2-3 | 0.59 | |
| | B2-4 | 0.97 | |
| Experimental group | G1-1 | 0.14 | 0.48 |
| | G1-2 | 0.00 | |
| | G1-3 | 0.65 | |
| | G1-4 | 0.39 | |
| | G2-1 | 0.61 | |
| | G2-2 | 0.56 | |
| | G2-3 | 0.77 | |
| | G2-4 | 0.68 | |

At the experimental endpoint, an average tumor volume in the negative control group was 961.75 mm³, an average tumor volume in the positive control group (gemcitabine) was 605.41 mm³, and an average tumor volume in the MNH 20645/gemcitabine combination group was significantly reduced to 429.52 mm³. On day 35, an average tumor weight in the negative control group was approximately 0.92 g, an average tumor weight in the positive control group was approximately 0.68 g, and an average tumor weight in the experimental group was approximately 0.48 g. Thus, the combination of MNH 20645 with gemcitabine could suppress tumor growth.

A of FIG. 17 shows curves of tumor volume changes at different intervention time points for mice in various groups, and B of FIG. 17 shows tumor volumes at endpoint for mice in various groups. In A and B of FIG. 17, data is expressed as mean tumor volume ± SD (mm³). According to A of FIG. 17, statistical analysis was conducted using Two-Way ANOVA. Compared with a tumor volume in the negative control group, tumor volumes in both the positive control group and the MNH 20645/gemcitabine combination group were significantly reduced. Further, the MNH 20645/gemcitabine combination group had a significantly smaller tumor volume than the positive control group. According to B of FIG. 17, statistical analysis was conducted using One-Way ANOVA in combination with Dunnett's multiple comparisons test. At the experimental endpoint, compared with the tumor volume in the negative control group, the tumor volumes in both the positive control group and the MNH 20645/gemcitabine combination group were significantly reduced. According to C of FIG. 17, statistical analysis was conducted using one-way ANOVA in combination with Dunnett's multiple comparisons test. At the experimental endpoint, compared with the tumor volume in the negative control group, a tumor volume in the positive control group tended to decrease without statistical significance, while a tumor volume in the MNH 20645/gemcitabine combination group was significantly reduced. D of FIG. 17 shows TGI values at endpoint for mice. According to one-way ANOVA results, at the experimental endpoint, TGI values at endpoint of both the positive control group and the MNH 20645/gemcitabine combination group were significantly higher than a TGI value at endpoint of the negative control group. E of FIG. 17 shows body weights of mice during the administration period. The results indicated that the body weights of mice showed an increasing trend throughout the administration period. Two-way ANOVA results revealed that there were no significant differences between the MNH 20645/gemcitabine combination group and the negative control group or the positive control group. According to A to D of FIG. 17, the combination of MNH 20645 with gemcitabine could significantly suppress the growth of pancreatic cancer.

In F to H of FIG. 17, a tumor volume change of an individual mouse is presented as a tumor volume value, and a dotted line represents a tumor volume of the negative control group corresponding to TGI of 40%. When a tumor volume of a mouse is greater than the tumor volume of the negative control group corresponding to TGI of 40%, TGI is less than 40% and there is no response to an anti-tumor treatment. When a tumor volume of a mouse is less than the tumor volume of the negative control group corresponding to TGI of 40%, TGI is higher than or equal to 40% and there is response to an anti-tumor treatment. Compared with a response rate of 0% in the negative control group, a response rate in the positive control group was 50%, and a response rate in the MNH 20645/gemcitabine combination group was significantly improved to 62.5%.

In summary, the combination of MNH 20645 with gemcitabine could inhibit the growth of pancreatic cancer and significantly lower the growth rate of pancreatic cancer in mice.

### Example 9: Analysis of immune activation in lung cancer-bearing mice by the strain MNH 26621

This example was based on the following principle: A tissue of a tumor-bearing mouse was collected and prepared into a single-cell suspension, and labeling was then conducted with fluorescently labeled specific molecules. A proportion of cells expressing specific molecules could be detected by flow cytometry. A proportional distribution of immune cells reflected, to some extent, an immune status of a mouse.

An appropriate amount of a tumor tissue was collected from a mouse (a tumor-bearing mouse) at the experimental endpoint in Example 6, rinsed with PBS, cut into small pieces, and then homogenized. A homogenized tumor tissue was placed in 5 mL of a collagenase IV digestion solution (1 mg/mL of collagenase IV, 0.1 mg/mL of DNase, and 10% of FBS) and digested at 37°C for 30 min. A digested tissue solution was filtered through a 70 µm filter membrane to produce a mouse tumor single-cell suspension. The mouse tumor single-cell suspension was then loaded for testing. A total number of cells in the tumor tissue was counted. The mouse tumor single-cell suspension was taken, and T-Live-Tumor/NK-Live-Tumor and T-Tumor/NK-Tumor (a corresponding antibody combination) were added to stain T cells and NK cells on the surface of tumor cells. Stained cells were resuspended in PBS and then loaded for testing. The mouse tumor single-cell suspension was taken, and MDSC-Live-Tumor and MDSC-Tumor (a corresponding antibody combination) were added to stain tumor-infiltrating myeloid-derived suppressor cells (MDSCs). Stained cells were resuspended in PBS and then loaded for testing. The mouse tumor single-cell suspension was taken, and TAM-Live-Tumor and TAM-Tumor-Surface (a corresponding antibody combination) were added to stain tumor-infiltrating tumor-associated macrophages (TAMs). Stained cells were resuspended in PBS and then loaded for testing. The mouse tumor single-cell suspension was taken and stimulated with a stimulant for 4 h. Live-Tumor and Tumor-IFN-Surface (a corresponding antibody combination) were added to stain tumor cells. Stained cells were resuspended in PBS, treated with a fixation solution and a permeabilization solution successively according to instructions of a test kit, stained with T-IFN/TNF, resuspended in PBS, and then loaded for testing. The mouse tumor single-cell suspension was taken, and Treg-Live-Tumor and Treg-Surface-Tumor (a corresponding antibody combination) were added successively for staining. Stained cells were resuspended in PBS, treated with a fixation solution and a permeabilization solution successively according to instructions of a test kit, subjected to nuclear staining with Treg-Foxp3-Tumor, resuspended in PBS, and then loaded for testing.

Flow cytometry data was processed using CyExpert, and statistical processing was performed using Graphpad Prism V9. Statistical analysis was performed using one-way ANOVA with Dunnet's multiple comparison: ns: not significant, *p < 0.05, **p < 0.01, and ***p < 0.001.

Compared with the control group, proportions of cytotoxic CD4⁺IFN-γ⁺ cells (A of FIG. 18) and CD45⁺CD3⁺ cells (B of FIG. 18) in a mouse tumor tissue were significantly up-regulated, while a proportion of immunosuppressive Treg cells (C of FIG. 18) was significantly down-regulated.

The correlation between immune cells in a tumor tissue and a tumor volume at endpoint was analyzed (as shown in A to D of FIG. 19). Analysis results revealed that the tumor volume at endpoint showed a significant positive correlation with the proportions of immunosuppressive Treg cells and M2 macrophages in the tumor tissue, and a significant negative correlation with the proportions of tumor-killing CD8⁺IFN-γ⁺ cells and CD8⁺TNF-α⁺ cells in the tumor tissue.

The correlation between M2 macrophages and cytotoxic CD8⁺IFN-γ⁺ cells in a tumor tissue and a tumor weight was analyzed (as shown in A and B of FIG. 20). Analysis results revealed that the tumor weight showed a significant positive correlation with M2 macrophages in the tumor tissue, and a significant negative correlation with a proportion of tumor-killing CD8⁺IFN-γ⁺ cells in the tumor tissue.

Thus, the strain provided in the embodiments of the present application could activate local anti-tumor immunity in mice by inhibiting the proportion of immunosuppressive Treg cells and increasing the proportions of CD45⁺CD3⁺ cells and cytotoxic CD4⁺IFN-γ⁺ cells in mouse tumors, thereby exerting an anti-tumor effect.

Therefore, the strain MNH 26621 provided in the embodiments of the present application could exert anti-tumor activity by stimulating the activation of a mouse immune system.

### Example 10: Evaluation of the influence of the strains MNH 26621 and MNH 20645 on the differentiation and maturation of mouse bone marrow-derived DCs

This example is based on the following principle: DCs play a critical role in immune activation. It has been confirmed that DCs are the only antigen-presenting cells (APCs) capable of significantly stimulating the proliferation of naive T cells. Other types of APCs (such as monocyte-macrophages and B cells) can only stimulate activated or memory T cells. Therefore, DCs are the initiators of the adaptive T cell immune response in the body, and play an extremely critical role in tumor immunity. For mice, the most common method is to induce the generation of DCs from bone marrow cells, namely, bone marrow-derived dendritic cells (BMDCs). Therefore, in this study, with BMDC as a model, biomarkers associated with differentiation and maturation on the surface of DCs (such as CD11c, MHCII, CD80, and CD86) were detected by flow cytometry to evaluate the influence of the strain on the differentiation and maturation of DCs.

Experiment preparation: Complete DMEM used in this example was DMEM including 10% of FBS, 1% of penicillin-streptomycin, 1% of L-glutamine, and 1% of an N-2-hydroxyethylpiperazine-N'-2-ethanesulfonic acid (HEPES) buffer (pH: 6.8 to 8.2).

A BMDC induction medium was complete DMEM including 25 ng/mL of granulocyte-macrophage colony-stimulating factor (GM-CSF) and 10 ng/mL of interleukin-4 (IL-4).

Preparation of an MNH 26621 culture supernatant: The strain MNH 26621 was inoculated into an MM01 liquid medium and cultured anaerobically at 37°C for 48 h. Centrifugation was conducted. A resulting bacterial pellet was discarded. A resulting culture supernatant was filtered through a 0.22 µm filter, dispensed, and stored at -80°C for later use.

Preparation of an MNH 20645 culture supernatant: The strain MNH 20645 was inoculated into an MM01 liquid medium and cultured anaerobically at 37°C for 48 h. Centrifugation was conducted. A resulting bacterial pellet was discarded. A resulting culture supernatant was filtered through a 0.22 µm filter, dispensed, and stored at -80°C for later use.

Control group: Complete DMEM.

MM01 group: BMDC induction medium including 10% of the MM01 liquid medium.

MNH 26621 group: BMDC induction medium including 10% of the MNH 26621 culture supernatant.

MNH 20645 group: BMDC induction medium including 10% of the MNH 20645 culture supernatant.

A BMDC surface marker staining solution was prepared using CD45-AF700, CD11C-PC5.5, MHC-II-APC, CD86-PE-Cy7, and CD80-FITC at 1:100, with 100 µL per reaction system.

Experimental steps: Extraction of mouse bone marrow cells: A femur and a tibia were isolated from a healthy wild-type (C57BL/6) mouse. Two ends of each of the femur and the tibia were cut off. Bone marrow cells were flushed out using a 1 mL syringe filled with pre-cooled PBS, then pipetted up and down for dispersion, ground, and centrifuged at 1,000 rpm for 5 min. Red blood cell lysis was conducted. Resulting cells were resuspended in PBS to produce a single-cell suspension.

Induction and culture of BMDCs: Bone marrow cells were collected through centrifugation, resuspended in 10 mL of a BMDC induction medium, transferred to a 10 cm culture dish, and cultured for 6 d in a 37°C incubator to allow differentiation. During the culturing, a half-medium change was conducted with the BMDC induction medium every two days. After the 6 d culturing, relatively mature BMDCs were obtained.

BMDCs were treated with the culture supernatant. BMDCs were collected through centrifugation, resuspended, counted, and inoculated into a 12-well plate at 1 × 10⁶ cells/well. The cells were treated according to conditions of the control group, the MM01 group, the MNH 26621 group, and the MNH 20645 group, respectively. After 24 h of culturing, cells were collected through centrifugation, washed with PBS, and stained with the BMDC surface marker staining solution for 30 min. Finally, BMDCs were analyzed by flow cytometry to assess the expression of MHCII, CD80, and CD86 molecules associated with the differentiation and maturation of DCs.

Experimental results: FIG. 21 shows flow cytometry results of molecules MHCII, CD80, and CD86 on the surface of DCs in the MNH 26621 group. Compared to the MM01 group, MNH 26621 can significantly up-regulate the expression of the MHCII molecule on DCs, and can also markedly promote the up-regulation of CD80 and CD86 molecules. These results indicate that MNH 26621 is capable of promoting the differentiation and maturation of DCs and may consequently enhance the activation of anti-tumor immunity.

FIG. 22 shows flow cytometry results of molecules MHCII and CD86 on the surface of DCs in the MNH 20645 group. Compared to the MM01 group, MNH 20645 can significantly up-regulate the expression of the MHCII molecule on DCs, and can also markedly promote the up-regulation of the CD86 molecule. These results indicate that MNH 20645 is capable of promoting the differentiation and maturation of DCs and may consequently enhance the activation of anti-tumor immunity.

In FIG. 21 to FIG. 22, data is expressed as mean ± SD. Statistical analysis is performed using one-way ANOVA in combination with Tukey's multiple comparisons test. A statistical significance is indicated by *, and significance levels are defined as follows: no significant difference (ns): p ≥ 0.05; *: p < 0.05; **: p < 0.01; ***: p < 0.001; and ****: p < 0.0001.

### Example 11: Evaluation of inhibitory effects of the strains MNH 26621 and MNH 20645 on M2 TAMs

This example is based on the following principle: TAMs play a critical role in the malignant progression of tumors. TAMs can be broadly categorized into M1 and M2 phenotypes. The M1 phenotype exerts a pro-inflammatory effect and inhibits tumor growth. The M2 phenotype shows an anti-inflammatory function, suppresses anti-tumor immune activation, and promotes malignant tumor progression. Therefore, for M2 macrophages, an M2 TAM model was constructed by *in vitro* inducing mouse monocytic macrophage leukemia cells (RAW264.7) with IL-4. Expression levels of biomarkers CD206 and Arg1 by M2 macrophages were detected using real-time quantitative PCR to evaluate the influence of each strain on the M2 macrophage phenotype.

Experiment preparation: Preparation of an MNH 26621 culture supernatant: The strain MNH 26621 was inoculated into an MM01 liquid medium and cultured anaerobically at 37°C for 48 h. Centrifugation was conducted. A resulting bacterial pellet was discarded. A resulting culture supernatant was filtered through a 0.22 µm filter, dispensed, and stored at -80°C for later use.

Preparation of an MNH 20645 culture supernatant: The strain MNH 20645 was inoculated into an MM01 liquid medium and cultured anaerobically at 37°C for 48 h. Centrifugation was conducted. A resulting bacterial pellet was discarded. A resulting culture supernatant was filtered through a 0.22 µm filter, dispensed, and stored at -80°C for later use.

Complete DMEM used in this example included DMEM, FBS, and penicillin-streptomycin (P/S) at 9:1:0.1, for example, 45 mL of the DMEM + 5 mL of the FBS + 500 µL of the penicillin-streptomycin (P/S).

Control group: Complete DMEM including 5% of the MM01 liquid medium.

TAM group: Complete DMEM including 20 ng/mL of IL4 and 5% of the MM01 liquid medium.

MNH 26621 group: Complete DMEM including 20 ng/mL of IL4 and 5% of the MNH 26621 culture supernatant.

MNH 20645 group: Complete DMEM including 20 ng/mL of IL4 and 5% of the MNH 20645 culture supernatant.

After counting, RAW264.7 cells were inoculated into a 6-well plate at 5 * 10⁵ cells/2 mL/well and cultured in a 5% CO₂ and 37°C incubator for 24 h to allow cell adhesion. The next day, the original medium was removed using a pipette, 2 mL of a medium corresponding to the Control, TAM, MNH 26621, or MNH 20645 was added, and thorough mixing was conducted gently. The plate was incubated in a 5% CO₂ and 37°C incubator for 2 d. Cells were washed with PBS, and RNA was extracted (TAKARA MiniBEST Universal RNA Extraction Kit, TAKARA) and reverse-transcribed (PrimeScriptTM RT reagent Kit with gDNA Eraser, TAKARA). The expression of CD206, Arg1, and an internal reference gene GAPDH was detected through qPCR (TB Green^{®} Premix Ex Taq^{™} II, TAKARA). All operations were conducted according to instructions of respective kits.

CD206 primers: F-CTCTGTTCAGCTATTGGACGC (SEQ ID NO: 5) and R-CGGAATTTCTGGGATTCAGCTTC (SEQ ID NO: 6). Arg1 primers: F-CATTGGCTTGCGAGACGTAGAC (SEQ ID NO: 7) and R-GCTGAAGGTCTCTTCCATCACC (SEQ ID NO: 8). GAPDH primers: F-GTGTTCCTACCCCCAATGTGT (SEQ ID NO: 9) and R-ATTGTCATACCAGGAAATGAGCTT (SEQ ID NO: 10).

Experimental results: FIG. 23 shows qPCR detection results of the expression of M2 markers CD206 and Arg1 by RAW264.7 cells in the MNH 26621 group. According to the results, IL-4 can significantly promote the up-regulation of the expression of M2 macrophage biomarkers CD206 and Arg1, indicating that IL-4 can successfully induce the transition of RAW265.7 cells to the M2 phenotype. The culture supernatant of the strain MNH 26621 can significantly suppress the IL-4-induced up-regulation of CD206 and Arg1, indicating that MNH 26621 can inhibit the IL-4-induced transition of RAW264.7 cells to the M2 phenotype. These results indicate that MNH 26621 possesses the ability to counteract the immunosuppression of M2 macrophages in a tumor microenvironment, thereby promoting the activation of anti-tumor immunity.

FIG. 24 shows qPCR detection results of the expression of M2 markers CD206 and Arg1 by RAW264.7 cells in the MNH 20645 group. According to the results, IL-4 can significantly promote the up-regulation of the expression of M2 macrophage biomarkers CD206 and Arg1, indicating that IL-4 can successfully induce the transition of RAW265.7 cells to the M2 phenotype. The culture supernatant of the strain MNH 20645 can significantly suppress the IL-4-induced up-regulation of CD206 and Arg1, indicating that MNH 20645 can inhibit the IL-4-induced transition of RAW264.7 cells to the M2 phenotype. These results indicate that MNH 20645 possesses the ability to counteract the immunosuppression of M2 macrophages in a tumor microenvironment, thereby promoting the activation of anti-tumor immunity.

In FIG. 23 to FIG. 24, data is expressed as mean ± SD. Statistical analysis is performed using one-way ANOVA in combination with Tukey's multiple comparisons test. A statistical significance is indicated by *, and significance levels are defined as follows: no significant difference (ns): p ≥ 0.05; *: p < 0.05; **: p < 0.01; ***: p < 0.001; and ****: p < 0.0001.

The present application has been described in detail above with reference to the examples, but the present application is not limited to the above examples. Within the scope of knowledge possessed by those of ordinary skill in the art, various modifications can be made without departing from the purpose of the present application. In addition, in the case of no conflict, the examples of the present application and the features in the examples may be combined with each other.

## Claims

1. A *Parabacteroides* strain, comprising any one of following A1) to A5):
A1) a strain, wherein 16S rRNA of the strain has an identity of at least 96.74%, 97%, 98%, 98.5%, 98.65%, 99%, 99.5%, 99.6%, 99.7%, 99.8%, 99.9%, 99.99%, or 100% to a sequence shown in SEQ ID NO: 1;
A2) a strain with an accession number GDMCC No: 62664 or GDMCC No: 62665;
A3) a strain, wherein 16S rRNA of the strain has an identity of at least 96.74%, 97%, 98%, 98.5%, 98.65%, 99%, 99.5%, 99.6%, 99.7%, 99.8%, 99.9%, 99.99%, or 100% to 16S rRNA of the strain described in the A2);
A4) a strain, wherein 16S rRNA of the strain has an identity of at least 97%, 98%, 98.65%, 99%, 99.5%, 99.9%, or 100% to a sequence shown in SEQ ID NO: 2,
wherein preferably, the strain is at least one selected from *Parabacteroides hominis*, *Parabacteroides merdae*, *Parabacteroides johnsonii*, *Parabacteroides massiliensis*, *Parabacteroides acidifaciens*, *Parabacteroides faecis*, *Parabacteroides bouchesdurhonensis*, *Parabacteroides provencensis*, *Parabacteroides goldsteinii*, *Parabacteroides gordonii*, *Parabacteroides timonensis*, *Parabacteroides chinchillae*, *Parabacteroides distasonis*, and *Parabacteroides hominis*; and
A5) a strain. wherein a genome of the strain has an average nucleotide identity (ANI) of at least 87%, 88%, 88.81%, 88.89%, 89%, 90%, 95%, 96%, 97%, 98%, 99%, 99.5%, 99.6%, 99.7%, 99.8%, 99.9%, 99.99%, or 100% to a genome of a strain described in any of the A1) to the A4); and/or, a strain, wherein a genome of the strain has an alignment fraction (AF) of at least 70%, 73.54%, 74.55%, 75%, 80%, 85%, 90%, 95%, 96%, 97%, 98%, 99%, or 100% to a genome of a strain described in any of the A1) to the A4),
wherein preferably, in the A2), a 16S rRNA sequence of the strain with the accession number GDMCC No: 62664 is shown in SEQ ID NO: 1, and a 16S rRNA sequence of the strain with the accession number GDMCC No: 62665 is shown in SEQ ID NO: 2;
preferably, the *Parabacteroides* strain is derived from a human gut;
preferably, the *Parabacteroides* strain is capable of producing short-chain fatty acids (SCFAs) with a high yield; and
preferably, the *Parabacteroides* strain is capable of producing acetic acid and/or propionic acid with a high yield.

2. A culture of the *Parabacteroides* strain according to claim 1, wherein
preferably, the culture comprises any one of following B1) to B5):
B1) a fermentation medium in which the *Parabacteroides* strain has been cultured;
B2) a supernatant resulting from the fermentation medium in which the *Parabacteroides* strain has been cultured;
B3) a concentrate of the B1) or the B2);
B4) an extract of the B1) or the B2); and
B5) a dried product of any of the B1) to the B4);
preferably, the fermentation medium is a liquid medium;
preferably, the culture is produced by culturing the *Parabacteroides* strain in the liquid medium under anaerobic culture conditions; and
preferably, the fermentation medium is an MM01 liquid medium, and/or the *Parabacteroides* strain is cultured at 35°C to 42°C, and/or the *Parabacteroides* strain is cultured for 24 h to 72 h, and/or a pH of the fermentation medium is 6 to 10.

3. A microbial agent comprising the *Parabacteroides* strain according to claim 1, metabolites of the *Parabacteroides* strain according to claim 1, or the culture according to claim 2.

4. A composition, wherein the composition or a raw material for the composition comprises the *Parabacteroides* strain according to claim 1, metabolites of the *Parabacteroides* strain according to claim 1, the culture according to claim 2, or the microbial agent according to claim 3;
preferably, the composition further comprises at least one of an excipient, a diluent, a carrier, and an adjuvant;
preferably, the composition further comprises at least one probiotic, and the at least one probiotic is at least one of *Bifidobacterium spp*., *Lactobacillus spp.*, *Lactococcus spp.*, *Lacticaseibacillus spp.*, *Pediococcus acidilactici*, *Akkermansia*, *Pediococcus pentosaceus*, and *Saccharomyces boulardii*;
preferably, the probiotic is selected from *Bifidobacterium adolescentis*, *Bifidobacterium animalis subsp.animalis*, *Bifidobacterium animalis subsp.lactis*, *Bifidobacterium bifidum*, *Bifidobacterium breve*, *Bifidobacterium longum subsp.longum*, *Bifidobacterium longum subsp.infantis*, *Lactobacillus acidophilus*, *Lactobacillus crispatus*, *Lactobacillus delbrueckii subsp.Bulgaricus*, *Lactobacillus delbrueckii subsp.Lactis*, *Lactobacillus gasseri*, *Lactobacillus helveticus*, *Lactobacillus johnsonii*, *Lactobacillus kefiranofaciens subsp.Kefiranofaciens*, *Lacticaseibacillus casei*, *Lacticaseibacillus paracasei*, *Lacticaseibacillus rhamnosus*, *Limosilactobacillus fermentum*, *Limosilactobacillus reuteri*, *Lactiplantibacillus plantarum*, *Ligilactobacillus salivarius*, *Latilactobacillus curvatus*, *Latilactobacillus sakei*, *Streptococcus salivarius subsp.thermophilus*, *Lactococcus lactis subsp.lactis*, *Lactococcus lactis subsp.lactis biovardiacetylactis*, *Lactococcus cremoris*, and *Akkermansia muciniphila*;
preferably, the adjuvant comprises at least one of a lubricant, a wetting agent, a binder, an emulsifier, a suspension stabilizer, a solubilizer, a preservative, a sweetener, and a flavoring agent;
preferably, the composition comprises at least one of a pharmaceutical, a nutritional supplement, a dietary supplement, a food, and a food additive;
preferably, the composition is a solid preparation or a liquid preparation;
preferably, a dosage form of the composition is any one of a powder, a granule, a tablet, a capsule, a gel, a suspension, a drop, a dropping pill, an injection, a suppository, an aerosol, an oral liquid, an ointment, an emulsion, and a lavage solution; and
preferably, the composition further comprises a package insert.

5. A pharmaceutical composition, comprising following C1) and C2):
C1) the *Parabacteroides* strain according to claim 1, metabolites of the *Parabacteroides* strain according to claim 1, the culture according to claim 2, or the microbial agent according to claim 3; and
C2) a drug for combination therapy,
wherein preferably, the drug for combination therapy comprises at least one of an anti-tumor drug, an anti-metabolic disease drug, an anti-inflammatory or autoimmune disease drug, an anti-infectious disease drug, and an anti-central nervous system disease drug;
preferably, the anti-tumor drug comprises at least one of a chemotherapeutic drug, a photosensitizer, a photothermal agent, and an immunotherapeutic drug;
preferably, the chemotherapeutic drug comprises at least one of paclitaxel, camptothecin, 5-fluorouracil, cisplatin, doxorubicin, mitomycin, epirubicin, and gemcitabine;
preferably, the photosensitizer comprises at least one of boron dipyrromethene, chlorin, and rose bengal;
preferably, the photothermal agent comprises at least one of a noble metal nanoparticle, an organic polymer, a carbon-based nanomaterial, a magnetic nanomaterial, and a semiconductor nanomaterial;
preferably, the immunotherapeutic drug comprises at least one of an immune cell therapy drug and an immune checkpoint inhibitor;
preferably, the immune cell therapy drug comprises at least one of a T cell therapy drug, a tumor-infiltrating lymphocyte therapy drug, and a natural killer (NK) cell therapy drug;
preferably, the immune checkpoint inhibitor comprises an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA;
preferably, the pharmaceutical composition comprises 1 × 10³ CFU/g to 1 × 10¹³ CFU/g or 1 × 10³ CFU/mL to 1 × 10¹³ CFU/mL of the *Parabacteroides* strain;
preferably, each gram or each milliliter of the pharmaceutical composition comprises at least 1 × 10³, 2 × 10³, 5 × 10³, 1 × 10⁴, 2 × 10⁴, 5 × 10⁴, 1 × 10⁵, 2 × 10⁵, 5 × 10⁵, 1 × 10⁶, 2 × 10⁶, 5 × 10⁶, 1 × 10⁷, 2 × 10⁷, 5 × 10⁷, 1 × 10⁸, 2 × 10⁸, 5 × 10⁸, 1 × 10⁹, 2 × 10⁹, 5 × 10⁹, 1 × 10¹⁰, 2 × 10¹⁰, 5 × 10¹⁰, 1 × 10¹¹, 2 × 10¹¹, 5 × 10¹¹, 1 × 10¹², 2 × 10¹², 5 × 10¹², or 1 × 10¹³ colony-forming units (CFU) of the *Parabacteroides* strain;
preferably, the *Parabacteroides* strain is present in the pharmaceutical composition in a form of live bacteria, attenuated bacteria, killed bacteria, lyophilized bacteria, or irradiated bacteria;
preferably, the pharmaceutical composition or a raw material for the pharmaceutical composition comprises the C1) in a mass percentage of about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, or 80%;
preferably, the pharmaceutical composition or the raw material for the pharmaceutical composition comprises the C1) in a mass percentage of 1% to 80%, 2% to 70%, 5% to 60%, 10% to 50%, 20% to 40%, or 40% to 60%;
preferably, the pharmaceutical composition further comprises at least one of an excipient, a diluent, and a carrier;
preferably, the pharmaceutical composition further comprises an adjuvant;
preferably, the adjuvant comprises at least one of a lubricant, a wetting agent, a binder, an emulsifier, a suspension stabilizer, a solubilizer, a preservative, a sweetener, and a flavoring agent;
preferably, the pharmaceutical composition is in an infant-suitable dosage form, a child-suitable dosage form, or an adult-suitable dosage form;
preferably, the pharmaceutical composition is in a dosage form for enteral administration or a dosage form for parenteral administration;
preferably, the pharmaceutical composition is an oral preparation or an injection;
preferably, a dosage form of the pharmaceutical composition is any one of a powder, a granule, a tablet, a capsule, a gel, a suspension, a drop, a dropping pill, an injection, a suppository, an aerosol, an oral liquid, an ointment, an emulsion, and a lavage solution;
preferably, the *Parabacteroides* strain is capable of at least partially proliferating in a gut of a subject; and
preferably, the pharmaceutical composition further comprises a package insert.

6. A pharmaceutical combination product, comprising following independent components:
a first product comprising the *Parabacteroides* strain according to claim 1, metabolites of the *Parabacteroides* strain according to claim 1, the culture according to claim 2, or the microbial agent according to claim 3; and
a second product comprising a drug for combination therapy,
wherein preferably, the drug for combination therapy comprises at least one of an anti-tumor drug, an anti-metabolic disease drug, an anti-inflammatory or autoimmune disease drug, an anti-infectious disease drug, and an anti-central nervous system disease drug;
preferably, the anti-tumor drug comprises at least one of a chemotherapeutic drug, a photosensitizer, a photothermal agent, and an immunotherapeutic drug;
preferably, the chemotherapeutic drug comprises at least one of paclitaxel, camptothecin, 5-fluorouracil, cisplatin, doxorubicin, mitomycin, epirubicin, and gemcitabine;
preferably, the photosensitizer comprises at least one of boron dipyrromethene, chlorin, and rose bengal;
preferably, the photothermal agent comprises at least one of a noble metal nanoparticle, an organic polymer, a carbon-based nanomaterial, a magnetic nanomaterial, and a semiconductor nanomaterial;
preferably, the immunotherapeutic drug comprises at least one of an immune cell therapy drug and an immune checkpoint inhibitor;
preferably, the immune cell therapy drug comprises at least one of a T cell therapy drug, a tumor-infiltrating lymphocyte therapy drug, and an NK cell therapy drug;
preferably, the immune checkpoint inhibitor comprises an inhibitor targeting at least one of PD-1, PD-L1, PD-L2, CTLA-4, TIM-3, LAG-3, TIGIT, VISTA, BTLA, CD27, CD28, CD70, CD80, CD86, CD137, CD276, KIRs, TNFRSF4, GITR, GITRL, 4-1BBL, A2aR, VTCN1, IDO, and KLRA;
preferably, the first product comprises 1 × 10³ CFU/g to 1 × 10¹³ CFU/g or 1 × 10³ CFU/mL to 1 × 10¹³ CFU/mL of the *Parabacteroides* strain;
preferably, each gram or each milliliter of the first product comprises at least 1 × 10³, 2 × 10³, 5 × 10³, 1 × 10⁴, 2 × 10⁴, 5 × 10⁴, 1 × 10⁵, 2 × 10⁵, 5 × 10⁵, 1 × 10⁶, 2 × 10⁶, 5 × 10⁶, 1 × 10⁷, 2 × 10⁷, 5 × 10⁷, 1 × 10⁸, 2 × 10⁸, 5 × 10⁸, 1 × 10⁹, 2 × 10⁹, 5 × 10⁹, 1 × 10¹⁰, 2 × 10¹⁰, 5 × 10¹⁰, 1 × 10¹¹, 2 × 10¹¹, 5 × 10¹¹, 1 × 10¹², 2 × 10¹², 5 × 10¹², or 1 × 10¹³ CFU of the *Parabacteroides* strain;
preferably, the *Parabacteroides* strain is present in the first product in a form of live bacteria, attenuated bacteria, killed bacteria, lyophilized bacteria, or irradiated bacteria;
preferably, the first product and/or the second product further comprises at least one of an excipient, a diluent, and a carrier;
preferably, the first product and the second product each independently further comprise at least one adjuvant;
preferably, the adjuvant comprises at least one of a lubricant, a wetting agent, a binder, an emulsifier, a suspension stabilizer, a solubilizer, a preservative, a sweetener, and a flavoring agent;
preferably, the pharmaceutical combination product is in an infant-suitable dosage form, a child-suitable dosage form, or an adult-suitable dosage form;
preferably, the first product and the second product are each independently in a dosage form for enteral administration or a dosage form for parenteral administration;
preferably, the first product and the second product are each independently an oral preparation or an injection;
preferably, the first product and the second product are each independently any one of a powder, a granule, a tablet, a capsule, a gel, a suspension, a drop, a dropping pill, an injection, a suppository, an aerosol, an oral liquid, an ointment, an emulsion, and a lavage solution;
preferably, the *Parabacteroides* strain is capable of at least partially proliferating in a gut of a subject; and
preferably, the pharmaceutical combination product further comprises a package insert.

7. Use of the *Parabacteroides* strain according to claim 1, the culture according to claim 2, the microbial agent according to claim 3, the composition according to claim 4, the pharmaceutical composition according to claim 5, or the pharmaceutical combination product according to claim 6 in preparation of a drug for preventing and/or treating at least one disease of following (1) to (6):
(1) at least one of a tumor or cancer, a metabolic disease, an inflammatory or autoimmune disease, an infectious disease, and a central nervous system disease;
(2) at least one of a solid tumor, a soft tissue tumor, a hematopoietic tumor, a glandular tumor, and a metastatic tumor;
wherein preferably, the solid tumor comprises at least one of head and neck cancer, upper gastrointestinal cancer, lower gastrointestinal cancer, hepatobiliary cancer, neuroendocrine tumor, thoracic cancer, bone tumor, soft tissue sarcoma, skin cancer, breast cancer, female reproductive system cancer, male reproductive system cancer, urinary system cancer, ocular cancer, central nervous system cancer, and endocrine system cancer;
preferably, the hematopoietic tumor comprises a tumor in at least one of blood, lymph, and bone marrow;
preferably, the tumor or cancer comprises at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejunoileal neuroendocrine tumor, appendiceal neuroendocrine tumor, colorectal neuroendocrine tumor, pancreatic neuroendocrine tumor, thymic carcinoma, lung cancer, malignant pleural mesothelioma, angiosarcoma, desmoid tumor, Ewing sarcoma, fibrosarcoma, gastrointestinal stromal tumor, Kaposi sarcoma, leiomyosarcoma, liposarcoma, myxofibrosarcoma, malignant peripheral nerve sheath tumor, rhabdomyosarcoma, synovial sarcoma, undifferentiated pleomorphic sarcoma, dermatofibrosarcoma protuberans, Merkel cell carcinoma, cutaneous malignant melanoma, vulvar cancer, vaginal cancer, cervical cancer, endometrial cancer, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid cancer, conjunctival cancer, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia;
preferably, the tumor or cancer comprises a tumor or cancer caused by a bacterium or virus; and
preferably, the bacterium or virus comprises at least one of hepatitis B virus, hepatitis C virus, human papillomavirus, Epstein-Barr (EB) virus, *Helicobacter pylori*, and *Fusobacterium nucleatum*;
(3) a metabolic disease comprising a disease **characterized by** a metabolic disorder in at least one of an amino acid, an organic acid, a sugar, a fat, a purine, a pigment, blood ammonia, and a metal ion;
(4) an inflammatory or autoimmune disease comprising at least one of psoriasis, psoriatic arthritis, multiple sclerosis, inflammatory bowel disease, ankylosing spondylitis, chronic obstructive pulmonary disease, mesangiocapillary glomerulonephritis, myocarditis, dry eye syndrome, uveitis, Behcet's disease, asthma, atopic dermatitis, acne, Crohn's disease, ulcerative colitis, bronchitis, allergic rhinitis, Graves' disease, Hashimoto's thyroiditis, rheumatoid arthritis, type 1 diabetes, systemic lupus erythematosus, scleroderma, glomerulonephritis, systemic vasculitis, Addison's disease, autoimmune myositis, and Sjogren's syndrome;
(5) an infectious disease comprising a disease caused by an infection with at least one of a bacterium, a virus, and a fungus; and
(6) a central nervous system disease comprising at least one of stroke, schizophrenia, drug addiction, Alzheimer's disease, Parkinson's disease, anxiety disorder, and depression,
wherein preferably, preventing and/or treating a tumor is achieved through at least one of following (a) to (l):
(a) inhibiting an increase in tumor volume;
(b) inhibiting an increase in tumor weight;
(c) inhibiting proliferation of a tumor cell;
(d) enhancing a response rate of a tumor treatment;
(e) enhancing therapeutic efficacy of an immune checkpoint inhibitor or a chemotherapeutic drug, comprising enhancing therapeutic efficacy of an anti-PD-1 antibody or gemcitabine;
(f) preventing and/or inhibiting dissemination or metastasis of a tumor cell;
(g) reducing resistance to an anti-tumor drug;
(h) inhibiting an activity of histone deacetylase (HDAC) through at least one of SCFAs comprising acetic acid or acetate, propionic acid or propionate, butyric acid or butyrate, and valeric acid or valerate;
(i) inhibiting an activity of HDAC and/or promoting a transcriptional activity of interferon beta (IFN-β);
(j) regulating or activating an immune system of a subject;
(k) promoting senescence or apoptosis of a tumor cell;
(l) inhibiting angiogenesis within a tumor tissue;
(m) enhancing an effect of a tumor-killing immune cell;
(n) local tumor immunotherapy; and
(o) relieving immunosuppression in a tumor microenvironment.

8. Use of the *Parabacteroides* strain according to claim 1, the culture according to claim 2, the microbial agent according to claim 3, the composition according to claim 4, the pharmaceutical composition according to claim 5, or the pharmaceutical combination product according to claim 6 in preventing and/or treating at least one disease of following (1) to (6):
(1) at least one of a tumor or cancer, a metabolic disease, an inflammatory or autoimmune disease, an infectious disease, and a central nervous system disease;
(2) at least one of a solid tumor, a soft tissue tumor, a hematopoietic tumor, a glandular tumor, and a metastatic tumor;
wherein preferably, the solid tumor comprises at least one of head and neck cancer, upper gastrointestinal cancer, lower gastrointestinal cancer, hepatobiliary cancer, neuroendocrine tumor, thoracic cancer, bone tumor, soft tissue sarcoma, skin cancer, breast cancer, female reproductive system cancer, male reproductive system cancer, urinary system cancer, ocular cancer, central nervous system cancer, and endocrine system cancer;
preferably, the hematopoietic tumor comprises a tumor in at least one of blood, lymph, and bone marrow;
preferably, the tumor or cancer comprises at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejunoileal neuroendocrine tumor, appendiceal neuroendocrine tumor, colorectal neuroendocrine tumor, pancreatic neuroendocrine tumor, thymic carcinoma, lung cancer, malignant pleural mesothelioma, angiosarcoma, desmoid tumor, Ewing sarcoma, fibrosarcoma, gastrointestinal stromal tumor, Kaposi sarcoma, leiomyosarcoma, liposarcoma, myxofibrosarcoma, malignant peripheral nerve sheath tumor, rhabdomyosarcoma, synovial sarcoma, undifferentiated pleomorphic sarcoma, dermatofibrosarcoma protuberans, Merkel cell carcinoma, cutaneous malignant melanoma, vulvar cancer, vaginal cancer, cervical cancer, endometrial cancer, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid cancer, conjunctival cancer, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia;
preferably, the tumor or cancer comprises a tumor or cancer caused by a bacterium or virus; and
preferably, the bacterium or virus comprises at least one of hepatitis B virus, hepatitis C virus, human papillomavirus, EB virus, *Helicobacter pylori*, and *Fusobacterium nucleatum*;
(3) a metabolic disease comprising a disease **characterized by** a metabolic disorder in at least one of an amino acid, an organic acid, a sugar, a fat, a purine, a pigment, blood ammonia, and a metal ion;
(4) an inflammatory or autoimmune disease comprising at least one of psoriasis, psoriatic arthritis, multiple sclerosis, inflammatory bowel disease, ankylosing spondylitis, chronic obstructive pulmonary disease, mesangiocapillary glomerulonephritis, myocarditis, dry eye syndrome, uveitis, Behcet's disease, asthma, atopic dermatitis, acne, Crohn's disease, ulcerative colitis, bronchitis, allergic rhinitis, Graves' disease, Hashimoto's thyroiditis, rheumatoid arthritis, type 1 diabetes, systemic lupus erythematosus, scleroderma, glomerulonephritis, systemic vasculitis, Addison's disease, autoimmune myositis, and Sjogren's syndrome;
(5) an infectious disease comprising a disease caused by an infection with at least one of a bacterium, a virus, and a fungus; and
(6) a central nervous system disease comprising at least one of stroke, schizophrenia, drug addiction, Alzheimer's disease, Parkinson's disease, anxiety disorder, and depression,
wherein preferably, preventing and/or treating a tumor is achieved through at least one of following (a) to (o):
(a) inhibiting an increase in tumor volume;
(b) inhibiting an increase in tumor weight;
(c) inhibiting proliferation of a tumor cell;
(d) enhancing a response rate of a tumor treatment;
(e) enhancing therapeutic efficacy of an immune checkpoint inhibitor or a chemotherapeutic drug, comprising enhancing therapeutic efficacy of an anti-PD-1 antibody or gemcitabine;
(f) preventing and/or inhibiting dissemination or metastasis of a tumor cell;
(g) reducing resistance to an anti-tumor drug;
(h) inhibiting an activity of HDAC through at least one of SCFAs comprising acetic acid or acetate, propionic acid or propionate, butyric acid or butyrate, and valeric acid or valerate;
(i) inhibiting an activity of HDAC and/or promoting a transcriptional activity of IFN-β;
(j) regulating or activating an immune system of a subject;
(k) promoting senescence or apoptosis of a tumor cell;
(l) inhibiting angiogenesis within a tumor tissue;
(m) enhancing an effect of a tumor-killing immune cell;
(n) local tumor immunotherapy; and
(o) relieving immunosuppression in a tumor microenvironment.

9. A method for treating a disease, comprising administering an effective dose of the *Parabacteroides* strain according to claim 1, the culture according to claim 2, the microbial agent according to claim 3, the composition according to claim 4, the pharmaceutical composition according to claim 5, or the pharmaceutical combination product according to claim 6 to a subject,
wherein preferably, the disease comprises at least one of a tumor or cancer, a metabolic disease, an inflammatory or autoimmune disease, an infectious disease, and a central nervous system disease;
preferably, the tumor or cancer comprises at least one of a solid tumor, a soft tissue tumor, a hematopoietic tumor, a glandular tumor, and a metastatic tumor;
preferably, the solid tumor comprises at least one of head and neck cancer, upper gastrointestinal cancer, lower gastrointestinal cancer, hepatobiliary cancer, neuroendocrine tumor, thoracic cancer, bone tumor, soft tissue sarcoma, skin cancer, breast cancer, female reproductive system cancer, male reproductive system cancer, urinary system cancer, ocular cancer, central nervous system cancer, and endocrine system cancer;
preferably, the hematopoietic tumor comprises a tumor in at least one of blood, lymph, and bone marrow;
preferably, the tumor or cancer comprises at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejunoileal neuroendocrine tumor, appendiceal neuroendocrine tumor, colorectal neuroendocrine tumor, pancreatic neuroendocrine tumor, thymic carcinoma, lung cancer, malignant pleural mesothelioma, angiosarcoma, desmoid tumor, Ewing sarcoma, fibrosarcoma, gastrointestinal stromal tumor, Kaposi sarcoma, leiomyosarcoma, liposarcoma, myxofibrosarcoma, malignant peripheral nerve sheath tumor, rhabdomyosarcoma, synovial sarcoma, undifferentiated pleomorphic sarcoma, dermatofibrosarcoma protuberans, Merkel cell carcinoma, cutaneous malignant melanoma, vulvar cancer, vaginal cancer, cervical cancer, endometrial cancer, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid cancer, conjunctival cancer, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia;
preferably, the tumor or cancer comprises a tumor or cancer caused by a bacterium or virus;
preferably, the bacterium or virus comprises at least one of hepatitis B virus, hepatitis C virus, human papillomavirus, EB virus, *Helicobacter pylori*, and *Fusobacterium nucleatum*;
preferably, the metabolic disease comprises a disease **characterized by** a metabolic disorder in at least one of an amino acid, an organic acid, a sugar, a fat, a purine, a pigment, blood ammonia, and a metal ion;
preferably, the inflammatory or autoimmune disease comprises at least one of psoriasis, psoriatic arthritis, multiple sclerosis, inflammatory bowel disease, ankylosing spondylitis, chronic obstructive pulmonary disease, mesangiocapillary glomerulonephritis, myocarditis, dry eye syndrome, uveitis, Behcet's disease, asthma, atopic dermatitis, acne, Crohn's disease, ulcerative colitis, bronchitis, allergic rhinitis, Graves' disease, Hashimoto's thyroiditis, rheumatoid arthritis, type 1 diabetes, systemic lupus erythematosus, scleroderma, glomerulonephritis, systemic vasculitis, Addison's disease, autoimmune myositis, and Sjogren's syndrome;
preferably, the infectious disease comprises a disease caused by an infection with at least one of a bacterium, a virus, and a fungus;
preferably, the central nervous system disease comprises at least one of stroke, schizophrenia, drug addiction, Alzheimer's disease, Parkinson's disease, anxiety disorder, and depression;
preferably, preventing and/or treating a tumor is achieved through at least one of following (a) to (o):
(a) inhibiting an increase in tumor volume;
(b) inhibiting an increase in tumor weight;
(c) inhibiting proliferation of a tumor cell;
(d) enhancing a response rate of a tumor treatment;
(e) enhancing therapeutic efficacy of an immune checkpoint inhibitor or a chemotherapeutic drug, comprising enhancing therapeutic efficacy of an anti-PD-1 antibody or gemcitabine;
(f) preventing and/or inhibiting dissemination or metastasis of a tumor cell;
(g) reducing resistance to an anti-tumor drug;
(h) inhibiting an activity of HDAC through at least one of SCFAs comprising acetic acid or acetate, propionic acid or propionate, butyric acid or butyrate, and valeric acid or valerate;
(i) inhibiting an activity of HDAC and/or promoting a transcriptional activity of IFN-β;
(j) regulating or activating an immune system of a subject;
(k) promoting senescence or apoptosis of a tumor cell;
(l) inhibiting angiogenesis within a tumor tissue;
(m) enhancing an effect of a tumor-killing immune cell;
(n) local tumor immunotherapy; and
(o) relieving immunosuppression in a tumor microenvironment;
preferably, the *Parabacteroides* strain, the culture, the microbial agent, the composition, the pharmaceutical composition, or the pharmaceutical combination product is administered at least once daily;
preferably, when the administering is conducted twice or more daily, a same dose is administered each time;
preferably, when the administering is conducted twice or more daily, a different dose is administered each time; and
preferably, a route for the administering comprises at least one of oral administration, sublingual administration, nasal administration, rectal administration, inhalation administration, transdermal administration, intraperitoneal injection, subcutaneous injection, intradermal injection, intramuscular injection, and intravenous injection.

10. A method for preparing the culture according to claim 2, comprising a following step:
inoculating the *Parabacteroides* strain according to claim 1 into a fermentation medium to produce the fermentation medium in which the *Parabacteroides* strain has been cultured; and
further comprising at least one of following steps: (1) centrifuging the fermentation medium in which the *Parabacteroides* strain has been cultured to produce the supernatant;
(2) concentrating the fermentation medium in which the *Parabacteroides* strain has been cultured or the supernatant through at least one of evaporation, lyophilization, dialysis, extraction, and membrane separation to produce the concentrate;
(3) subjecting the fermentation medium in which the *Parabacteroides* strain has been cultured or the supernatant to at least one of extraction and solvent extraction to produce the extract; and
(4) drying any one of the fermentation medium in which the *Parabacteroides* strain has been cultured, the supernatant, the concentrate, and the extract to produce the dried product.

11. A therapeutic composition, comprising: any one of following strains:
*Parabacteroides hominis*, *Parabacteroides merdae*, *Parabacteroides johnsonii*, *Parabacteroides massiliensis*, *Parabacteroides acidifaciens*, *Parabacteroides faecis*, *Parabacteroides bouchesdurhonensis*, *Parabacteroides provencensis*, *Parabacteroides goldsteinii*, *Parabacteroides gordonii*, *Parabacteroides timonensis*, *Parabacteroides chinchillae*, *Parabacteroides distasonis*, and *Parabacteroides hominis*; and/or a pharmaceutically acceptable carrier,
wherein the therapeutic composition prevents and/or treats a tumor through at least one of following (a) to (o):
(a) inhibiting an increase in tumor volume;
(b) inhibiting an increase in tumor weight;
(c) inhibiting proliferation of a tumor cell;
(d) enhancing a response rate of a tumor treatment;
(e) enhancing therapeutic efficacy of an immune checkpoint inhibitor or a chemotherapeutic drug, comprising enhancing therapeutic efficacy of an anti-PD-1 antibody or gemcitabine;
(f) preventing and/or inhibiting dissemination or metastasis of a tumor cell;
(g) reducing resistance to an anti-tumor drug;
(h) inhibiting an activity of HDAC through at least one of SCFAs comprising acetic acid or acetate, propionic acid or propionate, butyric acid or butyrate, and valeric acid or valerate;
(i) inhibiting an activity of HDAC and/or promoting a transcriptional activity of IFN-β;
(j) regulating or activating an immune system of a subject;
(k) promoting senescence or apoptosis of a tumor cell;
(l) inhibiting angiogenesis within a tumor tissue;
(m) enhancing an effect of a tumor-killing immune cell;
(n) local tumor immunotherapy; and
(o) relieving immunosuppression in a tumor microenvironment.
